# EUROPEAN PATENT SPECIFICATION

(11) **EP 4 087 657 B1**
(45) Date of publication and mention of the grant of the patent: **09.09.2026**
(21) Application number: 21702801.8
(22) Date of filing: 07.01.2021
(51) Int. Cl.: A61K 31/4439, A61P 35/00, A61K 39/395, A61K 45/06, A61K 47/68, C07K 16/28, C07K 16/42, A61P 11/00, C07K 16/40

(54) **ALK5 INHIBITOR CONJUGATES AND USES THEREOF**
ALK5-INHIBITOREN, KONJUGATE UND VERWENDUNGEN DAVON
CONJUGUÉS INHIBITEURS D'ALK5 ET LEURS UTILISATIONS

(30) Priority: 08.01.2020 US 202062958461 P
(43) Date of publication of application: 16.11.2022
(73) Proprietor: Synthis Therapeutics, Inc., Long Island City, NY 11101 (US)
(72) Inventor: THOMAS-KARYAT, Dori, Jersey City, New Jersey 07302 (US)
(74) Representative: Abel & Imray LLP
(86) International application number: PCT/US2021/012450
(87) International publication number: WO 2021/142086

(56) References cited:
- WO-A1-2020/013803
- WO-A1-2020/256721
- WO-A1-2020/256751
- US-A1- 2020 147 234
- MARIKE MARJOLIJN VAN BEUGE ET AL: "Enhanced Effectivity of an ALK5-Inhibitor after Cell-Specific Delivery to Hepatic Stellate Cells in Mice with Liver Injury", PLOS ONE, vol. 8, no. 2, 18 February 2013 (2013-02-18), pages 1 - 9, XP055604603, DOI: 10.1371/journal.pone.0056442

## Description

### 1. TECHNICAL FIELD OF THE INVENTION

The present invention relates to the treatment of fibrosis and cancer. In particular, the invention relates to targeted drug conjugates comprising ALK5 inhibitors, and their use as a medicament for the treatment of fibrosis and cancer.

### 2. BACKGROUND

### 2.1. Fibrosis

Idiopathic pulmonary fibrosis (IPF) is a devastating, chronic disease in the lung characterized by progressive stiffening and scarring of lung tissue (Lederer et al., 2018, NEJM, 378:1811-23; Barratt et al., 2018, J Clin Med 7(8):201). Approximately 130,000 patients are diagnosed annually in the US, with an 80% mortality rate at 5 years. To date, there are no cures for this disease, only options to slow its progress (Somogyi et al., 2019, Eur Respir Rev, 28(153):190021). IPF begins with repeated alveolar epithelial damage by extrinsic irritants (such as smoking) followed by persistent fibroblast activation, which is one of the drivers of fibrosis. Fibrosis is essentially a wound that cannot heal, due to constant lung injury.

Differentiation of lung fibroblasts into myofibroblasts is a primary step in the development of tissue fibrosis (Yazdani et al., 2017, Adv Drug Deliv Rev 121:101-116; Huang et al., 2014, Austin J Pulm Resp, 1(1):3-9). Myofibroblasts are responsible for fibrogenesis and predominately found and active in fibrotic areas. There are three possible sources of myofibroblasts in IPF: 1) resident lung epithelial cells that are converted into myofibroblasts in a process known as epithelial-mesenchymal transition (EMT); 2) resident lung fibroblasts that are converted to myofibroblasts (FMT); and/or 3) myofibroblasts that are recruited into the lung to drive fibrosis and scarring (Pardali et al., 2017, Int J Mol Sci, 18(10)). The combination of these three pathways leads to an increase in lung-resident myofibroblasts, which drive fibrotic diseases. Inhibiting myofibroblasts would be a major step in reversing fibrotic lung disease.

The pleiotropic cytokine transforming growth factor-beta (TGF-β) is responsible for the development, maintenance and homeostasis of the majority of tissues in the body. TGF-β initiates signaling via binding to TGF-β receptor II and TGF-β receptor I/ALK5. ALK5 is a serine threonine kinase receptor, which phosphorylates downstream signaling mediators, Smad2 and Smad3. Activated Smad2/3 forms a complex with Smad4 and translocates into the nucleus to regulate gene expression, which is determined by the cellular context (Derynck et al., 2003, Nature, 425(6958):577-84). In the lungs, TGF-β is produced by a wide variety of cell types, including alveolar macrophages, neutrophils, activated alveolar epithelial cells, endothelial cells, fibroblasts, and myofibroblasts (Caja et al., 2018, Int J Mol Sci, 19(5)). TGF-β is one of the most potent inducers of extracellular matrix (ECM) production, including alpha-smooth muscle actin (αSMA), collagen and fibronectin (Pohlers et al., 2009, Biochim Biophys Acta, 1792(8):746-56; Kim et al., 2018, Cold Spring Harb Perspect Biol, 10(4)). During IPF disease progression, TGF-β increases collagen expression and ECM deposition, myofibroblast expansion, fibroblast to myofibroblast transitions and epithelial-to-mesenchymal transformation (EMT) (Pardali et al., 2017, Int J Mol Sci, 18(10); Yue et al., 2010, Curr Enzym Inhib, 6(2)). Moreover, expression of TGF-β is elevated in both animal models of lung fibrosis and in fibrotic human lungs (Tashiro et al., 2017, Front Med (Lausanne), 4:118). In animal models of lung fibrosis, increased TGF-β levels precede collagen synthesis and deposition. As further proof of TGF-β's role as driver of *in vivo* lung fibrosis, animal models of adenoviral expressed TGF-β1 in the lung or transgenic lung specific expression of TGF-β1 was sufficient to drive lung fibrosis (Lee et al., Korean J Intern Med, 29: 281). In the classic mouse model of bleomycin induced IPF, TGF-β levels are elevated in the lung and blockade of TGF-β signaling by either Smad 3 knockout mice or dominant negative expression of the TGFbRII specifically in fibroblasts led to a decrease in disease severity (Fernandez et al., 2012, Proc Am Thorac Soc, 9(3):111-116; Degryse et al., 2011, Am J Physiol Lung Cell Mol Physiol, 300(6):887-897; Li et al., 2011, J Clin Invest, 121(1):277-87). Therapeutically, treatment with small molecule TGF-β receptor inhibitors or anti-TGF-β antibodies also inhibited disease in bleomycin and radiation induced fibrosis (Giri et al., 1993, Thorax, 48:959-66; Flechsig et al., 2012, Clin Cancer Res, 18(13):3616-27).

Due to TGF-β's prominent role in driving IPF, therapies targeting the TGF-β pathway have been investigated for the treatment of IPF. However, due to the broad expression of TGF-β and its receptors in the body, the risk of host tissue toxicity has made developing safe and effective therapies difficult (Anderton et al., 2011, Toxicologic Path, 39:916-24; Stauber et al., 2014, Clinical Tox, 4(3):1-10; Lonning et al, 2011, Curr Pharma Biotech, 12:2176-89). For example, a Phase 2 trial of the αvβ6 integrin antibody (BG00011), which systemically blocks TGF-β activation, was recently terminated due to safety concerns (Arefayene, et al., 2018, European Respiratory Journal, 52 (suppl 62) PA596). Like most drugs, the toxicity and therapeutic window has to be balanced; with broad acting TGF-β inhibitors, the safety and toxicity risks are paramount. A selective and potent TGF-β inhibitor that reverses fibrosis safely would likely stop disease progression and but could also improve patient survival rates.

In 2014, two IPF drugs were approved, pirfenidone, an anti-fibrotic molecule, as well as nintedanib, a tyrosine kinase inhibitor, both of which may partially block TGF-β signaling, along with other pathways (Gan et al., 2011, Ther Clin Risk Manag, 7:39-47; Margaritopoulos et al., 2016, Core Evid, 11:11-22; Lunardi et al., 2018, Arch Pathol Lab Med, 142:1090-1097). In general, pirfenidone and nintedanib treatment reduced the risk of IPF disease progression by 50% in patients with mild-moderate disease (Ren et al., 2017, Saudi Med J, 38(9):889-894; Case et al., 2017, BMJ Open Resp Res, 4:e000192). However, IPF patients with severely reduced lung function of <50%, (as measured by FVC, forced vital capacity, the total amount of air a patient can exhale), elderly patients with comorbidities, or those whose disease was not officially diagnosed as IPF, were excluded from these trials. While both drugs could slow disease, they were not able to completely stop or reverse disease progression. Trials in IPF are of keen interest, due to the significant unmet need that remains in fibrotic patients.

Other therapies, such as IFN-γ inhibitors, angiogenesis inhibitors, and TNF-α blockers, proved unsuccessful in treating IPF (Yazdani et al., 2017, Adv Drug Deliv Rev, 121:101-116; Somogyi et al., 2019, Eur Respir Rev, 28(153):190021). Ongoing trials in IPF include the serum amyloid protein P (Pentraxin; PTX-2), which is a circulating protein that binds to monocytes and inhibits their differentiation into pro-fibrotic fibrocytes, promoting epithelial healing and resolution of fibrosis. Levels of pentraxin are low in IPF patients and an ongoing phase 2 trial demonstrates improved lung function and in the 6-minute walk test. In a phase 2 trial, pamrevlumab, a fully recombinant human monoclonal antibody against connective tissue growth factor (CTGF) that decreases fibrosis, reduced the decline in lung function (FVC) in IPF patients (Somogyi et al., 2019, Eur Respir Rev, 28(153):190021). In contrast, tralokinumab, an IL-13 antibody, which decreases TGF-β and CCl2 expression, and simtuzumab, an anti-LOXL2 antibody that decreases ECM crosslinking, both failed phase 2 trials due to lack of improvement in respiratory function (Raghu, 2017, European Respiratory Review, 26:170071). Van Beuge et al. (PLOS One, 2013, 8(2), e56442) reported that a conjugate of the ALK5-inhibitor LY-364947 coupled to mannose-6-phosphate human serum albumin (M6PHSA) could inhibit fibrotic markers in hepatic stellate cells, and reduced fibrogenic parameters *in vivo* without affecting other cell-types. Many of the therapies directly or indirectly modify TGF-β function. However, despite these efforts, there remains an unmet need for improved treatments for IPF patients, particularly for therapies that could selectively and safely modify disease.

Fibrosis is driven by TGF-β in multiple diseases other than IPF, including other types of pulmonary fibrosis *(e.g.,* associated with systemic sclerosis), liver fibrosis *(e.g.,* associated with nonalcoholic steatohepatitis (NASH)), kidney fibrosis, and cardiac fibrosis (Meng et al., 2016, Nat Rev Nephrol. 12(6):325-38; Biernacka et al., 2011, Growth Factors, 29(5):196-202; Gyorfi et al., 2017, Matrix Biology, 68-69:8-27. Thus, there is an unmet need for therapies capable of reversing TGF-β driven myofibroblast activation and decreasing fibrosis in subjects in need thereof, particularly patients suffering from pulmonary fibrosis, *e.g.,* IPF, liver fibrosis, *e.g.,* associated with NASH, kidney fibrosis, cardiac fibrosis, and systemic sclerosis.

### 2.2. Cancer

TGF-β signaling is also implicated in tumor progression, and inhibition of the TGF-β pathway as a cancer therapy has long been of interest (Syed, 2016, J Cell Biochem. 117(6):1279-87). However, due to concerns of host toxicity since TGF-β receptors are ubiquitously expressed and fears of inadvertently promoting tumor growth, most of the TGF-β inhibitors have remained in preclinical discovery stages.

TGF-β is secreted by tumor cells, cancer associated fibroblasts (CAFs) and/or surrounding tumor microenvironment (TME) cells. Among the stromal cells of the TME, CAFs are the most abundant and are critically involved in cancer progression (Pure and Blomberg, 2018, Oncogene, 37(32):4343-4357; Calon et al., 2014, Seminars in Cancer Bio, 25:15-22; Chen and Song, 2019, Nat Rev Drug Disc. 18:90). TGF-β is a key driver of CAF activation, recruitment, and viability, driving their differentiation from tissue resident fibroblasts and epithelial cells via epithelial-to-mesenchymal transition (EMT) and supporting their survival. In turn, CAFs have an effect on tumor growth, angiogenesis, cancer stemness, ECM remodeling, tissue invasion, metastasis, and even chemoresistance (Harryvan and van der Burg, 2019, J Clin Med, 8:1989). CAFs are a complex and often heterogenous population of cells identified using a combination of various intracellular and cell surface markers, including elevated expression of intracellular alpha-smooth muscle actin (SMA) and cell surface fibroblast activation protein (FAP) (Pure and Blomberg, 2018, Oncogene, 37(32):4343-4357). In bladder and colorectal cancer patients, TGF-β signaling promotes immune excluded or "cold" tumors, where T cells remain trapped outside the tumor by CAFs, which physically block them from infiltrating the tumor (Hegde, 2020, Immunity, 52: 17-35; Gajewski, 2015, Semin Oncol, 42: 663-671; Mariathasan and Powles, 2018, Nature, 554: 544-48).

Although TGF-β therapies are of interest for the treatment of cancer, historically they have not reached their full therapeutic potential due to the broad expression of TGF-β and its receptors and their role in the development, maintenance and homeostasis of tissues including the heart and bones. In addition, TGF-β is an early tumor suppressor, responsible for controlling the growth of early tumors, and systemic TGF-β therapies have been shown to cause tissue toxicity and increased early tumor growth (Anderton and Heier, 2011, Toxicologic Path, 39: 916; Stauber et al., 2014, Clinical Tox, 4(3):1-10; Lonning and McPherson, 2011, Curr Pharma Biotech, 12:2176-89).

Accordingly, there is a need to target TGF-β inhibitors to cell types in which the inhibition of TGF-β signaling is therapeutically useful, for example cancer associated fibroblasts ("CAFs"), while minimizing host tissue toxicity.

### 3. SUMMARY

The references to methods of treatment in the subsequent paragraphs of this description are to be interpreted as references to the compounds, pharmaceutical compositions and medicaments of the present invention for use in a method for treatment of the human body by therapy. The present disclosure relates to compositions and methods for treatment of fibrosis and cancer. The compositions and methods advantageously avoid on-target host toxicity associated with systemic administration of TGF-β inhibitors by targeting them primarily, and preferably only, to those cells in which they would confer a therapeutic benefit, thereby circumventing pleiotropic off target effects.

Herein is provided a targeted drug conjugate comprising an ALK5 inhibitor covalently attached to a targeting moiety which comprises an antibody or an antibody fragment that binds to a cell surface molecule expressed on the surface of myofibroblasts, activated fibroblasts, fibroblasts transitioning to myofibroblasts, or a combination thereof, wherein the ALK5 inhibitor is N-methyl-2-(4-(4-(3-(6-methylpyridin-2-yl)-1H-pyrazol-4-yl)pyridin-2-yl)phenoxy)ethan-1-amine.

Herein is provided a pharmaceutical composition comprising a targeted drug conjugate described above and a pharmaceutically acceptable carrier.

Herein is provided a targeted drug conjugate as described above for use in a method of treating fibrosis, optionally wherein the fibrosis is (a) pulmonary fibrosis, liver fibrosis, kidney fibrosis, cardiac fibrosis, skin fibrosis, or esophagus fibrosis or (b) idiopathic pulmonary fibrosis (IPF).

Herein is provided a targeted drug conjugate as described above for use in a method of treating systemic sclerosis.

Herein is provided a targeted drug conjugate as described above for use in a method of treating cancer.

In particular, the compositions and methods direct ALK5 inhibitors to myofibroblasts, activated fibroblasts (*e.g*., cancer-associated fibroblasts ("CAFs")), and fibroblasts transitioning to myofibroblasts (each cell type a "target cell") via an antibody or antibody fragment targeting moiety, that binds to a target cell cell surface molecule. Without being bound by theory, it is believed that the use of a targeting moiety can result in the localization of the ALK5 inhibitor to and internalization into target cells, thereby inhibiting the TGFβ pathway in the target cell while limiting systemic toxicity. Inhibition of the TGFβ pathway in, e.g., myofibroblasts or fibroblasts transitioning into myofibroblasts can result in inhibition of fibrogenesis (in the case of a subject having fibrosis or a disease associated with fibrosis). Inhibition of the TGFβ pathway in CAFs can result in inhibition of tumor progression (in the case of a subject having cancer). Without being bound by theory, it is believed that selective blockade of TGF-β signaling in CAFs can 1) remove the CAF-mediated block on immune cell infiltration, and/or 2) drive tumor clearance, and/or 3) decrease CAF viability and/or 4) bypass toxicity concerns associated with systemic TGF-β inhibitors.

Accordingly, the present disclosure provides targeted drug conjugates (TDCs) in which the drug is an ALK5 inhibitor. The TDCs of the disclosure comprise an antibody or antibody fragment targeting component that binds to a cell surface molecule of a target cell (*e.g*., a human myofibroblast cell surface molecule). Without being bound by theory, it is believed that TDCs of the disclosure can provide a therapeutic effect by promoting target cell de-differentiation to resting fibroblasts and/or by promoting target cell apoptosis. Section 5.2 describes exemplary targeting moieties that can be used in the TDCs of the disclosure. The ALK5 inhibitor is N-methyl-2-(4-(4-(3-(6-methylpyridin-2-yl)-1H-pyrazol-4-yl)pyridin-2-yl)phenoxy)ethan-1-amine (referred to herein as "Compound C").

The ALK5 inhibitor can be directly conjugated to the targeting moiety or linked to the targeting moiety by a linker. The linker can be a non-cleavable linker or, preferably, a cleavable linker. Exemplary non-cleavable and cleavable linkers are described in Section 5.4. The average number of ALK5 inhibitor molecules attached per targeting moiety can vary, and generally ranges from 2 to 8 ALK5 inhibitor molecules per targeting moiety. Drug loading is described in detail in Section 5.5.

The present disclosure further provides pharmaceutical compositions comprising a TDC of the disclosure. Exemplary pharmaceutical excipients that can be used to formulate a pharmaceutical composition comprising a TDC of the disclosure are described in Section 5.6.

The present disclosure further provides a TDC of the disclosure or a pharmaceutical composition of the disclosure for use in methods of treating fibrosis and methods of treating cancer. The TDCs and pharmaceutical compositions of the disclosure can be administered as monotherapy or as part of a combination therapy, for example in combination with another therapeutic agent such as pirfenidone or nintedanib (when treating a subject having fibrosis or a disease associated with fibrosis) or a chemotherapeutic agent (when treating a subject having cancer). As another example, the TDCs and pharmaceutical compositions can be administered in combination with a checkpoint inhibitor when treating a subject having cancer. Exemplary types of conditions that can be treated with the TDCs and pharmaceutical compositions of the disclosure and exemplary combination therapies are described in Section 5.7.

### 4. BRIEF DESCRIPTION OF THE FIGURES

**FIGS. 1A-1D** show inhibition of TGF-β-induced luciferase activity in HEK293T cells by Compounds A-D. FIG. 1A: Compound A; FIG. 1B: Compound B; FIG. 1C: Compound C; FIG. 1D: Compound D.
**FIGS. 2A-2C** show that anti-FAP antibody binds to HEK cells only when human FAP cDNA is transfected and expressed on the cell surface. FIG. 2A: unstained HEK cells. FIG. 2B: HEK cells stained with anti-FAP antibody. FIG. 2C: HEK cells transfected with FAP cDNA and stained with anti-FAP antibody.
**FIGS. 3A-3B** show the linkers and payloads used in targeted drug conjugates SYN-301 (FIG. 3A) and SYN-302 (FIG. 3B).
**FIGS. 4A-4B** show that SYN-301 represses TGF-β signaling in HEK cells expressing human FAP protein. FIG. 4A: relative luciferase reporter expression in HEK cells expressing human FAP protein. FIG. 4B: relative luciferase reporter expression in untransfected HEK cells.
**FIGS. 5A-5E** show that 50-60% of WI-38 cells express FAP. FIG. 5A: unstained WI-38 cells. FIG. 5B: WI-38 cells stained with anti-FAP antibody. FIG. 5C: WI-38 cells stained with SYN-301. FIG. 5D: WI-38 cells stained with SYN-302. FIG. 5E: WI-38 cells stained with isotype control ADC.
**FIG. 6** shows percent FAP internalization induced by anti-FAP antibody (63%), SYN-301 (63%), and SYN-302 (52%).
**FIGS. 7A-7B** show effects of SYN-301 and SYN-302 on RNA expression of collagen and fibronectin (FIG. 7A) and LRRC15 (FIG. 7B) in WI-38 cells.

### 5. DETAILED DESCRIPTION

The disclosure provides targeted drug conjugates (TDCs) useful for treating fibrosis and cancer comprising a targeting moiety covalently bonded to an ALK5 inhibitor, either directly or through a linker. An overview of the TDCs of the disclosure is presented in Section 5.1. The targeting moiety of the TDCs can comprise an intact antibody or a fragment thereof. Targeting moieties that can be used in the TDCs of the disclosure are described in detail in Section 5.2. ALK5 inhibitors that can be used in the TDCs of the disclosure are described in Section 5.3. The TDCs of the disclosure typically contain a linker between the targeting moiety and ALK5 inhibitor. Exemplary linkers that can be used in TDCs of the disclosure are described in Section 5.4. The TDCs of the disclosure can contain varying numbers of ALK5 inhibitor moieties per targeting moiety. Drug loading is discussed in detail in Section 5.5. The disclosure further provides pharmaceutical formulations comprising a TDC of the disclosure. Pharmaceutical formulations comprising TDCs are described in Section 5.6. The disclosure further provides methods of treating fibrosis and methods of treating cancer using the TDCs of the disclosure. Methods of using the TDCs of the disclosure as monotherapy or as part of a combination therapy for the treatment of fibrosis or cancer are described in Section 5.7.

### 5.1. Drug Conjugates

The TDCs of the disclosure are composed of an ALK5 inhibitor covalently attached to an antibody or antibody fragment targeting moiety, typically via a linker, such that covalent attachment does not interfere with binding to the targeting moiety's target.

Techniques for conjugating drugs to targeting moieties such as antibodies and antibody fragments are well known in the art (See, *e.g.,* Hellstrom et al., Controlled Drug Delivery, 2nd Ed., at pp. 623-53 (Robinson et al., eds., 1987)); Thorpe et al., 1982, Immunol. Rev. 62:119-58; Dubowchik et al., 1999, Pharmacology and Therapeutics 83:67-123; and Zhou, 2017, Biomedicines 5(4):E64). The ALK5 inhibitors are preferably attached to a targeting moiety in the TDCs of the disclosure via site-specific conjugation. For example, an ALK5 inhibitor can be conjugated to the targeting moiety via one or more native or engineered cysteine, lysine, or glutamine residues, one or more unnatural amino acids (*e.g., p*-acetylphenylalanine (pAcF), *p-*azidomethyl-L-phenylalanine (pAMF), or selenocysteine (Sec)), one or more glycans (*e.g*., fucose, 6-thiofucose, galactose, *N*-acetylgalactosamine (GalNAc), *N*-acetylglucosamine (GlcNAc), or sialic acid (SA)), or one or more short peptide tags of four to six amino acids. See, *e.g.*, Zhou, 2017, Biomedicines 5(4):E64.

In one example, the targeting moiety is fused via a covalent bond (*e.g*., a peptide bond), through the targeting moiety's N-terminus or the C-terminus or internally, to an amino acid sequence of another protein (or portion thereof; for example, at least a 10, 20 or 50 amino acid portion of the protein). The targeting moiety can be linked to the other protein at the N-terminus, for example an antibody or antibody fragment can be linked at the N-terminus of the constant domain of the antibody. Recombinant DNA procedures can be used to create such fusions, for example as described in WO 86/01533 and EP0392745. In another example, an effector molecule can increase half-life *in vivo,* and/or enhance the delivery of a TDC to target cells. Examples of suitable effector molecules of this type include polymers, albumin, albumin binding proteins or albumin binding compounds such as those described in PCT publication no. WO 2005/117984.

The metabolic process or reaction may be an enzymatic process, such as proteolytic cleavage of a peptide linker of the TDC, or hydrolysis of a functional group such as a hydrazone, ester, or amide. Intracellular metabolites include, but are not limited to, peptides and free drug which have undergone intracellular cleavage after entry, diffusion, uptake or transport into a cell.

The terms "intracellularly cleaved" and "intracellular cleavage" refer to a metabolic process or reaction inside a cell on a drug conjugate whereby the covalent attachment, *e.g*., linker, between the drug moiety (D) and the targeting moiety is broken, resulting in the free drug dissociated from the targeting moiety inside the cell. The cleaved moieties of the TDC are thus intracellular metabolites.

### 5.2. The Targeting Moiety

The present disclosure provides drug conjugates in which the targeting moiety binds to a target cell cell surface molecule. The targeting moiety comprises an antibody or antibody fragment (such conjugates are sometimes referred to herein as "antibody drug conjugates" or "ADCs"). Thus, it should be understood that the term "targeting moiety" encompasses peptides (*e.g.,* peptides that are ten to forty amino acids in length), single chain polypeptides *(e.g.,* polypeptides more than forty amino acids in length, for example single chain variable regions or scFvs), and molecules comprising multiple polypeptide chains (*e.g*., multimeric immunoglobulin molecules).

Unless indicated otherwise, the term "antibody" refers to an immunoglobulin molecule that specifically binds to, or is immunologically reactive with, a particular antigen, and includes polyclonal, monoclonal, genetically engineered and otherwise modified forms of antibodies, including but not limited to chimeric antibodies, humanized antibodies, heteroconjugate antibodies (*e.g*., bispecific antibodies, diabodies, triabodies, and tetrabodies), and antibody fragments of antibodies, including, *e.g*., Fab', F(ab')₂, Fab, Fv, rlgG, and scFv fragments. Moreover, unless otherwise indicated, the term "monoclonal antibody" (mAb) is meant to include both intact molecules, as well as, antibody fragments (such as, for example, Fab and F(ab')₂ fragments) which are capable of specifically binding to a protein. Fab and F(ab')₂ fragments lack the Fc fragment of intact antibody, clear more rapidly from the circulation of the animal or plant, and may have less non-specific tissue binding than an intact antibody (Wahl et al., 1983, J. Nucl. Med. 24:316).

References to "VH" refer to the variable region of an immunoglobulin heavy chain of an antibody, including the heavy chain of an Fv, scFv, or Fab. References to "VL" refer to the variable region of an immunoglobulin light chain, including the light chain of an Fv, scFv, dsFv or Fab. Antibodies and immunoglobulins (Igs) are glycoproteins having the same structural characteristics. While antibodies exhibit binding specificity to a specific target, immunoglobulins include both antibodies and other antibody-like molecules which lack target specificity. Native antibodies and immunoglobulins are usually heterotetrameric glycoproteins of about 150,000 Daltons, composed of two identical light (L) chains and two identical heavy (H) chains. Each heavy chain has at the amino terminus a variable domain (VH) followed by a number of constant domains. Each light chain has a variable domain at the amino terminus (VL) and a constant domain at the carboxy terminus.

For optimal delivery of the ALK5 inhibitor within a cell, the targeting moieties are preferably internalizing, for example internalizing antibodies. Internalizing targeting moieties, after binding to their target molecules on cellular surface, are internalized by the cells as a result of the binding. The effect of this is that the TDC is taken up by cells. Processes which allow the determination of the internalization of, *e*.*g*., an antibody after binding to its antigen are known to the skilled person and are described for example on page 80 of PCT publication no. WO 2007/070538. Once internalized, if a cleavable linker is used to attach the ALK5 inhibitor to the targeting moiety, for example as described in Section 5.4, the ALK5 inhibitor can be released from the targeting moiety by cleavage in the lysosome or by other cellular mechanism.

The term "antibody fragment" refers to a portion of a full-length antibody, generally the target binding or variable region. Examples of antibody fragments include Fab fragments, Fab' fragments, F(ab')₂ fragments, Fv fragments, scFv fragments, dsFv fragments and single domain antibodies.

An "Fv" fragment is the minimum antibody fragment which contains a complete target recognition and binding site. This region consists of a dimer of one heavy and one light chain variable domain in a tight, noncovalent association (VH-VL dimer). It is in this configuration that the three CDRs of each variable domain interact to define a target binding site on the surface of the VH-VL dimer. Often, the six CDRs confer target binding specificity to the antibody. However, in some instances even a single variable domain (or half of an Fv comprising only three CDRs specific for a target) can have the ability to recognize and bind target.

"Single chain Fv" or "scFv" antibody fragments comprise the VH and VL domains of an antibody in a single polypeptide chain. Generally, the scFv polypeptide further comprises a polypeptide linker between the VH and VL domain that enables the scFv to form the desired structure for target binding. Various scFv linkers have been described in the art. See, *e.g*., Shen et al., 2008, Anal Chem. 80(6): 1910-1917; Yusakul, et al., 2016, Biosci Biotechnol Biochem. 80(7):1306-12. An exemplary scFv linker comprises the sequence (GGGGS)ₙ, where n is between 1 and 10.

"Disulfide stabilized Fv" or "dsFv" antibody fragments comprise the VH and VL domains of an antibody stabilized by an interdomain disulfide bond. See, Brinkmann U., 2010, Disulfide-Stabilized Fv Fragments. In: Kontermann R., Dübel S. (eds) Antibody Engineering. Springer, Berlin, Heidelberg.

"Single domain antibodies" are composed of a single VH or VL domains (*e.g*., of a human or murine antibody) which exhibit sufficient affinity to the target (*e.g.*, FAP). In a specific embodiment, the single domain antibody is a camelid V_{H}H antibody fragment (see, *e.g*., Riechmann, 1999, Journal of Immunological Methods 231:25-38). Use of single domain antibodies in the TDCs of the disclosure can be advantageous because of their small size compared to full length antibodies, high solubility, high stability, and excellent tissue penetration *in vivo.* Various methods for making single domain antibodies have been described. See, *e.g*., U.S. Patent No. 10,030,068, US 2006/0246058, U.S. Patent. No. 7,371,849, Vincke et al, 2008, JBC, 284(5):3273-3284.

The Fab fragment contains the constant domain of the light chain and the first constant domain (CH1) of the heavy chain. Fab' fragments differ from Fab fragments by the addition of a few residues at the carboxyl terminus of the heavy chain CH1 domain including one or more cysteines from the antibody hinge region. F(ab') fragments are produced by cleavage of the disulfide bond at the hinge cysteines of the F(ab')₂ pepsin digestion product. Additional chemical couplings of antibody fragments are known to those of ordinary skill in the art.

In certain embodiments, the antibodies of the disclosure are monoclonal antibodies. The term "monoclonal antibody" as used herein is not limited to antibodies produced through hybridoma technology. The term "monoclonal antibody" refers to an antibody that is derived from a single clone, including any eukaryotic, prokaryotic, or phage clone and not the method by which it is produced. Monoclonal antibodies useful in connection with the present disclosure can be prepared using a wide variety of techniques known in the art including the use of hybridoma, recombinant, and phage display technologies or a combination thereof. The antibodies of the disclosure include chimeric, primatized, humanized, or human antibodies.

The antibodies of the disclosure can be chimeric antibodies. The term "chimeric" antibody as used herein refers to an antibody having variable sequences derived from a non-human immunoglobulin, such as rat or mouse antibody, and human immunoglobulin constant regions, typically chosen from a human immunoglobulin template. Methods for producing chimeric antibodies are known in the art. See, *e.g.*, Morrison, 1985, Science 229(4719):1202-7; Oi et al., 1986, BioTechniques 4:214-221; Gillies et al., 1985, J. Immunol. Methods 125:191-202; U.S. patent nos. 5,807,715; 4,816,567; and 4,816,397.

The antibodies of the disclosure can be humanized. "Humanized" forms of non-human (*e.g*., murine) antibodies are chimeric immunoglobulins, immunoglobulin chains or fragments thereof (such as Fv, Fab, Fab', F(ab')₂ or other target-binding subdomains of antibodies) which contain minimal sequences derived from non-human immunoglobulin. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody can also comprise at least a portion of an immunoglobulin constant region (Fc), typically that of a human immunoglobulin consensus sequence. Methods of antibody humanization are known in the art. See, *e.g.,* Riechmann et al., 1988, Nature 332:323-7; U.S. patent nos. 5,530,101; 5,585,089; 5,693,761; 5,693,762; and 6,180,370 to Queen et al.*;* European patent publication no. EP239400; PCT publication WO 91/09967; U.S. patent no. 5,225,539; European patent publication no. EP592106; European patent publication no. EP519596; Padlan, 1991, Mol. Immunol., 28:489-498; Studnicka et al., 1994, Prot. Eng. 7:805-814; Roguska et al., 1994, Proc. Natl. Acad. Sci. 91:969-973; and U.S. patent no. 5,565,332.

The antibodies of the disclosure can be human antibodies. Completely "human" antibodies can be desirable for therapeutic treatment of human patients. As used herein, "human antibodies" include antibodies having the amino acid sequence of a human immunoglobulin and include antibodies isolated from human immunoglobulin libraries or from animals transgenic for one or more human immunoglobulin and that do not express endogenous immunoglobulins. Human antibodies can be made by a variety of methods known in the art including phage display methods using antibody libraries derived from human immunoglobulin sequences. See U.S. patent nos. 4,444,887 and 4,716,111; and PCT publication nos. WO 98/46645; WO 98/50433; WO 98/24893; WO 98/16654; WO 96/34096; WO 96/33735; and WO 91/10741. Human antibodies can also be produced using transgenic mice which are incapable of expressing functional endogenous immunoglobulins but which can express human immunoglobulin genes. See, *e.g*., PCT publications WO 98/24893; WO 92/01047; WO 96/34096; WO 96/33735; U.S. patent nos. 5,413,923; 5,625,126; 5,633,425; 5,569,825; 5,661,016; 5,545,806; 5,814,318; 5,885,793; 5,916,771; and 5,939,598. In addition, companies such as Medarex (Princeton, N.J.), Astellas Pharma (Deerfield, Ill.), Amgen (Thousand Oaks, Calif.) and Regeneron (Tarrytown, N.Y.) can be engaged to provide human antibodies directed against a selected antigen using technology similar to that described above. Completely human antibodies that recognize a selected epitope can be generated using a technique referred to as "guided selection." In this approach a selected non-human monoclonal antibody, *e.g*., a mouse antibody, is used to guide the selection of a completely human antibody recognizing the same epitope (Jespers et al., 1988, Biotechnology 12:899-903).

The antibodies of the disclosure can be primatized. The term "primatized antibody" refers to an antibody comprising monkey variable regions and human constant regions. Methods for producing primatized antibodies are known in the art. See, *e.g*., U.S. patent nos. 5,658,570; 5,681,722; and 5,693,780.

The antibodies of the disclosure include derivatized antibodies. For example, but not by way of limitation, derivatized antibodies are typically modified by glycosylation, acetylation, pegylation, phosphorylation, amidation, derivatization by known protecting/blocking groups, proteolytic cleavage, linkage to a cellular ligand or other protein (see Section 5.1 for a discussion of antibody conjugates), *etc.* Any of numerous chemical modifications can be carried out by known techniques, including, but not limited to, specific chemical cleavage, acetylation, formylation, metabolic synthesis of tunicamycin, *etc.* Additionally, the derivative can contain one or more non-natural amino acids, *e.g.,* using ambrx technology (See, *e.g.,* Wolfson, 2006, Chem. Biol. 13(10):1011-2).

In yet another embodiment of the disclosure, the antibodies or fragments thereof can be antibodies or antibody fragments whose sequence has been modified to alter at least one constant region-mediated biological effector function relative to the corresponding wild type sequence. For example, in some embodiments, an antibody of the disclosure can be modified to reduce at least one constant region-mediated biological effector function relative to an unmodified antibody, *e.g*., reduced binding to the Fc receptor (FcγR) or to C1q. FcγR and C1q binding can be reduced by mutating the immunoglobulin constant region segment of the antibody at particular regions necessary for FcγR or C1q interactions (See, *e.g*., Canfield and Morrison, 1991, J. Exp. Med. 173:1483-1491; Lund et al., 1991, J. Immunol. 147:2657-2662; Lo. et al., 2017, J Biol Chem 292: 3900-08; Wang et al., 2018, Protein Cell 9:63-73).

Reduction in FcγR binding ability of the antibody can also reduce other effector functions which rely on FcγR interactions, such as opsonization, phagocytosis and antibody-dependent cellular cytotoxicity ("ADCC"), while reduction of C1q binding can reduce complement-dependent cytotoxicity ("CDCC"). Reduction or elimination of effector function can thus prevent target cells targeted by a drug conjugate of the disclosure from being destroyed via ADCC or CDCC. Accordingly, in some embodiments, effector function of an antibody is modified by selective mutation of the Fc portion of the antibody, so that it maintains antigen specificity and internalization capacity but eliminates ADCC/CDCC function. In other embodiments, effector function of an antibody is not modified to reduce or eliminate ADCC/CDCC function. Without being bound by theory, it is believed that TDCs of the disclosure comprising an antibody or antibody fragment with ADCC/CDCC function can enhance the therapeutic effect of inhibiting TGFβ signaling by promoting apoptosis of target cells, thereby further inhibiting fibrogenesis.

Numerous mutations have been described in the art for reducing FcγR and C1q binding and such mutations can be included in a drug conjugate of the disclosure. For example, U.S. Pat. No. 6,737,056 discloses that single position Fc region amino acid modifications at positions 238, 265, 269, 270, 292, 294, 295, 298, 303, 324, 327, 329, 333, 335, 338, 373, 376, 414, 416, 419, 435, 438 or 439 result in reduced binding to FcγRII and FcγRII. U.S. patent no. 9,790,268 discloses that an asparagine residue at amino acid position 298 and a serine or threonine residue at amino acid position 300 reduce FcγR binding. PCT publication no. WO 2014/190441 describes modified Fc domains with reduced FcγR binding having L234D/L235E : L234R/L235R/E233K, L234D/L235E/D265S : E233K/L234R/L235R/D265S, L234D/L235E/E269K : E233K/L234R/L235R/E269K, L234D/L235E/K322A : E233K/L234R/L235R/K322A, L234D/L235E/P329W : E233K/L234R/L235R/P329W, L234D/L235E/E269K/D265S/K322A : E233K/L234R/L235R/E269K/D265S/K322A, L234D/L235E/E269K/D265S/K322E/E333K: E233K/L234R/L235R/E269K/D265S/K322E/E333K mutations, where the set of mutations preceding a semicolon is in a first Fc polypeptide and the mutations following the semicolon are in a second Fc polypeptide of an Fc dimer. Mutations that can reduce FcγR receptor binding as well as C1q binding include N297A, N297Q, N297G, D265A/N297A, D265A/N297G, L235E, L234A/L235A, and L234A/L235A/P329A (Lo. et al., 2017, J Biol Chem 292: 3900-08; Wang et al., 2018, Protein Cell 9:63-73).

As an alternative to mutating a constant region to reduce effector function, *e.g*., mutating an Fc domain as described above, effector function can be eliminated by utilizing an antibody fragment (*e.g*., a Fab, Fab', or F(ab')₂ fragment).

In other embodiments of the disclosure, an antibody or fragment thereof can be modified to acquire or improve at least one constant region-mediated biological effector function relative to an unmodified antibody, *e.g*., to enhance FcγR interactions (See, *e.g.*, US 2006/0134709). For example, an antibody of the disclosure can have a constant region that binds FcγRIIA, FcγRIIB and/or FcγRIIIA with greater affinity than the corresponding wild type constant region.

Thus, antibodies of the disclosure can have alterations in biological activity that result in decreased opsonization, phagocytosis, or ADCC. Such alterations are known in the art. For example, modifications in antibodies that reduce ADCC activity are described in U.S. patent no. 5,834,597.

In yet another aspect, the antibodies or fragments thereof can be antibodies or antibody fragments that have been modified to increase or reduce their binding affinities to the fetal Fc receptor, FcRn, for example, by mutating the immunoglobulin constant region segment at particular regions involved in FcRn interactions (See, *e.g*., WO 2005/123780). Such mutations can increase the antibody's binding to FcRn, which protects the antibody from degradation and increases its half-life.

In yet other aspects, an antibody has one or more amino acids inserted into one or more of its hypervariable regions, for example as described in Jung and Plückthun, 1997, Protein Engineering 10(9):959-966; Yazaki et al., 2004, Protein Eng. Des Sel. 17(5):481-9; and U.S. patent publication no. 2007/0280931.

The targets of the targeting moieties will depend on the desired therapeutic applications of the TDCs. The targets are molecules present on the surfaces of cells into which it is desirable to deliver ALK5 inhibitors, such as myofibroblasts or cancer associated fibroblasts, and the targeting moieties preferably internalize upon binding to the target. Internalizing targeting moieties, *e.g.,* antibodies are described in, *e.g.,* Franke et al., 2000, Cancer Biother. Radiopharm. 15:459 76; Murray, 2000, Semin. Oncol. 27:64 70; Breitling et al., Recombinant Antibodies, John Wiley, and Sons, New York, 1998). In certain embodiments, the targeting moieties do not significantly block the activity of the target cell surface molecule. For example, an agonistic antibody or fragment thereof or non-antagonistic antibody or fragment thereof can be used as a targeting moiety, for example when the target molecule is FAP or αvβ6.

The targeting moiety selectively binds to myofibroblasts, activated fibroblasts, fibroblasts transitioning to myofibroblasts, or a combination thereof, over other cell types, for example, resting fibroblasts, lung epithelial cells, hepatocytes, T cells, cells that do not express collagen and/or cells that do not express α-smooth muscle actin (αSMA). Resting, or quiescent fibroblasts can be identified as spindle-shaped single cells, while activated fibroblasts gain expression of αSMA and vimentin and become stellate in shape. Selective binding can be accomplished by targeting a cell surface molecule that is expressed on the surface of a target cell or target cells, but that has low or no expression on other cell types. Selectivity can be measured by various assays known in the art, for example, by flow cytometry. In some embodiments, the targeting moiety of a TDC of the disclosure has at least 2 fold or at least 3 fold selectivity for myofibroblasts over resting fibroblasts, for example as measured by FACS *(e.g.,* 2 to 1000 fold, 2 to 100 fold, 2 to 50 fold, 2 to 10 fold, 3 to 1000 fold, 3 to 100 fold, 3 to 50 fold, 3 to 10 fold, 5 to 1000 fold, 5 to 100 fold, 5 to 50 fold, 5 to 10 fold, 20 to 1000 fold, 20 to 100 fold, 20 to 50 fold, 50 to 1000 fold, 50 to 100 fold, 100 to 1000 fold or more than 1000 fold). In some embodiments, the targeting moiety of a TDC of the disclosure has at least 2 fold or at least 3 fold selectivity for activated fibroblasts (*e.g*., CAFs) over resting fibroblasts, for example as measured by FACS (*e.g*., 2 to 1000 fold, 2 to 100 fold, 2 to 50 fold, 2 to 10 fold, 3 to 1000 fold, 3 to 100 fold, 3 to 50 fold, 3 to 10 fold, 5 to 1000 fold, 5 to 100 fold, 5 to 50 fold, 5 to 10 fold, 20 to 1000 fold, 20 to 100 fold, 20 to 50 fold, 50 to 1000 fold, 50 to 100 fold, 100 to 1000 fold or more than 1000 fold). In some embodiments, the targeting moiety of a TDC of the disclosure has at least 2 fold or at least 3 fold selectivity for fibroblasts transitioning to myofibroblasts over resting fibroblasts, for example as measured by FACS (*e.g.*, 2 to 1000 fold, 2 to 100 fold, 2 to 50 fold, 2 to 10 fold, 3 to 1000 fold, 3 to 100 fold, 3 to 50 fold, 3 to 10 fold, 5 to 1000 fold, 5 to 100 fold, 5 to 50 fold, 5 to 10 fold, 20 to 1000 fold, 20 to 100 fold, 20 to 50 fold, 50 to 1000 fold, 50 to 100 fold, 100 to 1000 fold or more than 1000 fold). Examples of cell surface molecules suitable for targeting by a targeting moiety include, but are not limited to, fibroblast activation protein (FAP), platelet-derived growth factor receptor beta (PDGFR-β), fibroblast growth factor receptor 1 (FGFR1), peroxisome proliferator-activated receptor gamma (PPAR-γ), fibroblast-specific protein 1 (FSP1), glial fibrillary acidic protein (GFAP), fascin, CD147, C-X-C chemokine receptor type 4 (CXCR4) alpha V beta6 (αvβ6), AXL, and MERTK. AXL and MERTK are members of the TAM receptor kinase family. A further example of a cell surface molecule suitable for targeting by a targeting moiety is Leucine Rich Repeat Containing 15 (LRRC15).

In some embodiments, a targeting moiety of a TDC of the disclosure binds to FAP. In other embodiments, a targeting moiety of a TDC of the disclosure binds to PDGFR-β. In other embodiments, a targeting moiety of a TDC of the disclosure binds to FGFR1. In other embodiments, a targeting moiety of a TDC of the disclosure binds to PPAR-γ. In other embodiments, a targeting moiety of a TDC of the disclosure binds to FSP1. In other embodiments, a targeting moiety of a TDC of the disclosure binds to GFAP. In other embodiments, a targeting moiety of a TDC of the disclosure binds to fascin. In other embodiments, a targeting moiety of a TDC of the disclosure binds to CD147. In other embodiments, a targeting moiety of a TDC of the disclosure binds to CXCR4. In other embodiments, a targeting moiety of a TDC of the disclosure binds to αvβ6. In other embodiments, a targeting moiety of a TDC of the disclosure binds to AXL. In other embodiments, a targeting moiety of a TDC of the disclosure binds to MERTK. In other embodiments, a targeting moiety of a TDC of the disclosure binds to LRRC15.

Fibroblast activation protein (FAP) is a member of the dipeptidyl peptidase (DPP) family and is expressed as type II integral membrane protein. Membrane-bound FAP contains a short cytoplasmic tail (residues 1-4), a transmembrane region (residues 5-25), and an extracellular domain (residues 26-760) (www.uniprot.org/uniprot/Q12884). FAP is active as a 170 kD dimer on the cell surface but its extracellular domain is also active following cleavage from the membrane. FAP has both dipeptidase and endopeptidase activity. Like other members of the DPP family of enzymes, FAP is a prolyl specific serine protease, but FAP also having gelatinase activity, which allows it to degrade denatured collagen I and III, human fibroblast growth factor 21 (FGF-21) and human alpha2 antiplasmin. FAP is expressed during development, but only rarely in healthy adult tissues. However, elevated FAP expression in activated fibroblasts is expressed at sites of inflammation and active tissue remodeling, including wound healing, fibrosis, and cancer. Because FAP is a marker for activated fibroblasts and myofibroblasts in several disease settings, FAP-targeted therapies are not limited to cancer patients, but are broadly applicable to treat IPF and other fibrotic diseases such as NASH (liver), cardiac and/ or kidney fibrosis. Moreover, FAP expression can be increased by TGF-β, where FAP's promoter has Smad3 binding elements. Due to this limited tissue expression and localized expression in fibrotic tissues, FAP has been used for imaging as well as therapeutic targeting to diseased tissues. In cancer, FAP is highly and selectively expressed on cancer associated fibroblasts (CAFs) which support cancer growth and metastases. FAP readily internalizes into cells, making it an excellent cell targeting and delivery vehicle. Anti-FAP antibodies coupled to a cytotoxic payload demonstrated tumor clearance in combination with chemotherapy, while anti-FAP conjugated to a radionucleotide led to increased survival in murine tumor models in vivo (Ostermann et al., 2008, Clin Cancer Res, 2008. 14(14):4584-92; Fang et al., 2016, Int J Cancer, 138(4):1013-23; Fischer et al., Clin Cancer Res 18(22):6208-18). While an unconjugated humanized anti-FAP antibody, sibroluzumab did not show single agent efficacy in metastatic FAP+ cancer patients, it did specifically accumulate in the tumors and not in normal tissues and was well tolerated in patients with limited adverse events. The soluble form of FAP, also known as antiplasmin-cleaving enzyme (APCE), lacks the cytoplasmic tail and transmembrane region of membrane-bound FAP. Soluble FAP has shown to be elevated in patients with liver cirrhosis, with levels increasing with severity of disease (de Willige et al., 2013, J Thromb Haemost. 11(11):2029-36). Preferably, a targeting moiety targeting FAP preferentially binds to the membrane bound form of FAP over the soluble form. Without being bound by theory, it is believed soluble FAP can act as a sink, reducing the *in vivo* activity of a TDC which binds the soluble form of FAP as compared to a TDC which preferentially binds the membrane bound form of FAP.

Examples of antibodies that bind to FAP are described in WO 2012/020006, WO 2016/116399 (*e.g*., antibody F5), and WO 2016/110598. In some embodiments, the targeting moiety of a TDC of the disclosure comprises an anti-FAP antibody described in WO 2012/020006, WO 2016/116399, or WO 2016/110598, or a fragment thereof (*e.g*., a Fab fragment, Fab' fragment, F(ab')₂ fragment, Fv fragment, scFv fragment, dsFv fragment, or a single domain antibody). In some embodiments, the targeting moiety of a TDC of the disclosure comprises sibroluzumab (Boehringer Ingelheim) or a fragment thereof (*e.g*., a Fab fragment, Fab' fragment, F(ab')₂ fragment, Fv fragment, scFv fragment, dsFv fragment, or a single domain antibody).

Examples of antibodies that bind to PDGFR-β are described in WO 2017/106609 and WO 2014/109999. In some embodiments, the targeting moiety of a TDC of the disclosure comprises an anti- PDGFR-β antibody described in WO 2017/106609 or WO 2014/109999, or a fragment thereof (*e.g*., a Fab fragment, Fab' fragment, F(ab')₂ fragment, Fv fragment, scFv fragment, dsFv fragment, or a single domain antibody). In some embodiments, the targeting moiety of a TDC of the disclosure comprises IMC-2C5 (ImClone) or a fragment thereof (*e.g*., a Fab fragment, Fab' fragment, F(ab')₂ fragment, Fv fragment, scFv fragment, dsFv fragment, or a single domain antibody).

Examples of antibodies that bind to FGFR1 are described in WO 2018/095932 and WO 2012/125124. In some embodiments, a targeting moiety of a TDC of the disclosure comprises an anti-FGFR1 antibody described in WO 2018/095932 or WO 2012/125124 or a fragment thereof (*e.g*., a Fab fragment, Fab' fragment, F(ab')₂ fragment, Fv fragment, scFv fragment, dsFv fragment, or a single domain antibody). In some embodiments, the targeting moiety of a TDC of the disclosure comprises an antibody comprising a heavy chain having the amino acid sequence of SEQ ID NO:45 of WO 2012/125124 and light chain having the amino acid sequence of SEQ ID NO:50 of WO 2012/125124, or a fragment thereof (*e.g.*, a Fab fragment, Fab' fragment, F(ab')₂ fragment, Fv fragment, scFv fragment, dsFv fragment, or a single domain antibody).

Examples of antibodies that bind to PPAR-γ are described in WO 2005/026336. In some embodiments, the targeting moiety of a TDC of the disclosure comprises an anti-PPAR-γ antibody described in WO 2005/026336 or a fragment thereof (*e.g*., a Fab fragment, Fab' fragment, F(ab')₂ fragment, Fv fragment, scFv fragment, dsFv fragment, or a single domain antibody). In some embodiments, the targeting moiety of a TDC of the disclosure comprises the antibody designated Pγ48.34A in WO 2005/026336, or a fragment thereof (*e.g*., a Fab fragment, Fab' fragment, F(ab')₂ fragment, Fv fragment, scFv fragment, dsFv fragment, or a single domain antibody).

Examples of antibodies that bind to FSP1 are described in WO 2011/157724. In some embodiments, the targeting moiety of a TDC of the disclosure comprises an anti-FSP1 antibody described in WO 2011/157724 or a fragment thereof (*e.g*., a Fab fragment, Fab' fragment, F(ab')₂ fragment, Fv fragment, scFv fragment, dsFv fragment, or a single domain antibody). In some embodiments, the targeting moiety of a TDC of the disclosure comprises antibody MAB4137 (R&D Systems) or a fragment thereof (*e.g*., a Fab fragment, Fab' fragment, F(ab')₂ fragment, Fv fragment, scFv fragment, dsFv fragment, or a single domain antibody).

Examples of antibodies that bind to GFAP are described in WO 2018/081649. In some embodiments, the targeting moiety of a TDC of the disclosure comprises an anti-GFAP antibody described in WO 2018/081649 or a fragment thereof (*e.g*., a Fab fragment, Fab' fragment, F(ab')₂ fragment, Fv fragment, scFv fragment, dsFv fragment, or a single domain antibody). In some embodiments, the targeting moiety of a TDC of the disclosure comprises one of the antibodies designated GFAP-1 to GFAP-19 in WO 2018/081649, or a fragment thereof (*e.g*., a Fab fragment, Fab' fragment, F(ab')₂ fragment, Fv fragment, scFv fragment, dsFv fragment, or a single domain antibody).

Examples of antibodies that bind to fascin include FCN01 (ThermoFisher), ab126772 (Abcam), and ab183891 (Abcam). In some embodiments, the targeting moiety of a TDC of the disclosure comprises one of FCN01, ab126772 or ab183891, or a fragment thereof (*e.g*., a Fab fragment, Fab' fragment, F(ab')₂ fragment, Fv fragment, scFv fragment, dsFv fragment, or a single domain antibody).

Examples of antibodies that bind to CD147 are described in WO 2015/160853, WO 2018/121578, and WO 2018/165619. In some embodiments, the targeting moiety of a TDC of the disclosure comprises an anti-CD147 antibody described in WO 2015/160853, WO 2018/121578, or WO 2018/165619 or a fragment thereof (*e.g.*, a Fab fragment, Fab' fragment, F(ab')₂ fragment, Fv fragment, scFv fragment, dsFv fragment, or a single domain antibody). In some embodiments, the targeting moiety of a TDC of the disclosure comprises the antibody designated 3A11 in WO 2018/165619 or a humanized variant described in WO 2018/165619, or a fragment of such antibody (*e.g*., a Fab fragment, Fab' fragment, F(ab')₂ fragment, Fv fragment, scFv fragment, dsFv fragment, or a single domain antibody).

Examples of antibodies that bind to CXCR4 are described in WO 2011/098762, WO 2008/060367, and WO 2006/089141. In some embodiments, the targeting moiety of a TDC of the disclosure comprises an anti-CXCR4 antibody described in WO 2011/098762, WO 2008/060367, or WO 2006/089141 or a fragment thereof (*e.g*., a Fab fragment, Fab' fragment, F(ab')₂ fragment, Fv fragment, scFv fragment, dsFv fragment, or a single domain antibody). In some embodiments, the targeting moiety of a TDC of the disclosure comprises antibody C-9P21, B-1M22, C-1I24, D-1K21 or 9N10 described in WO 2011/098762, or a fragment thereof (*e.g*., a Fab fragment, Fab' fragment, F(ab')₂ fragment, Fv fragment, scFv fragment, dsFv fragment, or a single domain antibody).

Examples of antibodies that bind to αvβ6 are described in WO 2008/112004 and WO 2013/123152. In some embodiments, the targeting moiety of a TDC of the disclosure comprises an anti- αvβ6 antibody described in WO 2008/112004 or WO 2013/123152, or a fragment thereof (*e.g*., a Fab fragment, Fab' fragment, F(ab')₂ fragment, Fv fragment, scFv fragment, dsFv fragment, or a single domain antibody). In some embodiments, the targeting moiety of a TDC of the disclosure comprises antibody STX-100 (Biogen) or a fragment thereof (*e.g*., a Fab fragment, Fab' fragment, F(ab')₂ fragment, Fv fragment, scFv fragment, dsFv fragment, or a single domain antibody).

Examples of antibodies that bind to AXL are described in WO 2009/062690, WO 2010/130751, WO 2015/193430, and WO 2016/005593. In some embodiments, the targeting moiety of a TDC of the disclosure comprises an anti-AXL antibody described in WO 2009/062690, WO 2010/130751, WO 2015/193430, or WO 2016/005593, or a fragment thereof (*e.g*., a Fab fragment, Fab' fragment, F(ab')₂ fragment, Fv fragment, scFv fragment, dsFv fragment, or a single domain antibody). In some embodiments, the targeting moiety of a TDC of the disclosure comprises the antibody of ADCT-601 (ADC Therapeutics) or a fragment thereof (*e.g*., a Fab fragment, Fab' fragment, F(ab')₂ fragment, Fv fragment, scFv fragment, dsFv fragment, or a single domain antibody).

Examples of antibodies that bind to MERTK are described in WO 2016/106221, WO 2019/005756, and WO 2019/084307. In some embodiments, the targeting moiety of a TDC of the disclosure comprises an anti-MERTK antibody described in WO 2016/106221, WO 2019/005756, or WO 2019/084307, or a fragment thereof (*e.g.*, a Fab fragment, Fab' fragment, F(ab')₂ fragment, Fv fragment, scFv fragment, dsFv fragment, or a single domain antibody). In some embodiments, the targeting moiety of a TDC of the disclosure comprises antibody RGX-019 (Rgenix) or a fragment thereof (*e.g*., a Fab fragment, Fab' fragment, F(ab')₂ fragment, Fv fragment, scFv fragment, dsFv fragment, or a single domain antibody).

LRRC15 is expressed on cancer associated fibroblasts in many cancers, for example breast cancer, head and neck cancer, lung cancer, pancreatic cancer, ovarian cancer, colon cancer, renal cancer, esophageal cancer, stomach adenocarcinoma cancer and bladder cancer (Purcell et al., 2018, Cancer Res. 78(14):4059-4072; Dominguez et al., 2019, Cancer Discovery 10(2):232-253). Thus, in some embodiments, a TDC of the disclosure targets LRRC15. Examples of antibodies that bind to LRRC15 are described in WO 2017/095805. Antibodies that bind to LRRC15 are also commercially available, *e.g*., Abcam catalog # ab150376 and Creative Biolabs catalog # TAB-0709CL. ABBV-085 (Abbvie) is a MMAE containing ADC directed against LRRC15 (Purcell et al., 2018, Cancer Res. 78(14):4059-4072). In some embodiments, the targeting moiety of a TDC of the disclosure comprises an antibody described in WO 2017/095805, the antibody of ABBV-085, one of the commercially available antibodies described in this paragraph, or a fragment thereof (*e.g*., a Fab fragment, Fab' fragment, F(ab')₂ fragment, Fv fragment, scFv fragment, dsFv fragment, or a single domain antibody).

### 5.3. The ALK5 Inhibitor

The ALK5 inhibitors of the disclosure are small molecules that competitively and reversibly bind to ATP binding site in the cytoplasmic kinase domain of the ALK5 receptor.

The ALK5 inhibitors may but not need be specific or selective for ALK5 vs. other TGF-β family receptors, such as ALK4 and/or ALK7 and/or TGF-β receptor II. In some embodiments, the ALK5 inhibitors have activity towards both ALK5 and TGF-β receptor II. In some embodiments, the ALK5 inhibitor has limited inhibitory activity towards the BMP II receptor.

The ALK5 inhibitors of the disclosure preferably have an IC₅₀ of 100 nM or less, more preferably 50 nM or less, and most preferably 20 nM or less when measured in an *in vitro* cellular assay using HEK293T cells. An exemplary cellular assay set forth in Section 6.6 below.

The ALK5 inhibitor is the compound designated C in Table 1 below:

| **Table 1** | | |
|---|---|---|
| **Designation** | **Structure** | **Name** |
| A | 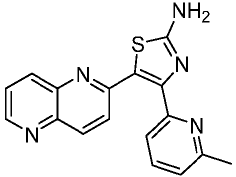 | 4-(6-methylpyridin-2-yl)-5-(1,5-naphthyridin-2-yl)thiazol-2-amine |
| B | 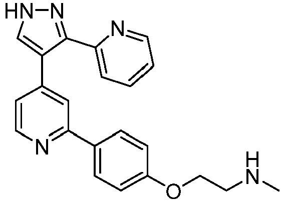 | N-methyl-2-(4-(4-(3-(pyridin-2-yl)-1H-pyrazol-4-yl)pyridin-2-yl)phenoxy)ethan-1-amine |
| C | 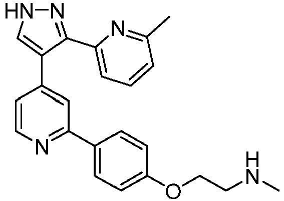 | N-methyl-2-(4-(4-(3-(6-methylpyridin-2-yl)-1H-pyrazol-4-yl)pyridin-2-yl)phenoxy)ethan-1-amine |
| D | 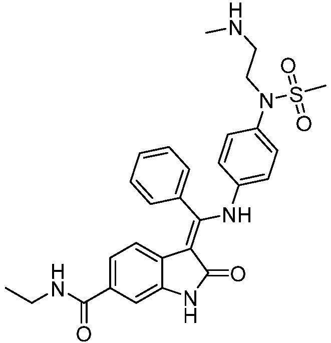 | (Z)-N-ethyl-3-(((4-(N-(2-(methylamino)ethyl)methylsulf onamido)phenyl)amino)(phenyl )methylene)-2-oxoindoline-6-carboxamide |
| E | 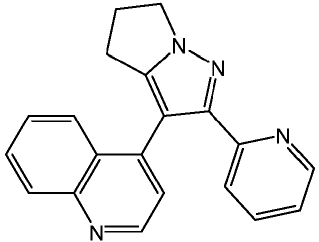 | 4-(2-(pyridin-2-yl)-5,6-dihydro-4H-pyrrolo[1,2-b]pyrazol-3-yl)quinoline |
| F | 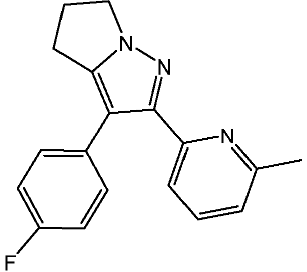 | 3-(4-fluorophenyl)-2-(6-methylpyridin-2-yl)-5,6-dihydro-4H-pyrrolo[1,2-b]pyrazole |
| G | 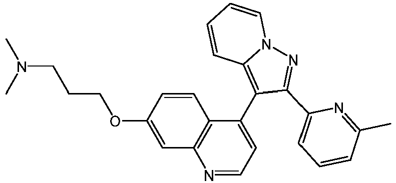 | N,N-dimethyl-3-((4-(2-(6-methylpyridin-2-yl)pyrazolo[1,5-a]pyridin-3-yl)quinolin-7-yl)oxy)propan-1-amine |
| H | 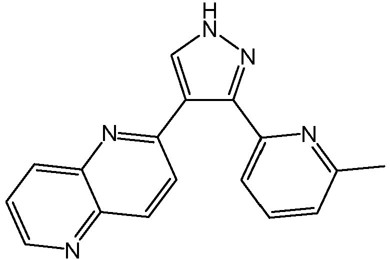 | 2-(3-(6-methylpyridin-2-yl)-1H-pyrazol-4-yl)-1,5-naphthyridine |
| I | 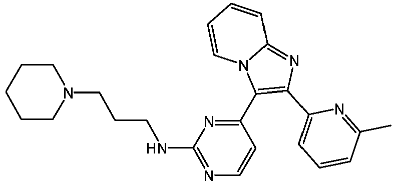 | 4-(2-(6-methylpyridin-2-yl)imidazo[1,2-a]pyridin-3-yl)-N-(3-(piperidin-1-yl)propyl)pyrimidin-2-amine |
| J | 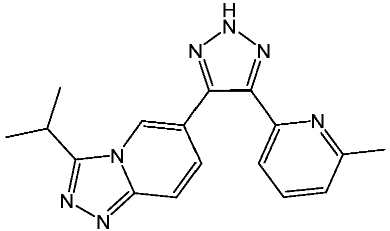 | 3-isopropyl-6-(5-(6-methylpyridin-2-yl)-2H-1,2,3-triazol-4-yl)-[1,2,4]triazolo[4,3-a]pyridine |
| K | 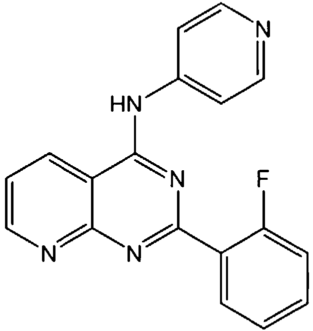 | 2-(2-fluorophenyl)-N-(pyridin-4-yl) pyrido[2,3-d] pyrimidin-4-amine |
| L | 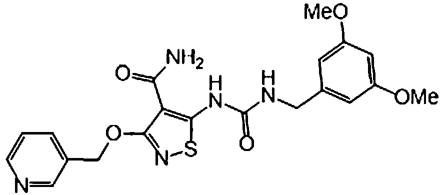 | 5-(3-(2,5-dimethoxybenzyl)ureido)-3-(pyridin-3-ylmethoxy)isothiazole-4-carboxamide |
| M | 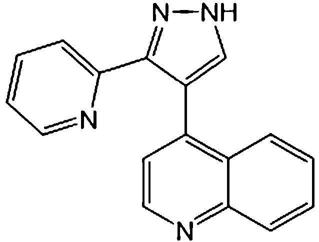 | 4-(3-(pyridin-2-yl)-1H-pyrazol-4-yl)quinolone |
| N | 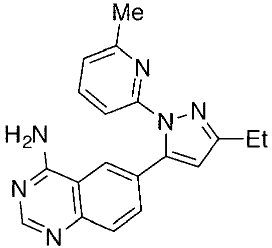 | |

The preparation and use of ALK5 inhibitors is well-known and well-documented in the scientific and patent literature. PCT publication no. WO 2000/61576 and U.S. patent publication no. US 2003/0149277 disclose triarylimidazole derivatives and their use as ALK5 inhibitors. PCT publication no. WO 2001/62756 discloses pyridinylimidazole derivatives and their use as ALK5 inhibitors. PCT publication no. WO 2002/055077 discloses use of imidazolyl cyclic acetal derivatives as ALK5 inhibitors. PCT publication no. WO 2003/087304 discloses tri-substituted heteroaryls and their use as ALK5 and/or ALK4 inhibitors. WO 2005/103028, U.S. patent publication no. US 2008/0319012 and U.S. patent no. 7,407,958 disclose 2-pyridyl substituted imidazoles as ALK5 and/or ALK4 inhibitors. One of the representative compounds, IN-1130, shows ALK5 and/or ALK4 inhibitor activity in several animal models. The following patents and patent publications provide additional examples of ALK5 inhibitors and provide illustrative synthesis schemes and methods of using ALK5 inhibitors: U.S. patent nos. 6,465,493; 6,906,089; 7,365,066; 7,087,626; 7,368,445; 7,265,225; 7,405,299; 7,407,958; 7,511,056; 7,612,094; 7,691,865; 7,863,288; 8,410,146; 8,410,146; 8,420,685; 8,513,2228,614,226; 8,791,113; 8,815,893; 8,846,931;8,912,216; 8,987,301; 9,051,307; 9,051,318; 9,073,918 and PCT publication nos. WO 2004/065392; WO 2009/050183; WO 2009/133070; WO 2011/146287; and WO 2013/009140.

Several ALK5 inhibitors are commercially available, including SB-525334 (CAS 356559-20-1), SB-505124 (CAS 694433-59-5), SB-431542 (CAS 301836-41-9), SB-202474 (EMD4 Biosciences Merck KGaA, Darmstadt, Germany), LY-364947 (CAS 396129-53-6), IN-1130, GW-788388 and D4476 (EMD4 Biosciences Merck KGaA, Darmstadt, Germany).

The structures and names of ALK5 inhibitors described herein refer to the molecule prior to the attachment to the antibody and/or linker.

The ALK5 inhibitors can be attached to a linker via a free NH or NH₂ group, preferably an NH or NH₂ group attached to or part of an alkyl, heteroaryl, or aryl group. The ALK5 inhibitors can be derivatized to add a free NH or NH₂ group. Design of derivatized ALK5 inhibitors should preferably take into account the inhibitors' structure activity relationships (SAR) to reduce the likelihood of abolishing inhibitory activity when adding an NH or NH₂ group, although the activity may also be determined empirically. Derivatized counterparts of several compounds shown in Table 1 are shown below in Table 2.

| **Table 2** | | |
|---|---|---|
| **Table 1 Designation** | **Derivative 1** | **Derivative 2** |
| A | 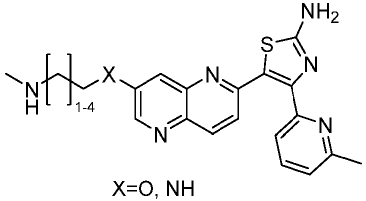 | 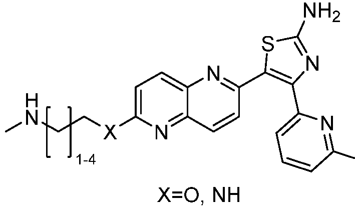 |
| E | 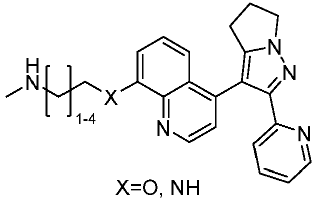 | 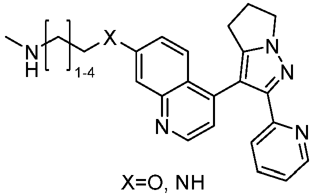 |
| F | 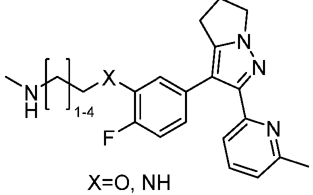 | |
| H | 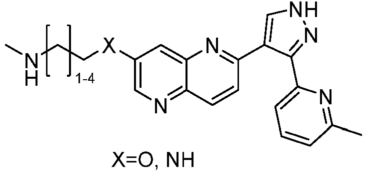 | 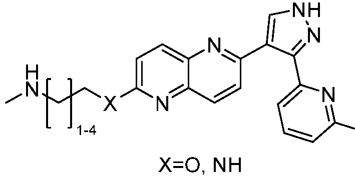 |
| L | 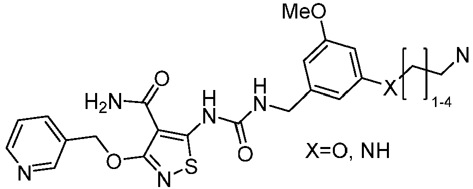 | |
| M | 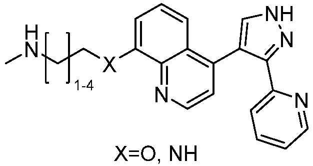 | 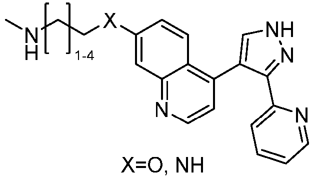 |

### 5.4. Linkers

Typically, the TDCs comprise a linker between the ALK5 inhibitor and the targeting moiety. Linkers are moieties comprising a covalent bond or a chain of atoms that covalently attaches a targeting moiety to a drug moiety. In various embodiments, linkers include a divalent radical such as an alkyldiyl, an aryldiyl, a heteroaryldiyl, moieties such as:-(CR₂)ₙO(CR₂)ₙ-, repeating units of alkyloxy (*e.g.,* polyethylenoxy, PEG, polymethyleneoxy) and alkylamino (*e.g.,* polyethyleneamino, Jeffamine^{™}); and diacid ester and amides including succinate, succinamide, diglycolate, malonate, and caproamide. For example, various PEG containing linkers are known in the art and commercially available (*e.g*., from BroadPharm (broadpharm.com). Exemplary PEG containing linkers include Mal-PEG2-Val-Cit-PAB-OH (BroadPharm cat. no. BP-23203), Mal-PEG4-Val-Cit-PAB-OH (BroadPharm cat. no. BP-23204), Mal-PEG4-Val-Cit-PAB-PNP (BroadPharm cat. no. BP-23668), Mal-amido-PEG2-Val-Cit-PAB-PNP (BroadPharm cat. no. BP-23675), Azido-PEG3-Val-Cit-PAB-OH (BroadPharm cat. no. BP-23206), Azido-PEG4-Val-Cit-PAB-OH (BroadPharm cat. no. BP-23207), Azido-PEG3-Val-Cit-PAB-PNP (BroadPharm cat. no. BP-23368), Fmoc-PEG4-Ala-Ala-Asn-PAB (BP-23328), Azido-PEG5-Ala-Ala-Asn-PAB (BroadPharm cat. no. BP-23329), Fmoc-PEG3-Ala-Ala-Asn(Trt)-PAB (BroadPharm cat no. BP-23285), Azido-PEG4-Ala-Ala-Asn(Trt)-PAB (BroadPharm cat no. BP-23284), and Fmoc-PEG3-Ala-Ala-Asn(Trt)-PAB-PNP (BroadPharm cat no. BP-23297). In some embodiments, a TDC linker comprises a PEG and a peptide, *e.g.*, one of the dipeptides described in this section such as Val-Cit.

A linker can comprise one or more linker components, such as stretcher and spacer moieties. For example, a peptidyl linker can comprise a peptidyl component of two or more amino acids and, optionally, one or more stretcher and/or spacer moieties. Various linker components are known in the art, some of which are described below.

A linker can be a "cleavable linker," facilitating release of a drug in the cell. For example, an acid-labile linker (*e.g.,* hydrazone), protease-sensitive (*e.g.,* peptidase-sensitive) linker, photolabile linker, dimethyl linker or disulfide-containing linker (Chari et al., 1992, Cancer Research 52:127-131; U.S. patent no. 5,208,020) can be used.

Examples of linkers and linker components known in the art include maleimidocaproyl (mc); maleimidocaproyl-p-aminobenzylcarbamate; maleimidocaproyl-peptide-aminobenzylcarbamate linkers, *e.g*., maleimidocaproyl-L-phenylalanine-L-lysine-p-aminobenzylcarbamate and maleimidocaproyl-L-valine-L-citrulline-p-aminobenzylcarbamate (vc); N-succinimidyl 3-(2-pyridyldithio)proprionate (also known as N-succinimidyl 4-(2-pyridyldithio)pentanoate or SPP); 4-succinimidyl-oxycarbonyl-2-methyl-2-(2-pyridyldithio)-toluene (SMPT); N-succinimidyl 3-(2-pyridyldithio)propionate (SPDP); N-succinimidyl 4-(2-pyridyldithio)butyrate (SPDB); 2-iminothiolane; S-acetylsuccinic anhydride; disulfide benzyl carbamate; carbonate; hydrazone linkers; N-(α-Maleimidoacetoxy)succinimide ester; N-[4-(p-Azidosalicylamido)butyl]-3'-(2'-pyridyldithio)propionamide (AMAS); N[β-Maleimidopropyloxy]succinimide ester (BMPS); [N-ε-Maleimidocaproyloxy]succinimide ester (EMCS); N-[γ-Maleimidobutyryloxy]succinimide ester (GMBS); Succinimidyl-4-[N-Maleimidomethyl]cyclohexane-1-carboxy-[6-amidocaproate] (LC-SMCC); Succinimidyl 6-(3-[2-pyridyldithio]-propionamido)hexanoate (LC-SPDP); m-Maleimidobenzoyl-N-hydroxysuccinimide ester (MBS); N-Succinimidyl[4-iodoacetyl]aminobenzoate (SIAB); Succinimidyl 4-[N-malei midomethyl]cyclohexane-1-carboxylate (SMCC); N-Succinimidyl 3-[2-pyridyldithio]-propionamido (SPDP); [N-ε-Maleimidocaproyloxy]sulfosuccinimide ester (Sulfo-EMCS); N-[γ-Maleimidobutyryloxy]sulfosuccinimide ester (Sulfo-GMBS); 4-Sulfosuccinimidyl-6-methyl-α-(2-pyridyldithio)toluamido]hexanoate-) (Sulfo-LC-SMPT); Sulfosuccinimidyl 6-(3'-[2-pyridyldithio]-propionamido)hexanoate (Sulfo-LC-SPDP); m-Maleimidobenzoyl-N-hydroxysulfosuccinimide ester (Sulfo-MBS); N-Sulfosuccinimidyl[4-iodoacetyl]aminobenzoate (Sulfo-SIAB); Sulfosuccinimidyl 4-[N-maleimidomethyl]cyclohexane-1-carboxylate (Sulfo-SMCC); Sulfosuccinimidyl 4-[p-maleimidophenyl]butyrate (Sulfo-SMPB); ethylene glycol-bis(succinic acid N-hydroxysuccinimide ester) (EGS); disuccinimidyl tartrate (DST); 1,4,7,10-tetraazacyclododecane-1,4,7,10-tetraacetic acid (DOTA); diethylenetriamine-pentaacetic acid (DTPA); thiourea linkers; and oxime containing linkers.

In some embodiments, the linker is cleavable under intracellular or extracellular conditions, such that cleavage of the linker releases the ALK5 inhibitor from the targeting moiety in the appropriate environment. In yet other embodiments, the linker is not cleavable and the drug is released, for example, by targeting moiety degradation in lysosomes (see U.S. patent publication 2005/0238649).

Examples of non-cleavable linkers that can be used in the TDCs of the disclosure include N-maleimidomethylcyclohexane1-carboxylate, maleimidocaproyl or mercaptoacetamidocaproyl linkers.

In some embodiments, the linker is cleavable by a cleaving agent that is present in the intracellular environment (for example, within a lysosome or endosome or caveolea). The linker can be, for example, a peptidyl linker that is cleaved by an intracellular peptidase or protease enzyme, including, but not limited to, a lysosomal or endosomal protease. In some embodiments, the peptidyl linker comprises a peptidyl component that is at least two amino acids long or at least three amino acids long or more.

Cleaving agents can include, without limitation, cathepsins B and D and plasmin, all of which are known to hydrolyze dipeptide drug derivatives resulting in the release of active drug inside target cells (see, *e.g*., Dubowchik and Walker, 1999, Pharm. Therapeutics 83:67-123). For example, a peptidyl linker that is cleavable by the thiol-dependent protease cathepsin-B (*e.g*., a Phe-Leu or a Gly-Phe-Leu-Gly linker). Other examples of such linkers are described, *e.g*., in U.S. patent no. 6,214,345.

In some embodiments, the peptidyl linker cleavable by an intracellular protease is a Val-Cit linker or a Phe-Lys linker (see, *e.g*., U.S. patent no. 6,214,345, which describes the synthesis of doxorubicin with the val-cit linker).

In other embodiments, the cleavable linker is pH-sensitive, that is, sensitive to hydrolysis at certain pH values. Typically, the pH-sensitive linker hydrolyzable under acidic conditions. For example, an acid-labile linker that is hydrolyzable in the lysosome (for example, a hydrazone, semicarbazone, thiosemicarbazone, cis-aconitic amide, orthoester, acetal, ketal, or the like) may be used. (See, *e.g*., U.S. patent nos. 5,122,368; 5,824,805; 5,622,929; Dubowchik and Walker, 1999, Pharm. Therapeutics 83:67-123; Neville et al., 1989, Biol. Chem. 264:14653-14661.) Such linkers are relatively stable under neutral pH conditions, such as those in the blood, but are unstable at below pH 5.5 or 5.0, the approximate pH of the lysosome. In certain embodiments, the hydrolyzable linker is a thioether linker (such as, *e.g*., a thioether attached to the therapeutic agent via an acylhydrazone bond (see, *e.g*., U.S. patent no. 5,622,929).

In yet other embodiments, the linker is cleavable under reducing conditions (for example, a disulfide linker). A variety of disulfide linkers are known in the art, including, for example, those that can be formed using SATA (N-succinimidyl-5-acetylthioacetate), SPDP (N-succinimidyl-3-(2-pyridyldithio)propionate), SPDB (N-succinimidyl-3-(2-pyridyldithio)butyrate) and SMPT (N-succinimidyl-oxycarbonyl-alpha-methyl-alpha-(2-pyridyl-dithio)toluene)-, SPDB and SMPT. (See, *e.g.,* Thorpe et al., 1987, Cancer Res. 47:5924-5931; Wawrzynczak et al., In Immunoconjugates: Antibody Conjugates in Radioimagery and Therapy of Cancer (C. W. Vogel ed., Oxford U. Press, 1987. See also U.S. patent no. 4,880,935.)

In other embodiments, the linker is a malonate linker (Johnson et al., 1995, Anticancer Res. 15:1387-93), a maleimidobenzoyl linker (Lau et al., 1995, Bioorg-Med-Chem. 3(10):1299-1304), or a 3'-N-amide analog (Lau et al., 1995, Bioorg-Med-Chem. 3(10):1305-12).

In some embodiments, the linker is a polyvalent linker that can be used to link many drug molecules to a single targeting moiety molecule (*e.g*., a single antibody molecule). For example, the Fleximer linker technology developed by Mersana is based on incorporating drug molecules into a solubilizing poly-acetal backbone via a sequence of ester bonds. The methodology enables highly-loaded TDCs (*e.g.,* having a drug antibody ratio (DAR) up to 20) while maintaining good physicochemical properties. Exemplary polyvalent linker are described, for example, in WO 2009/073445; WO 2010/068795; WO 2010/138719; WO 2011/120053; WO 2011/171020; WO 2013/096901; WO 2014/008375; WO 2014/093379; WO 2014/093394; and WO 2014/093640.

Often the linker is not substantially sensitive to the extracellular environment. As used herein, "not substantially sensitive to the extracellular environment," in the context of a linker, means that no more than about 20%, 15%, 10%, 5%, 3%, or no more than about 1% of the linkers, in a sample of TDC, are cleaved when the TDC presents in an extracellular environment (for example, in plasma).

Whether a linker is not substantially sensitive to the extracellular environment can be determined, for example, by incubating with plasma the TDC for a predetermined time period (for example, 2, 4, 8, 16, or 24 hours) and then quantitating the amount of free drug present in the plasma.

In other, non-mutually exclusive embodiments, the linker can promote cellular internalization. In certain embodiments, the linker promotes cellular internalization when conjugated to the therapeutic agent (that is, in the milieu of the linker-therapeutic agent moiety of the TDC as described herein). In yet other embodiments, the linker promotes cellular internalization when conjugated to both the ALK5 inhibitor and the antibody.

In many embodiments, the linker is self-immolative. As used herein, the term "self-immolative" refers to a bifunctional chemical moiety that is capable of covalently linking together two spaced chemical moieties into a stable tripartite molecule. It will spontaneously separate from the second chemical moiety if its bond to the first moiety is cleaved. See for example, PCT publication nos. WO 2007/059404, WO 2006/110476, WO 2005/112919, WO 2010/062171, WO 2009/017394, WO 2007/089149, WO 2007/018431, WO 2004/043493 and WO 2002/083180, which are directed to drug-cleavable substrate conjugates where the drug and cleavable substrate are optionally linked through a self-immolative linker. Examples of self-immolative spacer units that can be used to generated self-immolative linkers are described under Formula I below.

A variety of exemplary linkers that can be used with the present compositions and methods are described in PCT publication no. WO 2004/010957, U.S. patent publication no. US 2006/0074008, U.S. patent publication no. US 2005/0238649, and U.S. patent publication no. US 2006/0024317.

A TDC of the disclosure may be of Formula I, below, wherein an antibody or other targeting moiety (shown as "Ab" in Formula I) is conjugated to one or more ALK5 inhibitor drug moieties (D) through an optional linker (L)

Ab-(L-D)ₚ I

Accordingly, the targeting moiety may be conjugated to the drug either directly or via a linker. In Formula I, p is the average number of drug (*i.e.,* ALK5 inhibitor) moieties per targeting moiety, which can range, *e.g*., from about 1 to about 20 drug moieties per targeting moiety, and in certain embodiments, from 2 to about 8 drug moieties per targeting moiety. Further details of drug loading are described in Section 5.5 below.

In some embodiments, a linker component may comprise a "stretcher" that links a targeting moiety, *e.g*., via a cysteine residue, to another linker component or to a drug moiety. Exemplary stretchers are shown below (wherein the left wavy line indicates the site of covalent attachment to a targeting moiety and the right wavy line indicates the site of covalent attachment to another linker component or drug moiety): 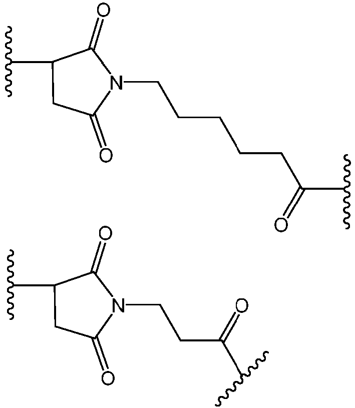 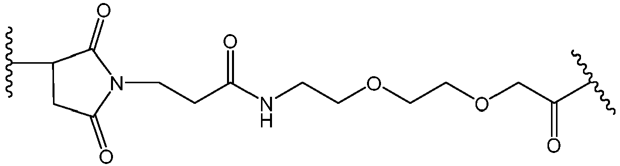 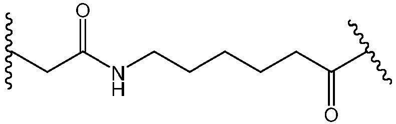 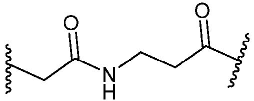 See, U.S. patent no. 9,109,035; Ducry et al., 2010, Bioconjugate Chem. 21:5-13.

In some embodiments, a linker component may comprise an amino acid unit. In one such embodiment, the amino acid unit allows for cleavage of the linker by a protease, thereby facilitating release of the drug from the TDC upon exposure to intracellular proteases, such as lysosomal enzymes. See, *e.g.,* Doronina et al., 2003, Nat. Biotechnol. 21:778-784. Exemplary amino acid units include, but are not limited to, a dipeptide, a tripeptide, a tetrapeptide, and a pentapeptide. Exemplary dipeptides include: valine-citrulline (VC or val-cit), alanine-phenylalanine (AF or ala-phe); phenylalanine-lysine (FK or phe-lys); or N-methyl-valine-citrulline (Me-val-cit). Exemplary tripeptides include: glycine-valine-citrulline (gly-val-cit) and glycine-glycine-glycine (gly-gly-gly). Exemplary tetrapeptides include glycine-glycine-phenylalanine-glycine (gly-gly-phe-gly). An amino acid unit may comprise amino acid residues that occur naturally, as well as minor amino acids and non-naturally occurring amino acid analogs, such as citrulline amino acid units can be designed and optimized in their selectivity for enzymatic cleavage by a particular enzyme, for example, cathepsin B, C and D, or a plasmin protease.

In some embodiments, a linker component may comprise a "spacer" unit that links the targeting moiety to a drug moiety, either directly or by way of a stretcher and/or an amino acid unit. A spacer unit may be "self-immolative" or a "non-self-immolative." A "non-self-immolative" spacer unit is one in which part or all of the spacer unit remains bound to the drug moiety upon enzymatic (*e.g*., proteolytic) cleavage of the TDC. Examples of non-self-immolative spacer units include, but are not limited to, a glycine spacer unit and a glycine-glycine spacer unit. A "self-immolative" spacer unit allows for release of the drug moiety without a separate hydrolysis step. In certain embodiments, a spacer unit of a linker comprises a p-aminobenzyl unit. In one such embodiment, a p-aminobenzyl alcohol is attached to an amino acid unit via an amide bond, and a carbamate, methylcarbamate, or carbonate is made between the benzyl alcohol and a cytotoxic agent. See, *e.g.,* Hamann et al., 2005, Expert Opin. Ther. Patents 15:1087-1103. In one embodiment, the spacer unit is p-aminobenzyloxycarbonyl (PAB). In certain embodiments, the phenylene portion of a p-amino benzyl unit is substituted with Qₘ, wherein Q is --C₁-C₈ alkyl, --O--(C₁-C₈ alkyl), -halogen, -nitro or -cyano; and m is an integer ranging from 0-4. Examples of self-immolative spacer units further include, but are not limited to, aromatic compounds that are electronically similar to p-aminobenzyl alcohol (see, *e.g*., U.S. patent publication no. US 2005/0256030), such as 2-aminoimidazol-5-methanol derivatives (Hay et al., 1999, Bioorg. Med. Chem. Lett. 9:2237) and ortho- or para-aminobenzylacetals. Spacers can be used that undergo cyclization upon amide bond hydrolysis, such as substituted and unsubstituted 4-aminobutyric acid amides (Rodrigues et al., 1995, Chemistry Biology 2:223); appropriately substituted bicyclo[2.2.1] and bicyclo[2.2.2] ring systems (Storm et al., 1972, Amer. Chem. Soc. 94:5815); and 2-aminophenylpropionic acid amides (Amsberry et al., 1990, J. Org. Chem. 55:5867). Elimination of amine-containing drugs that are substituted at the a-position of glycine (Kingsbury et al., 1984, J. Med. Chem. 27:1447) are also examples of self-immolative spacers useful in TDCs.

In one embodiment, a spacer unit is a branched bis(hydroxymethyl)styrene (BHMS) unit as depicted below, which can be used to incorporate and release multiple drugs. 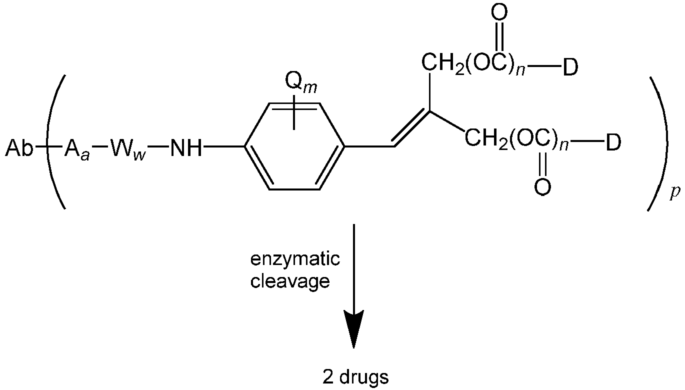 wherein Ab and D are defined as above for Formula I; A is a stretcher, and a is an integer from 0 to 1; W is an amino acid unit, and w is an integer from 0 to 12; Q is --C₁-C₈ alkyl, --O--(C₁-C₈ alkyl), -halogen, -nitro or -cyano; *m* is an integer ranging from 0-4; *n* is 0 or 1; and *p* ranges ranging from 1 to about 20.

A linker may comprise any one or more of the above linker components. In certain embodiments, a linker is as shown in brackets in the following TDC formula:

Ab-(-[A*a*-W*w*-Y*y*]-D)*ₚ* II

wherein Ab, A, a, W, w, D, and *p* are as defined in the preceding paragraph; Y is a spacer unit, and *y* is 0, 1, or 2. Exemplary embodiments of such linkers are described in U.S. patent publication no. 2005/0238649 A1.

Exemplary linker components and combinations thereof are shown below in the context of TDCs of Formula II:

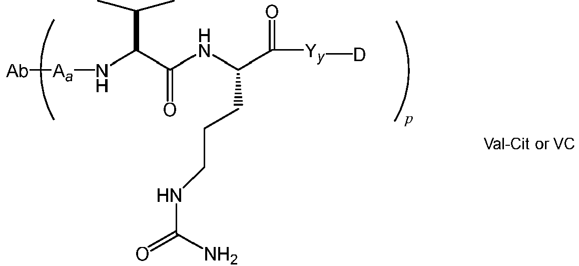

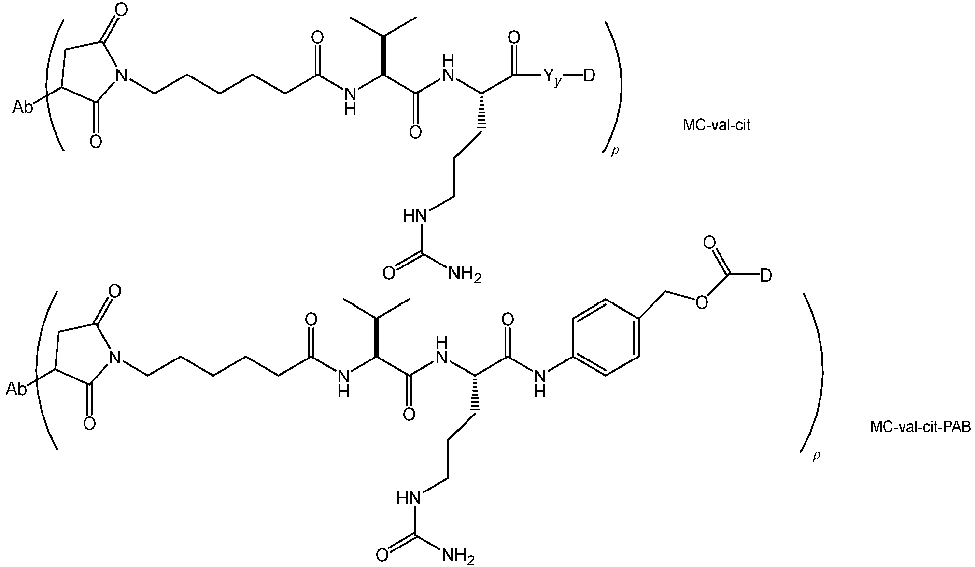

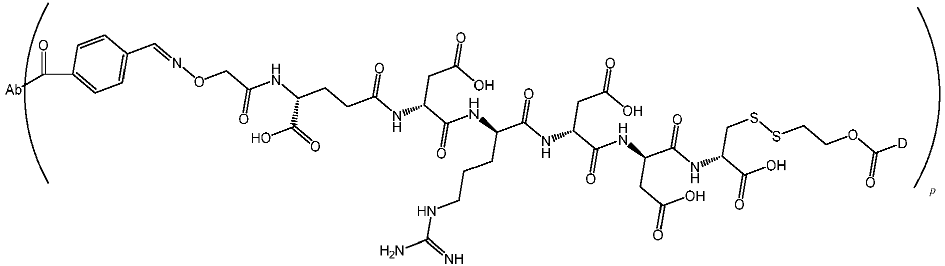

Linkers components, including stretcher, spacer, and amino acid units, may be synthesized by methods known in the art, such as those described in U.S. patent publication no. 2005/0238649.

### 5.5. Drug Loading

Drug loading is represented by p and is the average number of ALK5 inhibitor moieties per targeting moiety (*e.g*., per antibody) in a molecule. Drug loading ("p") may be 1, 2, 3, 4, 5, 6, 7, 8, 9, 10, 11, 12, 13, 14, 15, 16, 17, 18, 19, 20 or more moieties (D) per targeting moiety, although frequently the average number is a fraction or a decimal. Generally, ALK5 inhibitor loading averages from 2 to 8 drug moieties per targeting moiety, more preferably 2 to 4 drug moieties per antibody or 5 to 7 drug moieties per targeting moiety.

As would be understood by one of skill in the art, in many instances references to a TDC is shorthand for a population or collection of TDC molecules (sometimes in the context of a pharmaceutical composition), each molecule composed of a targeting moiety covalently attached to one or more ALK5 inhibitor moieties, with the drug loading ratio representing the average drug loading in the population or collection, although the ratio on an individual molecule basis may vary from one TDC molecule to another in the population. In some embodiments, the population or collection contains TDC molecules comprising an antibody covalently attached to anywhere between 1 and 30 drug moieties, and in some embodiments anywhere between 1 and 20, between 1 and 15, between 2 and 12, between 2 and 8, between 4 and 15, or between 6 and 12 drug moieties. Preferably, the average in the population is as described in the preceding paragraph, *e.g*., 2 to 8 drug moieties per targeting moiety, more preferably 4 to 8 drug moieties per targeting moiety or 5 to 7 drug moieties per targeting moiety.

Some TDC populations can be in the form of compositions comprising TDCs as described herein and targeting moiety molecules lacking drug moieties, *e.g*., antibodies to which attachment of the ALK5 inhibitor was unsuccessful.

The average number of ALK5 inhibitor moieties per targeting moiety in preparations of TDCs from conjugation reactions may be characterized by conventional means such as mass spectroscopy, hydrophobic interaction chromatography (HIC) and ELISA assays.

The quantitative distribution of TDCs in terms of p may also be determined. In some instances, separation, purification, and characterization of homogeneous TDCs where p is a certain value from TDCs with other ALK5 inhibitor loadings may be achieved by means such as electrophoresis.

For some drug conjugates, p may be limited by the number of attachment sites on the targeting moiety. For example, where the attachment is a cysteine thiol, as in the exemplary embodiments above, a targeting moiety (*e.g*., an antibody) may have only one or several cysteine thiol groups, or may have only one or several sufficiently reactive thiol groups through which a linker may be attached. In certain embodiments, higher drug loading, *e.g.,* p>5, may cause aggregation, insolubility, toxicity, or loss of cellular permeability of certain drug conjugates. In certain embodiments, the drug loading for a TDC of the disclosure ranges from 1 to about 8; from about 2 to about 6; from about 3 to about 5; from about 3 to about 4; from about 3.1 to about 3.9; from about 3.2 to about 3.8; from about 3.2 to about 3.7; from about 3.2 to about 3.6; from about 3.3 to about 3.8; or from about 3.3 to about 3.7. Indeed, it has been shown that for certain TDCs, the optimal ratio of drug moieties per antibody may be less than 8, and may be about 2 to about 5. See U.S. patent publication no. US 2005/0238649.

In certain embodiments, less than the theoretical maximum of drug moieties are conjugated to a targeting moiety during a conjugation reaction. A targeting moiety may contain, for example, lysine residues that do not react with the drug-linker intermediate or linker reagent, as discussed below. Generally, antibodies do not contain many free and reactive cysteine thiol groups which may be linked to a drug moiety; indeed most cysteine thiol residues in antibodies exist as disulfide bridges. In certain embodiments, an antibody or other targeting moiety may be reduced with a reducing agent such as dithiothreitol (DTT) or tricarbonylethylphosphine (TCEP), under partial or total reducing conditions, to generate reactive cysteine thiol groups. In certain embodiments, an antibody or other targeting moiety is subjected to denaturing conditions to reveal reactive nucleophilic groups such as lysine or cysteine.

The loading (drug/antibody ratio) of a TDC may be controlled in different ways, *e.g*., by:(i) limiting the molar excess of drug-linker intermediate or linker reagent relative to the targeting moiety, (ii) limiting the conjugation reaction time or temperature, (iii) partial or limiting reductive conditions for cysteine thiol modification, (iv) engineering by recombinant techniques the amino acid sequence of the targeting moiety such that the number and position of cysteine residues is modified for control of the number and/or position of linker-drug attachments (such as thioMab or thioFab prepared as disclosed in PCT publication no. WO 2006/034488).

It is to be understood that where more than one nucleophilic group reacts with a drug-linker intermediate or linker reagent followed by drug moiety reagent, then the resulting product is a mixture of TDC compounds with a distribution of one or more drug moieties attached to a targeting moiety. The average number of drugs per targeting moiety may be calculated from the mixture by a dual ELISA antibody assay, which is specific for targeting moiety and specific for the drug. Individual TDC molecules may be identified in the mixture by mass spectroscopy and separated by HPLC, *e.g*. hydrophobic interaction chromatography.

In some embodiments, a homogeneous TDC with a single loading value may be isolated from the conjugation mixture by electrophoresis or chromatography.

### 5.6. Formulations and Administration

Suitable routes of administration of the TDCs include, without limitation, oral, parenteral, rectal, transmucosal, intestinal administration, intramedullary, intrathecal, direct intraventricular, intravenous, intravitreal, intracavitary, intraperitoneal, or intratumoral injections. The preferred routes of administration are parenteral, more preferably intravenous. Alternatively, one may administer the compound in a local rather than systemic manner, for example, via injection of the compound directly into an area affected by fibrosis or via injection of the compound directly into a solid tumor.

Immunoconjugates can be formulated according to known methods to prepare pharmaceutically useful compositions, whereby the TDC is combined in a mixture with a pharmaceutically suitable excipient. Sterile phosphate-buffered saline is one example of a pharmaceutically suitable excipient. Other suitable excipients are well-known to those in the art. See, for example, Ansel et al., Pharmaceutical Dosage Forms And Drug Delivery Systems, 5th Edition (Lea & Febiger 1990), and Gennaro (ed.), Remington's Pharmaceutical Sciences, 18th Edition (Mack Publishing Company 1990), and revised editions thereof.

In a preferred embodiment, the TDC is formulated in Good's biological buffer (pH 6-7), using a buffer selected from the group consisting of N-(2-acetamido)-2-aminoethanesulfonic acid (ACES); N-(2-acetamido)iminodiacetic acid (ADA); N,N-bis(2-hydroxyethyl)-2-aminoethanesulfonic acid (BES); 4-(2-hydroxyethyl)piperazine-1-ethanesulfonic acid (HEPES); 2-(N-morpholino)ethanesulfonic acid (MES); 3-(N-morpholino)propanesulfonic acid (MOPS); 3-(N-morpholinyl)-2-hydroxypropanesulfonic acid (MOPSO); and piperazine-N,N'-bis(2-ethanesulfonic acid) [Pipes]. More preferred buffers are MES or MOPS, preferably in the concentration range of 20 to 100 mM, more preferably about 25 mM. Most preferred is 25 mM MES, pH 6.5. The formulation may further comprise 25 mM trehalose and 0.01% v/v polysorbate 80 as excipients, with the final buffer concentration modified to 22.25 mM as a result of added excipients. The preferred method of storage is as a lyophilized formulation of the conjugates, stored in the temperature range of -20°C to 2°C, with the most preferred storage at 2°C to 8°C.

The TDC can be formulated for intravenous administration via, for example, bolus injection, slow infusion or continuous infusion. Preferably, the TDC is infused over a period of less than about 4 hours, and more preferably, over a period of less than about 3 hours. For example, the first 25-50 mg could be infused within 30 minutes, preferably even 15 min, and the remainder infused over the next 2-3 hrs. Formulations for injection can be presented in unit dosage form, e.g., in ampoules or in multi-dose containers, with an added preservative. The compositions can take such forms as suspensions, solutions or emulsions in oily or aqueous vehicles, and can contain formulatory agents such as suspending, stabilizing and/or dispersing agents. Alternatively, the active ingredient can be in powder form for constitution with a suitable vehicle, *e.g*., sterile pyrogen-free water, before use.

Additional pharmaceutical methods may be employed to control the duration of action of the TDC. Control release preparations can be prepared through the use of polymers to complex or adsorb the TDC. For example, biocompatible polymers include matrices of poly(ethylene-co-vinyl acetate) and matrices of a polyanhydride copolymer of a stearic acid dimer and sebacic acid. Sherwood et al., 1992, Bio/Technology 10:1446. The rate of release of a TDC from such a matrix depends upon the molecular weight of the TDC, the amount of TDC within the matrix, and the size of dispersed particles. Saltzman et al., 1989, Biophys. J. 55:163; Sherwood *et al.,* supra. Other solid dosage forms are described in Ansel et al., Pharmaceutical Dosage Forms And Drug Delivery Systems, 5th Edition (Lea & Febiger 1990), and Gennaro (ed.), Remington's Pharmaceutical Sciences, 18th Edition (Mack Publishing Company 1990), and revised editions thereof.

Generally, the dosage of an administered TDC for humans will vary depending upon such factors as the patient's age, weight, height, sex, general medical condition and previous medical history. It may be desirable to provide the recipient with a dosage of TDC that is in the range of from about 0.3 mg/kg to 5 mg/kg as a single intravenous infusion, although a lower or higher dosage also may be administered as circumstances dictate. A dosage of 0.3-5 mg/kg for a 70 kg patient, for example, is 21-350 mg, or 12-20⁶ mg/m₂ for a 1.7-m patient. The dosage may be repeated as needed, for example, once per week for 2-10 weeks, once per week for 8 weeks, or once per week for 4 weeks. It may also be given less frequently, such as every other week for several months, or monthly or quarterly for many months, as needed in a maintenance therapy. Preferred dosages may include, but are not limited to, 0.3 mg/kg, 0.5 mg/kg, 0.7 mg/kg, 1.0 mg/kg, 1.2 mg/kg, 1.5 mg/kg, 2.0 mg/kg, 2.5 mg/kg, 3.0 mg/kg, 3.5 mg/kg, 4.0 mg/kg, 4.5 mg/kg, and 5.0 mg/kg. More preferred dosages are 0.6 mg/kg for weekly administration and 1.2 mg/kg for less frequent dosing. Any amount in the range of 0.3 to 5 mg/kg may be used. The dosage is preferably administered multiple times, once a week. A minimum dosage schedule of 4 weeks, more preferably 8 weeks, more preferably 16 weeks or longer may be used, with the dose frequency dependent on toxic side-effects and recovery therefrom, mostly related to hematological toxicities. The schedule of administration may comprise administration once or twice a week, for example on a cycle selected from the group consisting of:(i) weekly; (ii) every other week; (iii) one week of therapy followed by two, three or four weeks off; (iv) two weeks of therapy followed by one, two, three or four weeks off; (v) three weeks of therapy followed by one, two, three, four or five week off; (vi) four weeks of therapy followed by one, two, three, four or five week off; (vii) five weeks of therapy followed by one, two, three, four or five week off; and (viii) monthly. The cycle may be repeated 2, 4, 6, 8, 10, or 12 times or more.

Alternatively, a TDC may be administered as one dosage every 2 or 3 weeks, repeated for a total of at least 3 dosages. Or, twice per week for 4-6 weeks. The dosage may be administered once every other week or even less frequently, so the patient can recover from any drug-related toxicities. Alternatively, the dosage schedule may be decreased, namely every 2 or 3 weeks for 2-3 months. The dosing schedule can optionally be repeated at other intervals and dosage may be given through various parenteral routes, with appropriate adjustment of the dose and schedule.

### 5.7. Methods of Treatment

### 5.7.1. Fibrosis

The TDCs of the disclosure can be used for the treatment of various fibrotic conditions, for example fibrosis associated with systemic sclerosis (also known as scleroderma) or NASH. Patients with systemic sclerosis frequently suffer from lung fibrosis, skin fibrosis, and esophagus fibrosis, although fibrosis can occur in virtually any organ. Patients with NASH frequently suffer from liver fibrosis. The TDCs can be used as monotherapy or as part of a combination therapy regimen, for example with a standard of care agent or regimen. In some embodiments, the combination therapy comprises administering a TDC in combination with pirfenidone, nintedanib, pentraxin-2, pamrevlumab, prednisone, cortisone, cyclophosphamide, azathioprine , or a combination thereof. In some embodiments, the combination therapy comprises administering a TDC in combination with pirfenidone and/or nintedanib.

Examples of conditions which can be treated using the TDCs of the disclosure include but not limited to pulmonary fibrosis, *e.g.,* IPF, liver fibrosis, *e.g.,* associated with NASH, kidney fibrosis, cardiac fibrosis, skin fibrosis, esophagus fibrosis, and systemic sclerosis. The TDCs of the disclosure can be administered to a subject having, *e.g*., diagnosed with, a disease associated with fibrosis, *e.g*., systemic sclerosis or NASH, prior to the development of signs and/or symptoms of fibrosis. Alternatively, or in addition, the TDCs can be administered to subjects having, *e.g*., diagnosed with, a disease associated with fibrosis after signs and/or symptoms of fibrosis are observed.

The use of a TDC of the disclosure in combination with one or more therapies does not restrict the order in which the therapies are administered. For example, the TDC of the disclosure can be administered before, during or after a subject is treated with one or more therapies. In some embodiments, a TDC of the disclosure is administered prior to (*e.g*., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g*., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) treatment of a patient with another therapy (*e.g*., a second therapeutic agent as described above). In some embodiments, the TDC of the disclosure is incorporated into the same regimen as a second therapeutic agent.

### 5.7.2. Cancer

The TDCs of the disclosure (*e.g*., FAP targeted TDCs) can be used for the treatment of various cancers. The TDCs can be used as monotherapy or as part of a combination therapy regimen, for example with a standard of care agent or regimen. In some embodiments, the combination therapy comprises administering a TDC in combination with immunotherapy, for example, checkpoint inhibitor therapy, chimeric antigen receptor (CAR) therapy, adoptive T cell therapy (*e.g*., autologous T cell therapy), oncolytic virus therapy, dendritic cell vaccine therapy, stimulator of interferon genes (STING) agonist therapy, toll-like receptor (TLR) agonist therapy, intratumoral CpG therapy, cytokine therapy (*e.g*., IL2, IL12, IFN-α, or INF-γ therapy), or a combination thereof. In some embodiments, the combination therapy comprises administering a TDC in combination an ADC having a cytotoxic payload, for example, a FAP targeting ADC such as OMTX705 (Oncomatryx). In some embodiments, the combination therapy comprises administering a TDC in combination with immune preserving chemotherapy (*e*.*g*., an antimetabolite, such as 5-fluorouracil, gemcitabine, or methotrexate, an alkylating agent such as cyclophosphamide, dacarbazine, mechlorethamine, diaziquone, or temozolomide, an anthracycline such as doxorubicin or epirubicin, an antimicrotubule agent such as vinblastine, a platinum compound such as cisplatin or oxaliplatin, a taxane such as paclitaxel or docetaxel, or a topoisomerase inhibitor such as etoposide or mitoxantrone, or a vinca alkaloid such as vincristine).

Examples of cancers which can be treated using the TDCs of the disclosure include but not limited to urothelial cancers (*e.g*., bladder cancer, urethral cancer, and ureteral cancer), lung cancers (*e.g*., non-small cell lung cancers (NSCLC) such as adenocarcinoma, squamous cell carcinoma, large cell carcinoma, and small cell lung cancer), breast cancer, colorectal cancers (*e.g*., adenocarcinoma, carcinoid tumors, gastrointestinal stromal tumors, and colorectal lymphoma), pancreatic cancer, prostate cancer, and esophageal cancer. Other examples of cancers that can be treated with TDCs of the disclosure include head and neck cancer, ovarian cancer, renal cancer and stomach adenocarcinoma.

TDCs of the disclosure can be used in combination with a checkpoint inhibitor, for example a checkpoint inhibitor targeting PD1, PDL1, CTLA4, TIGIT, LAG3, OX40, CD40 or VISTA. Checkpoint inhibitors include antibodies and small molecules. Exemplary checkpoint inhibitors targeting PD1 include pembrolizumab, nivolumab, cemiplimab, and dostarlimab. Exemplary checkpoint inhibitors targeting PDL1 include atezolizumab, avelumab, durvalumab, BMS-1001, and BMS-1166. An exemplary checkpoint inhibitor targeting CTLA4 is ipilimumab. Exemplary checkpoint inhibitors targeting TIGIT include etigilimab, tiragolumab, and AB154. Exemplary checkpoint inhibitors targeting LAG3 include LAG525, Sym022, relatlimab, and TSR-033. Exemplary checkpoint inhibitors targeting OX40 include MEDI6469, PF-04518600, and BMS 986178. Exemplary checkpoint inhibitors targeting CD40 include selicrelumab, CP-870,893, and APX005M. An exemplary checkpoint inhibitors targeting VISTA is HMBD-002.For treatment of urothelial cancers, the TDCs of the disclosure can be used in combination with standard of care treatments, including but not limited to cisplatin, mitomycin-C, carboplatin, docetaxel, paclitaxel, doxorubicin, 5-FU, methotrexate, vinblastine, ifosfamide, and pemetrexed. In addition, the TDCs can be used in combination with a checkpoint inhibitor, such as ipilimumab.

For treatment of non-small-cell lung carcinoma (NSCLC), the TDCs of the disclosure can be used in combination with standard of care chemotherapy treatments such as cisplatin, carboplatin, paclitaxel, gemcitabine, vinorelbin, irinotecan, etoposide, or vinblastine would be included. In addition, the TDCs can be used in combination with targeted therapies, such as bevacizumab or Erbitux. In addition, the TDCs can be used in combination with a checkpoint inhibitor, such as pembrolizumab, nivolumab, cemiplimab, dostarlimab, atezolizumab, avelumab, durvalumab, or ipilimumab.

For treatment of breast cancer, the TDCs of the disclosure can be used in combination with standard of care chemotherapeutic agents, such as the anthracyclines (doxorubicin or epirubicin) and the taxanes (paclitaxel or docetaxel), as well as fluorouracil, cyclophosphamide and carboplatin. In addition, the TDCs of the disclosure can be used in combination with targeted therapies. Targeted therapies for HER2/neu positive tumors include trastuzumab and pertuzumab and for estrogen receptor (ER) positive tumors include tamoxifen, toremifene and fulvestrant. In addition, the TDCs can be used in combination with a checkpoint inhibitor, such as atezolizumab.

For treatment of colorectal cancers, the TDCs of the disclosure can be used in combination with standard of care treatments, including but not limited to 5-FU, capecitabine, irinotecan, oxaliplatin, trifluridine and tipiracil. In addition, the TDCs of the disclosure can be used in combination with targeted therapies. Targeted therapies include bevacizumab, ramucirumab, and ziv-aflibercept. In addition, the TDCs can be used in combination with a checkpoint inhibitor, such as pembrolizumab, nivolumab, or ipilimumab.

For pancreatic cancer, the TDCs of the disclosure can be used in combination with standard of care chemotherapeutic agents, such as gemcitabine, 5-fluouracil, irinotecan, oxaliplatin, paclitaxel, capecitabine, cisplatin, or docetaxel. In addition, TDCs can be used in combination with targeted therapies, such as erlotinib, which inhibits EGFR.

For prostate cancer, the TDCs of the disclosure can be used in combination with standard of care chemotherapeutic agents, including docetaxel, optionally with the steroid prednisone, or cabazitaxel. In addition, the TDCs can be used in combination with a checkpoint inhibitor, such as ipilimumab.

For esophageal cancer, the TDCs of the disclosure can be used in combination with standard of care chemotherapeutic agents, such as carboplatin and paclitaxel, cisplatin and 5-FU, epirubicin, cisplatin, and 5-FU, docetaxel, cisplatin, and 5-FU, cisplatin with capecitabine, oxaliplatin and either 5-FU or capecitabine, irinotecan, or trifluridine and tipiracil. In addition, TDCs can be used in combination with targeted therapies, such as trastuzumab or ramucirumab. In addition, the TDCs can be used in combination with a checkpoint inhibitor, such as pembrolizumab.

The use of a TDC of the disclosure in combination with one or more therapies does not restrict the order in which the therapies are administered. For example, the TDC of the disclosure can be administered before, during or after a subject is treated with one or more therapies. In some embodiments, a TDC of the disclosure is administered prior to (*e.g.*, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g.*, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) treatment of a patient with another therapy (*e.g*., a second therapeutic agent as described above). In some embodiments, the TDC of the disclosure is incorporated into the same regimen as a second therapeutic agent.

### 6. EXAMPLES

The following abbreviations are found throughout the Examples:
Boc - *tert*-butyloxycarbonyl
DCM - dichloromethane
DMA - dimethylamine
DMF - dimethylformamide
DIPEA - N,N-Diisopropylethylamine
EtOAc - ethyl acetate
EtOH - ethanol
Fmoc - Fluorenylmethyloxycarbonyl
HOBt - Hydroxybenzotriazole
MeOH - methanol
NaHMDS - sodium hexamethyldisilazide
RT - room temperature, approximately 21 °C
TBTU - O-(Benzotriazol-1-yl)-N,N,N',N'-tetramethyluronium tetrafluoroborate
TEA - triethylamine
THF - tetrahyrdrofuran
TFA - trifluoroacetic acid
TMS-imidazole - 1-(Trimethylsilyl)imidazole

### 6.1. Example 1: Synthesis of 4-(6-methylpyridin-2-yl)-5-(1,5-naphthyridin-2-yl)-1,3-thiazol-2-amine (Compound A) (Comparative Example)

Compound A was prepared according to the general methodology in Scheme 1 below:

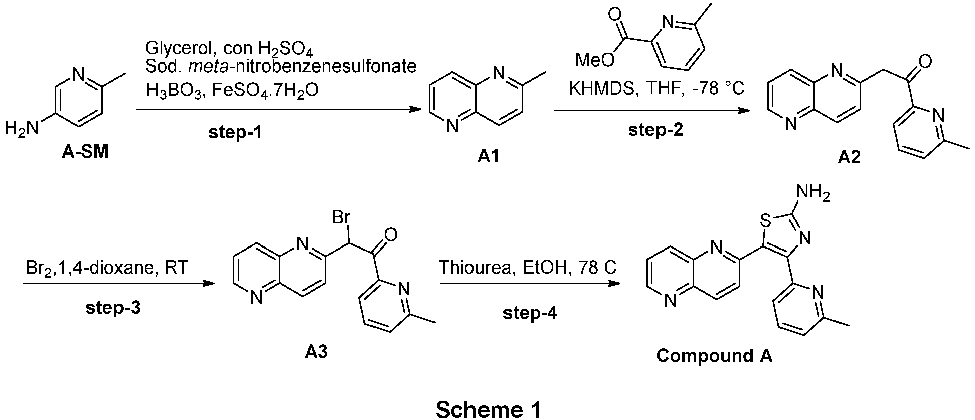

### 6.1.1. 2-methyl-1,5-naphthyridine (A1)

A mixture of concentrated sulfuric acid (2.5 ml), sodium m-nitrobenzenesulfonate (2.08 g, 9.24 mmol), boric acid (445 mg, 7.21 mmol) and ferrous sulfate heptahydrate (167 mg, 0.60 mmol) was stirred at room temperature. Glycerol (1.5 ml) followed by 5-Amino-2-methylpyridine **(A-SM)** (500 mg, 4.62 mmol) and water (2.5 ml) was added to the reaction mixture and heated at 135 °C for 18 h. After completion of the reaction as measured by TLC, the reaction mixture was cooled to approximately 21°C, basified using 4N NaOH and extracted with EtOAc (2 x 100 ml). The organic extracts were combined, washed with water (200 ml), dried over Na₂SO₄ and evaporated under reduced pressure to give the crude compound **A1.** The crude was purified by silica gel column chromatography using (2% MeOH/CH₂Cl₂) to afford compound **A1** as a pale brown crystalline solid (200 mg, 30%).

**¹H NMR (500** MHz, **CDCl₃)**: δ 8.92 (d, *J =* 3.0 Hz, 1H), 8.35 (d, *J* = 9.0 Hz, 1H), 8.31 (d, *J =* 5.9 Hz, 1H), 7.62 (dd, *J =* 8.5, 4.5 Hz, 1H), 7.54 (d, *J =* 5.9 Hz, 1H), 2.8 (s, 3H)

**LC-MS (ESI):** *m*/*z* 145 [M+H]⁺

### 6.1.2. 1-(6-methylpyridin-2-yl)-2-(1,5-naphthyridin-2-yl)ethan-1-one (A2)

A solution of **A1** (200 mg, 1.38 mmol) and methyl 6-methylpicolinate (209 mg, 1.38 mmol) in anhydrous THF (10ml) was placed under N₂ atmosphere and cooled to -78 °C. Potassium bis (trimethylsilyl) amide (0.5 M in toluene, 6.9 ml, 3.47 mmol) was added drop wise over a period of 5 min. The reaction mixture was stirred at -78 °C for 1h and then warmed to approximately 21 °C and maintained for 20 h. After completion of the reaction (as measured by TLC), the reaction mixture was quenched with saturated ammonium chloride solution (20 ml). The aqueous layer was extracted with EtOAc (2 x 20 ml). The combined organic extracts were washed with water (100 ml), dried over Na₂SO₄ and evaporated to give the crude compound **A2.** The crude material was purified by column chromatography (1% MeOH /CH₂Cl₂) to afford compound **A2** as an orange yellow solid (110 mg, 30.5%).

**¹H NMR** (400 **MHz, CDCl₃: *Enol form***):δ 15.74 (brs,-OH), 8.69 (t, *J =* 3.6, 1H), 8.12 (d, *J =* 9.2 Hz, 1H), 8.06 (dd, *J =* 8.4, 4.4 Hz, 2H), 7.82 (t, *J =* 7.6 Hz, 1H), 7.55 (dd, *J* = 8.4, 4.8 Hz, 1H) 7.45 (d, *J*= 9.6 Hz, 1H), 7.3 (dd, *J* = 7.6, 4.0 Hz, 1H), 7.16 ( s, 1H), 2.75(s, 3H)

**LC-MS** (ESI): *m*/*z* 264 [M+H]⁺

### 6.1.3. 4-(6-methylpyridin-2-yl)-5-(1,5-naphthyridin-2-yl)-1,3-thiazol-2-amine (Compound A)

A solution of **A2** (110 mg, 0.418 mmol) in 1,4-Dioxane (10 ml) was treated with bromine (0.025 ml, 0.501 mmol). The resulting reaction mixture was stirred at approximately 21 °C for 1h and then concentrated under reduced pressure to afford crude **A3** (120 mg), which was carried to the next step without further purification. The crude **A3** (120 mg) was dissolved in ethanol (15 ml). Thiourea (3.5 mg, 0.046 mmol) was then added and the reaction mixture was heated at 78 °C for 4h (until complete consumption of starting material was observed by TLC). The reaction mixture was cooled to approximately 21 °C and ammonia solution (25%, 1.5 ml) was added with gentle stirring. The solvent was evaporated, and then the residue was dissolved in CH₂Cl₂ (2 x 20 ml) and washed with water (50.0 ml). The separated organic layer was then washed with 1N HCl (30 ml x 2). The combined aqueous layer was basified with 35% aq. sodium hydroxide (20 ml) and extracted with CH₂Cl₂ (2 x 20 ml). The organic layer was dried over sodium sulfate and evaporated to give the crude **Compound A.** The crude **Compound A** was recrystallized from acetonitrile (2 ml) to afford purified **Compound A** as a yellow crystalline solid (35 mg, 49% yield over 2 steps).

**¹H NMR (400 MHz, CDCl₃)**: δ 8.86 (dd, *J* = 4.4, 1.6 Hz, 1H), 8.29 (t, *J* = 8.4 Hz, 1H), 8.06 (d, *J =* 9.2 Hz,1H), 7.64 (t, *J* = 7.6 Hz, 1H), 7.60-7.55 (m, 2H), 7.46 (d, *J =* 8 Hz, 1H), 7.20 (d, *J* = 7.6, 1H), 5.32 (brs, 2H), 2.57 (s, 3H)

**LC-MS** (ESI): *m*/*z* 320 [M+H]⁺

**UPLC** purity: 97.6%

### 6.2. Example 2: Synthesis of N-methyl-2-(4-{4-[3-(pyridin-2-yl)-1H-pyrazol-4-yl]pyridin-2-yl}phenoxy)ethan-1-amine (Compound B) (Comparative Example)

Compound B was prepared according to the general methodology in Scheme 2 below:

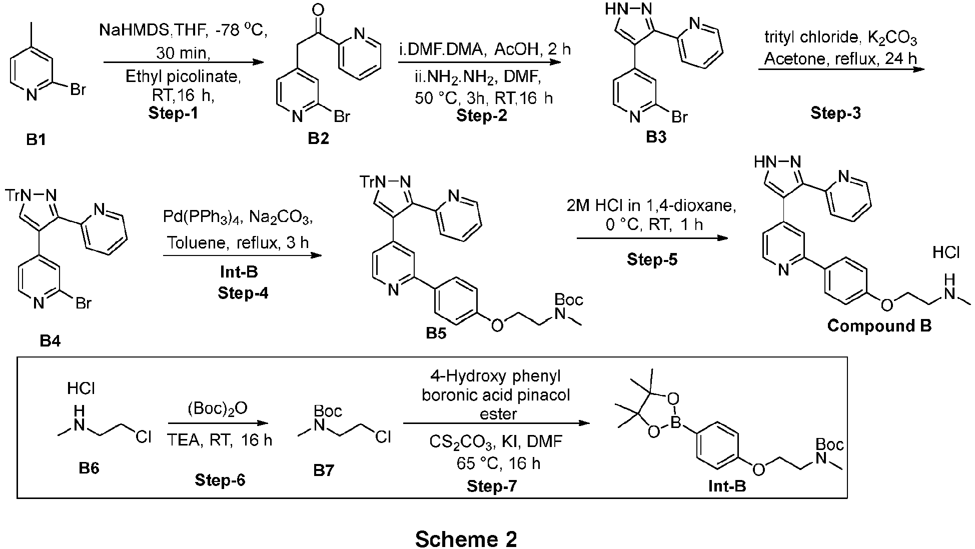

### 6.2.1. Tert-butyl (2-chloroethyl) (methyl) carbamate (B7)

To a stirred solution of Boc-anhydride (1.7 ml, 7.30 mmol) in THF (4 ml) were simultaneously added a solution of **B6** (1 g, 7.69 mmol) in water (4 ml) and a solution of TEA (1 ml, 7.69 mmol) in THF (4 ml) over the course of 1h. The resulting mixture was stirred at approximately 21 °C for 16 h. The reaction mixture was diluted with saturated NaCl solution (20 ml) and extracted with DCM (3 x 50 ml). The combined organic extracts were dried over Na₂SO₄, concentrated in *vacuo* to obtain the crude compound, which was purified by silica gel column chromatography using 10% EtOAc/Hexane to afford compound **B7** as a pale yellow liquid (1 g, 5.18 mmol, 71%).

**¹H** NMR **(400 MHz, CDCl₃)**: δ 3.58-3.52 (m, 4H), 2.93 (s, 3H), 1.46 (s, 9H)

### 6.2.2. Tert-butyl methyl (2-(4-(4, 4, 5, 5-tetramethyl-1, 3, 2-dioxaborolan-2-yl) phenoxy)ethyl) carbamate (Int-B)

To a stirred solution of 4-hydroxyphenylboronic acid pinacol ester (789 mg, 3.58 mmol) in DMF (13 ml) were added **B7** (900 mg, 4.66 mmol), KI (18 mg, 0.10 mmol) and Cs₂CO₃ (2.57 g, 7.88 mmol) under argon atmosphere. The reaction mixture was heated to 65 °C and stirred for 16 h. The reaction mixture was poured into water (20 ml) and extracted with EtOAc (3 X 20 ml). The combined organic layer was concentrated under reduced pressure to obtain the crude which was purified by column chromatography using 7% EtOAc/Hexane to afford **Int-B** as a pale yellow solid (580 mg, 1.53 mmol, 43%).

**¹H** NMR **(400 MHz, CDCl₃)**:δ 7.74 (d, *J* = 8.4 Hz, 2H), 6.87 (d, *J* = 8.8 Hz, 2 H), 4.16-4.06 (m, 2H), 3.65-3.59 (m, 2H), 2.97 (s, 3H), 1.45 (s, 9H), 1.33 (s, 12H)

### 6.2.3. 2-(2-bromopyridin-4-yl)-1-(pyridin-2-yl)ethan-1-one (B2)

To a stirred solution of 2-Bromo-4-methyl pyridine **(B1)** (2 g, 11.62 mmol) in THF (30 ml) at -78 °C under argon, a solution of NaHMDS (2 M in THF, 12.7 ml, 25.58 mmol) was added dropwise. The yellow solution was stirred at -78 °C for 30 min. Then a solution of ethyl picolinate (1.72 ml, 12.79 mmol) in THF (10 ml) was added and the reaction mixture warmed to approximately 21°C and stirred for 16 h. The solvent was evaporated under reduced pressure and the solid residue was triturated with diethyl ether, filtered and washed with diethyl ether. The solid was then diluted with saturated NH₄Cl solution (30 ml) and the aqueous phase was extracted with EtOAc (2 x 200 ml). The organic layer dried over Na₂SO₄ and concentrated. The crude product was purified by silica gel column chromatography using 10% EtOAc/Hexane to afford compound **B2** as a yellow solid (2.06 g, 7.46 mmol, 64.3%).

**¹H** NMR **(400 MHz, CDCl₃)**:δ 8.75 (d, *J* = 5.2 Hz, 1H), 8.32 (d, *J*= 5.2 Hz, 1H), 8.08 (d, *J=* 8.0 Hz, 1H), 7.89 (t, *J* =7.6 Hz 1H), 7.56-7.51(m, 2H), 7.28-7.25 (m, 1H), 4.55 (s, 2H)

**LC-MS (ESI):** *m*/*z* 277 [M]⁺

### 6.2.4. 2-bromo-4-[3-(pyridin-2-yl)-1H-pyrazol-4-yl]pyridine (B3)

A solution of **B2** (850 mg, 3.07 mmol) in dry DMF (3.4 ml) under argon was treated with glacial acetic acid (0.45 ml, 7.39 mmol) in DMF. DMA (0.6 ml, 4.61 mmol) was added drop wise and the mixture was stirred at approximately 21°C under argon atmosphere for 2 h. Hydrazine monohydrate (1.15 ml, 23.09 mmol) was added drop wise and the resulting mixture heated at 50 °C for 3 h and at approximately 21°C for 16 h. The reaction mixture was poured into water (30 ml) and extracted with CH₂Cl₂ (3 x 30 ml). The organic layer was dried over Na₂SO₄ and filtered. The solvent was evaporated under reduced pressure to afford the crude compound. The crude product was purified by silica gel column chromatography using 30% EtOAc/Hexane to afford compound **B3** as a yellow solid (560 mg, 1.86 mmol, 60.6%).

**¹H** NMR **(500 MHz, CDCl₃)**:δ 8.74 (brs, 1H), 8.34 (d, *J=* 5.0 Hz, 1H), 7.83 (brs, 1H), 7.81 (t, *J* = 6.0 Hz, 1H), 7.56 (s, 1H), 7.49 (d, *J=* 8.0 Hz, 1H), 7.39-7.84 (m, 1H), 7.31-7.26 (m, 1H)

**LC-MS** (ESI): *m*/*z* 301 [M]⁺

### 6.2.5. 2-Bromo-4-(3-(pyridin-2-yl)-1-trityl-1H-pyrazol-4-yl) pyridine (B4)

To a stirred solution of **B3** (500 mg, 1.66 mmol) in acetone (10 ml) was added K₂CO₃ (1.37 g, 9.99 mmol) and trityl chloride (464 mg, 2.49 mmol). The reaction mixture was subsequently heated to reflux and stirred for 24 h. The reaction mixture was filtered and the filtrate concentrated, and then partitioned between CH₂Cl₂ (20 ml) and water (10 ml). The organic phase was dried over Na₂SO₄ and concentrated. The crude solid was purified by silica gel column chromatography using 2% MeOH/CH₂Cl₂ to afford compound **B4** as a pale yellow solid (402 mg, 0.74 mmol, 44%).

**¹H NMR (500 MHz, CDCl₃)**: δ 8.53 (d, *J* = 4.5 Hz, 1H), 8.20 (d, *J* = 5.5 Hz, 1H), 7.75-7.05 (m, 2H), 7.56 (s, 1H), 7.51 (s, 1H), 7.35-7.32 (m, 9H), 7.25-7.22 (m, 8H)

### 6.2.6. Tert-butylmethyl (2-(4-(4-(3-(pyridin-2-yl)-1-trityl-1H-pyrazol-4-yl) pyridin-2-yl) phenoxy) ethyl) carbamate (B5)

To a stirred solution of **B4** (100 mg, 0.18 mmol) in toluene (2 ml) was added **Int-**B (185 mg, 0.49 mmol) in EtOH (0.75 ml) followed by 2M Na₂CO₃ solution (0.45 ml) under argon atmosphere. The reaction mixture was degassed with argon for 20 min and then Pd(PPh₃)₄ (16 mg, 0.01 mmol) was added and refluxed for 3 h. After complete consumption of starting material (monitored by TLC), the reaction mixture was poured into water and extracted with toluene (3 x 15 ml). The organic layer was dried over Na₂SO₄ and concentrated under reduced pressured to afford the crude product which was purified by silica gel column chromatography using 30% EtOAc /hexane to afford compound **B5** as a colorless solid (70 mg, 0.09 mmol, 53%).

**¹H NMR (400 MHz, CDCl₃)**: δ 8.53 (s, 1H), 8.49 (d, *J*= 4.8 Hz, 1H),7.82 (d, *J* = 8.8 Hz, 2H) 7.74-7.76 (m, 3H), 7.60 (s, 1H), 7.40-7.34 (s, 8H), 7.31-7.30 (m, 2H), 7.24-7.19 (m, 4H), 7.12- 7.10 (m, 1H), 6.93(d, *J* = 8.8 Hz, 2H), 4.19-4.12 (m, 2H), 3.66-3.58 (m, 2H), 2.98 (s, 3H), 1.46 (s, 9H).

### 6.2.7. N-methyl-2-(4-(4-(3-(pyridin-2-yl)-1H-pyrazol-4-yl) pyridin-2-yl) phenoxy) ethan-1-amine hydrochloride (Compound B)

To a stirred solution B5 (70 mg, 0.09 mmol) in CH₂Cl₂ (6 ml) was added 4 N HCl in 1,4-dioxane (0.5 ml) at 0 °C. The reaction mixture was stirred for 1 h under argon atmosphere. After complete consumption of starting material (monitored by TLC), the solvent was evaporated under reduced pressure to obtain the crude compound was triturated with *n-*pentane (2x 1 ml) and dried to afford **Compound B HCl salt** as a colorless solid (25 mg, 0.06 mmol, 69%).

**¹H NMR (400 MHz, DMSO-*d₆*):**δ 8.94 (brs, 2H), 8.62-8.56 (m, 3H), 8.30 (brs, 1H), 8.03-7.96 (m, 3H), 7.86 (d, *J = 7.6* Hz, 1H),7.69 (brs, 1H), 7.49 (dd, *J* =7.2, 5.6 Hz, 1H), 7.29 (d, *J*=7.6 Hz, 1H), 7.20 (d, *J= 8.4* Hz, 1H), 4.36 (t, *J =* 4.8 Hz, 2H), 3.39-3.35 (m, 2H), 2.67-2.63 (m, 3H)

**LC-MS (ESI):m/z** 372 [M+H]⁺

### 6.3. Example 3: Synthesis of N-methyl-2-(4-{4-[3-(6-methylpyridin-2-yl)-1H-pyrazol-4-yl]pyridin-2-yl}phenoxy)ethan-1-amine (Compound C)

Compound C was prepared according to the general methodology in Scheme 3 below:

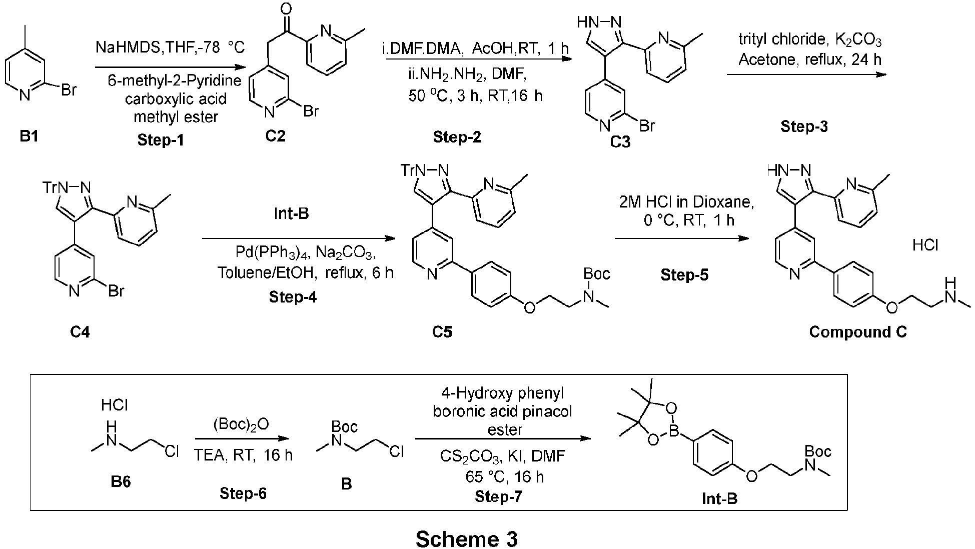

### 6.3.1. 2-(2-bromopyridin-4-yl)-1-(6-methylpyridin-2-yl)ethan-1-one (C2)

To a stirred solution of 2-Bromo-4-methyl pyridine **(B1)** (1 g, 5.81 mmol) in THF (15 ml) at -78 °C under argon, a solution of NaHMDS (2 M in THF, 6.39 ml, 12.8 mmol) was added dropwise. The yellow solution was stirred at -78 °C for 30 min. Then a solution of 6-methyl Picolinic acid methyl ester (1.19 ml, 8.72 mmol) in THF (7 ml) was added and the reaction mixture was allowed to warm up to approximately 21 °C and stirred for 16 h. The solvent was evaporated under reduced pressure and the solid residue was triturated with diethyl ether, filtered and washed with diethyl ether. The solid was then diluted with saturated NH₄Cl solution (20 ml) and the aqueous phase was extracted with EtOAc (2 x 150 ml). The organic layer was dried over Na₂SO₄ and concentrated. The crude product was purified by silica gel column chromatography using 10% EtOAc/Hexane to afford compound C2 as a yellow solid (1.1 g, 3.79 mmol, 65.4%).

**¹H NMR (500 MHz, CDCl₃)**: δ 8.30 (d, *J* = 5.0 Hz, 1H), 7.86 (d, *J* = 8 Hz, 1H), 7.73 (t, *J =* 7.5 Hz, 1H), 7.51 (s, 1H), 7.36 (d, *J =* 8 Hz, 1H), 7.24 (d, *J =* 5 Hz, 1H), 4.52 (s, 2H), 2.64 (s, 3H)

**LC-MS** (**ESI**):m/z 291 [M]⁺

### 6.3.2. 2-bromo-4-[3-(6-methylpyridin-2-yl)-1H-pyrazol-4-yl]pyridine (C3)

A solution of C2 (300 mg, 1.03 mmol) in dry DMF (1 ml) under argon was treated with glacial acetic acid (0. 14 ml, 2.48 mmol) in DMF. DMA (0.2 ml, 1.55 mmol) was added drop wise and the mixture was stirred at approximately 21 °C under argon atmosphere for 1 h. Hydrazine monohydrate (0.37 ml, 7.75 mmol) was added drop wise and the resulting mixture heated at 50 °C for 3 h and at approximately 21 °C for 16 h. The reaction mixture was poured into water (20 ml) and extracted with CH₂Cl₂ (3 x 20 ml). The organic layer was dried over Na₂SO₄ and filtered. The solvent was evaporated under reduced pressure to afford crude **C3.** The crude **C3** was purified by silica gel column chromatography using 2% MeOH/DCM to afford purified **C3** as a yellow solid (172 mg, 0.54 mmol, 53%).

**¹H NMR (500** MHz, **CDCl₃)**:δ 11.40 (brs, 1H), 8.37 (d, *J =* 5.0 Hz, 1H), 7.74 (s, 1H), 7.64 (s, 1H), 7.58 (t, *J=* 8.0 Hz, 1H), 7.34 (d, *J=* 6.0 Hz, 1H), 7.26 (d, *J* = 8.0 Hz, 1H), 7.17 (d, *J* = 8.0 Hz, 1H), 2.60 (s, 3H)

**LC-MS** (ESI): *m*/*z* 315 [M+H]⁺

### 6.3.3. 2-Bromo-4-(3-(6-methylpyridin-2-yl)-1-trityl-1H-pyrazol-4-yl) pyridine (C4)

To a stirred solution of C3 (40 mg, 0.12 mmol) in acetone (2 ml) was added K₂CO₃ (53 mg, 0.38 mmol) and trityl chloride (53 mg, 0.19 mmol). The reaction mixture was subsequently heated to reflux and stirred for 24 h. The reaction mixture was filtered and the filtrate concentrated, and then partitioned between CH₂Cl₂ (5 ml) and water (5 ml). The organic phase was dried over Na₂SO₄ and concentrated. The crude solid was purified by silica gel column chromatography using 2% MeOH/CH₂Cl₂ to afford compound **C4** as a pale yellow solid (30 mg, 0.05 mmol, 41%).

**¹H NMR (400 MHz, CDCl₃)**:δ 8.22 (d, *J* = 4.8 Hz, 1H), 7.73 (s, 1H), 7.59 (s, 3H), 7.39-7.35 (m, 9H), 7.31 (s, 1H), 7.28-7.25 (m, 6H), 7.24 (d, *J =* 12 Hz, 1H), 2.53 (s, 3H)

**LC-MS (ESI):**m/z 558 [M+H]⁺

### 6.3.4. Tert-butylmethyl (2-(4-(4-(3-(6-methylpyridin-2-yl)-1-trityl-1H-pyrazol-4-yl) pyridin-2-yl) phenoxy) ethyl) carbamate (C5)

To the stirred solution of **C4** (150 mg, 0.26 mmol) in toluene (5 ml) was added

**Int-B** (152 mg, 0.40 mmol) in EtOH (1 ml) followed by 2M Na₂CO₃ solution (0.7 ml) under argon atmosphere. The reaction mixture was degassed with argon for 20 min and then Pd(PPh₃)₄ (25 mg, 0.02 mmol) was added and refluxed for 6 h. After complete consumption of starting material (monitored by TLC), the reaction mixture was poured into water and extracted with toluene (3 x 10 ml). The organic layer was dried over Na₂SO₄ and concentrated under reduced pressure to afford crude **C5,** which was purified by silica gel column chromatography using 30% EtOAc/hexane to afford purified **C5** as a brown solid (51 mg, 0.07 mmol, 26%).

**¹H NMR (400 MHz, CDCl₃)**: δ 8.48 (d, *J* = 5.2 Hz, 1H), 7.82 (d, *J* = 8.8 Hz, 3H), 7.74 (s, 1H), 7.60 (s, 1H), 7.56 (d, *J* = 15.2Hz, *J* = 7.6Hz, 2H), 7.35-7.33 (m, 8H), 7.28-7.27 (m, 6H), 7.08 (d, *J =* 6.8 Hz, 2H), 6.93 (d, *J =* 8.8 Hz, 2H), 4.16-4.08 (m, 2H), 3.63-3.58 (m, 2H), 2.98 (s, 3H), 2.41 (s, 3H), 1.46 (s, 9H)

### 6.3.5. N-methyl-2-(4-{4-[3-(6-methylpyridin-2-yl)-1H-pyrazol-4-yl]pyridin-2-yl}phenoxy)ethan-1-amine (Compound C)

To a stirred solution of **C5** (51 mg, 0.07 mmol) in CH₂Cl₂ (5 ml) was added 4 N HCl in 1,4-dioxane (0.3 ml) at 0 °C. The reaction mixture was then stirred for 1 h under argon atmosphere. After complete consumption of starting material (monitored by TLC), the solvent was evaporated under reduced pressure to obtain crude **Compound C.** The crude **Compound C** was then triturated with *n*-pentane (2x 1 ml) and dried to afford **Compound C** as an HCl salt as a brown solid (20 mg, 0.05 mmol, 74%).

**¹H NMR (400 MHz, DMSO-*d₆*):**δ 8.93 (brs, 2H), 8.61 (d, *J =* 5.6 Hz, 1H),8.56 (brs, 1H), 8.33 (brs, 1H), 8.03 (d, *J* = 8.8 Hz, 2H), 7.88 (t, *J =* 7.6 Hz, 1H), 7.78-7.74 (m,1H), 7.65 (d, *J =* 7.2 Hz, 1H), 7.38 (d, *J = 7.6* Hz, 1H), 7.20 (d, *J =* 8.4 Hz, 2H), 4.36 (t, *J =* 5.2 Hz, 2H), 3.36 (t, J = 5.2 Hz, 2H), 2.66-2.63 (m, 3H), 2.50-2.46 (m, 3H)

**LC-MS** (**ESI**):m/z 386 [M+H]⁺

### 6.4. Example 4: Synthesis of (Z)-N-ethyl-3-(((4-(N-(2-(methylamino)ethyl)methylsulfonamido)phenyl)amino)(phenyl)methylene)-2-oxoindoline-6-carboxamide (Compound D) (Comparative Example)

Compound D was prepared according to the general methodology in Scheme 4 below:

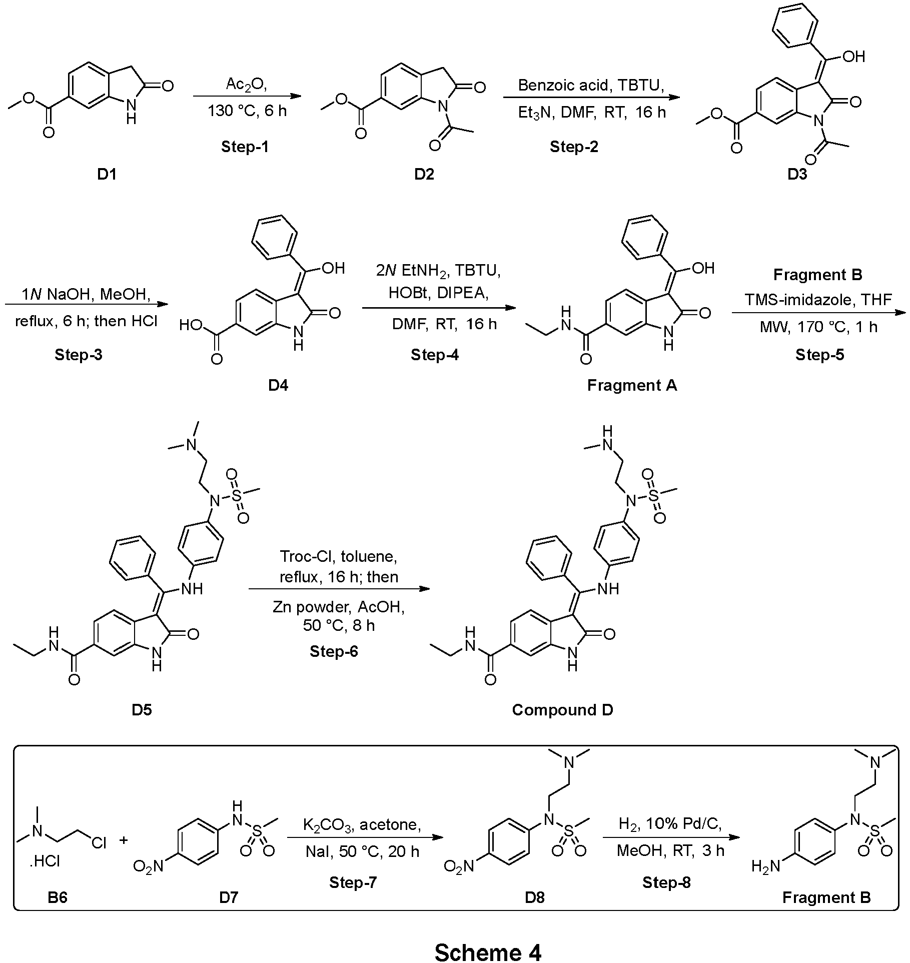

### 6.4.1. Methyl 1-acetyl-2-oxoindoline-6-carboxylate (D2)

A stirred solution of methyl 2-oxoindoline-6-carboxylate **(D1)** (2.0 g, 10.47 mmol) in acetic anhydride (16 ml) was heated to 130 °C under inert atmosphere for 6 h. After complete consumption of the starting material (monitored by TLC), the reaction mixture was cooled to approximately 21 °C. The precipitate was filtered, washed with n-hexane (2 x 50 ml) and dried *in vacuo* to afford compound **D2** as a yellow solid (1.5 g, 61.5%).

**¹H NMR (400 MHz, DMSO-*d₆*)**:δ 8.66 (s, 1H), 7.82 (d, *J* = 8.0 Hz, 1H), 7.48 (d, *J* = 8.0 Hz, 1H), 3.91 (s, 2H), 3.87 (s, 3H), 2.57 (s, 3H)

### 6.4.2. Methyl (Z)-1-acetyl-3-(hydroxy(phenyl)methylene)-2-oxoindoline-6-carboxylate (D3)

To a stirred solution of compound **D2** (1.5 g, 6.43 mmol) in DMF (10 ml) were added TBTU (2.69 g, 8.36 mmol), benzoic acid (903 mg, 7.40 mmol) and triethylamine (2.2 ml) at 0 °C under inert atmosphere. The reaction mixture was warmed to approximately 21 °C and stirred for 16 h. After complete consumption of the starting material (monitored by TLC), the reaction mixture was quenched with ice-cold water (30 ml) and extracted with EtOAc (2 x 40 ml). The combined organic extracts were dried over Na₂SO₄, filtered and concentrated *in vacuo* to obtain the crude product **D3,** which was purified by silica gel column chromatography using 80% EtOAc/Hexane to afford compound **D3** (900 mg, 42%) as a yellow solid.

**¹H NMR** (400 **MHz, CDCl₃):** δ 14.01 (brs, 1H), 8.93 (s, 1H), 7.76-7.70 (m, 3H), 7.67-7.63 (m, 1H), 7.59-7.56 (m, 2H), 7.12 (d, *J* = 8.0 Hz, 1H), 3.90 (s, 3H), 2.83 (s, 3H)

**LC-MS (ESI):***m*/*z* 338.3 [M+H]⁺

### 6.4.3. (Z)-3-(hydroxy(phenyl) methylene)-2-oxoindoline-6-carboxylic acid (D4)

To a stirred solution of compound D3 (900 mg, 2.67 mmol) in MeOH (15 ml) was added 1*N* aq. NaOH solution (15 ml) at approximately 21 °C. The mixture was heated to 100 °C and stirred for 6 h. After complete consumption of the starting material (monitored by TLC), the reaction mixture was cooled to approximately 21 °C, quenched with 1*N* aq. HCl solution (13 ml) and stirred for 30 min. The precipitated solid was filtered, washed with 20% EtOAc/Hexane to obtain compound **D4** (580 mg, 77%) as an off-white solid, which was carried to the next step without further purification.

**¹H NMR (400 MHz, DMSO-*d₆*)**:δ 12.76 (brs, 1H), 11.61 (brs, 1H), 7.77-7.50 (m, 8H), 7.13 (brs, 1H)

### 6.4.4. (Z)-N-ethyl-3-(hydroxy(phenyl)methylene)-2-oxoindoline-6-carboxamidelate (Fragment A)

To a stirred solution of compound **D4** (580 mg, 2.06 mmol) in DMF (10 ml) were added TBTU (729 mg, 2.27 mmol), HOBt (306 mg, 2.27 mmol) and *N*,*N*-diisopropyl ethylamine (1.9 ml, 10.32 mmol) at approximately 21 °C under inert atmosphere. After 30 min, 2*N* ethylamine in THF (2.1 ml, 4.12 mmol) was added at 0 °C and stirred for 1 h. The reaction mixture was then warmed to approximately 21 °C and stirred for additional 16 h. After complete consumption of the starting material (monitored by TLC), the volatiles were removed *in vacuo.* The residue was diluted with water (15 ml), filtered and washed with 20% EtOAc/Hexane (2 x 10 ml) to obtain the crude product, which was purified by silica gel column chromatography using 10% MeOH/CH₂Cl₂ to afford **Fragment A** (410 mg, 64.5%) as an off-white solid.

**¹H NMR (400 MHz, DMSO-*d₆*)**:δ 13.62 (brs, 1H), 11.39 (brs, 1H), 8.35-8.33 (m, 1H), 7.76-7.52 (m, 5H), 7.44-7.36 (m, 3H), 3.29-3.22 (m, 2H), 1.10 (t, *J* = 7.2 Hz, 3H)

LC-MS (ESI):m/z 307.1 (M - H⁺)

### 6.4.5. N-(2-(dimethylamino)ethyl)-N-(4-nitrophenyl)methanesulfonamide (D8)

To a stirred solution of compound **D7** (800 mg, 3.70 mmol) in acetone (15 ml) were added potassium carbonate (1.32 g, 9.62 mmol), sodium iodide (110 mg, 0.74 mmol) and compound **B6** (799 mg, 5.55 mmol) at 0 °C under inert atmosphere. The reaction mixture was heated to 50 °C and stirred for 20 h. After complete consumption of the starting material (monitored by TLC), the volatiles were removed *in vacuo.* The residue was diluted with water (20 ml) and extracted with EtOAc (2 x 40 ml). The combined organic extracts were dried over Na₂SO₄, filtered and concentrated *in vacuo* to obtain the crude product, which was purified by silica gel column chromatography using 5% MeOH/CH₂Cl₂ to afford compound **D8** (460 mg, 43%) as a pale yellow solid.

**¹H NMR (500 MHz, DMSO-*d₆***):δ 8.27 (d, *J* = 9.5 Hz, 2H), 7.68 (d, *J =* 9.5 Hz, 2H), 3.85 (t, *J* = 6.5 Hz, 2H), 3.13 (s, 3H), 2.31 (t, *J* = 6.5 Hz, 2H), 2.12 (s, 6H)

**LC-MS** (ESI):m/z 288.3 [M + H]⁺

### 6.4.6. N-(4-aminophenyl)-N-(2-(dimethylamino)ethyl)methanesulfonamide (Fragment B)

To a stirred solution of compound **D8** (460 mg, 1.60 mmol) in MeOH (10 ml) was added 10% Pd/C (40 mg) and stirred at approximately 21 °C under hydrogen atmosphere (balloon pressure) for 3 h. After complete consumption of the starting material (monitored by TLC), the reaction mixture was filtered through a pad of Celite^{®} and washed with MeOH (10 ml). The filtrate was concentrated *in vacuo* to obtain the crude product, which was purified by silica gel column chromatography using 10% MeOH/CH₂Cl₂ to afford **Fragment B** (300 mg 73%) as a pale yellow solid.

**¹H NMR (400 MHz, DMSO-*d₆***):δ 6.99 (d, *J* = 8.8 Hz, 2H), 6.54 (d, *J =* 8.8 Hz, 2H), 5.25 (s, 2H), 3.55 (t, *J* = 7.2 Hz, 2H), 2.91 (s, 3H), 2.24 (t, *J* = 7.2 Hz, 2H), 2.12 (s, 6H)

LC-MS (ESI):m/z 258.2 [M+H]⁺

### 6.4.7. (Z)-3-(((4-(N-(2-(dimethylamino)ethyl)methylsulfonamido)phenyl)amino)(phenyl)met hylene)-N-ethyl-2-oxoindoline-6-carboxamide (D5)

A solution of **Fragment A** (200 mg, 0.64 mmol), **Fragment B** (500 mg, 1.94 mmol) and TMS-imidazole (455 mg, 3.24 mmol) in THF (5 ml) was heated to 170 °C under microwave for 1 h. After consumption of the starting material (monitored by TLC and LC-MS), the volatiles were removed *in vacuo.* The residue was diluted with water (10 ml) and extracted with EtOAc (3 X 25 ml) to obtain the crude product, which was purified by preparative HPLC to afford compound **D5** (150 mg, 42%) as a pale yellow solid.

**¹H NMR (400 MHz, DMSO-*d₆*)**:δ 12.14 (s, 1H), 10.91 (s, 1H), 8.17 (t, *J =* 5.6 Hz, 1H), 7.64-7.57 (m, 3H), 7.53-7.51 (m, 2H), 7.34 (s, 1H), 7.17 (d, *J* = 8.8 Hz, 2H), 7.06 (d, *J* = 8.4 Hz, 1H), 6.84 (d, *J* = 8.8 Hz, 2H), 5.73 (d, *J* = 8.4 Hz, 1H), 3.58 (t, *J* = 6.8 Hz, 2H), 3.23-3.20 (m, 2H), 2.93 (s, 3H), 2.13 (t, *J* = 6.8 Hz, 2H), 1.90 (s, 6H), 1.06 (t, *J* = 7.2 Hz, 3H)

**LC-MS** (ESI):*m*/*z* 548.6 [M+H]⁺

### 6.4.8. (Z)-N-ethyl-3-(((4-(N-(2-(methylamino)ethyl)methylsulfonamido)phenyl)amino)(phenyl)meth ylene)-2-oxoindoline-6-carboxamide (Compound D)

To a stirred solution of compound **D5** (70 mg, 0.12 mmol) in dry toluene (3 ml) was added 2,2,2-trichlorethoxycarbonyl chloride (0.04 ml, 0.19 mmol) at approximately 21 °C under inert atmosphere. The reaction mixture was heated to reflux temperature (120 °C) and maintained for 16 h. After consumption of the starting material (monitored by TLC), the reaction mixture was cooled to approximately 21 °C, diluted with EtOAc (30 ml) and washed with 1*N* aq. HCl solution (15 ml). The organic layer was dried over Na₂SO₄, filtered and concentrated *in vacuo* to obtain the mono de-methylated with di-troc-protected compound (40 mg).

The crude product from the above reaction was dissolved in acetic acid (3 ml) and zinc powder (9 mg, 0.13 mmol) was added at approximately 21 °C under inert atmosphere. The reaction mixture was heated to 50 °C and stirred for 8 h. After complete consumption of the starting material (monitored by TLC), the reaction mixture was cooled to approximately 21 °C and the volatiles were removed *in vacuo.* The residue was diluted with water (20 ml) and extracted with EtOAc (2 x 25 ml). The combined organic extracts were washed with saturated NaHCO₃ solution (20 ml), dried over Na₂SO₄, filtered and concentrated under reduced pressure to obtain the crude **Compound D,** which was purified by silica gel column chromatography using 5-6% MeOH/CH₂Cl₂ to afford 12 mg of **Compound D** with 83% HPLC purity.

The reaction was repeated on a 60 mg scale and the obtained crude product was combined with above batch and purified by preparative HPLC to afford **Compound D** (8.0 mg, 6.3%) as a pale yellow solid.

**¹H NMR** (400 **MHz,** CD₃OD):δ 7.65-7.59 (m, 3H), 7.52.7.50 (m, 2H), 7.40 (s, 1H), 7.31 (d, *J* = 8.8 Hz, 2H), 7.07 (d, *J* = 8.4 Hz, 1H), 6.90 (d, *J* = 8.8 Hz, 2H), 5.95 (d, *J* = 8.4 Hz, 1H), 3.95 (t, *J* = 5.6 Hz, 2H), 3.39-3.32 (m, 2H), 3.05 (t, *J =* 5.6 Hz, 2H), 2.93 (s, 3H), 2.71 (s, 3H), 1.19 (t, *J* = 7.2 Hz, 3H)

**LC-MS (ESI)**:*m*/*z* 534.6 [M+H]⁺

**UPLC purity:** 99.18%

### 6.5. Example 5: Alternative synthesis of (Z)-N-ethyl-3-(((4-(N-(2-(methylamino)ethyl)methylsulfonamido)phenyl)amino)(phenyl)methylene)-2-oxoindoline-6-carboxamide (Compound D) (Comparative Example)

Compound D was also prepared according to the general methodology in Scheme 5 below:

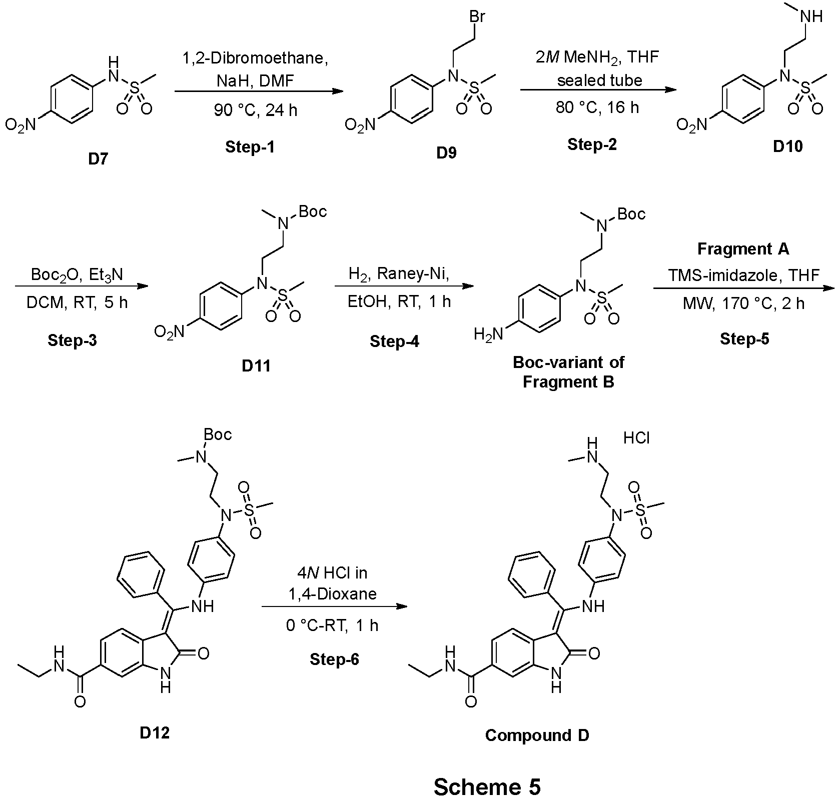

### 6.5.1. N-(2-bromoethyl)-N-(4-nitrophenyl)methanesulfonamide (D9)

To a stirred solution of compound D7 (1.0 g, 4.65 mmol) in DMF (10 ml) was added sodium hydride (60% in mineral oil; 320 mg, 7.99 mmol) at 0 °C under inert atmosphere and stirred at approximately 21 °C for 30 min. To this mixture, 1,2-dibromoethane (2.18 g, 11.60 mmol) was added at approximately 21 °C. The mixture was heated to 90 °C and stirred for 24 h. The reaction was monitored by TLC. The reaction mixture was cooled to approximately 21 °C, quenched with ice-cold water (30 ml) and extracted with EtOAc (2 x 40 ml). The combined organic extracts were dried with Na₂SO₄, filtered and concentrated *in vacuo* to obtain the crude product, which was purified by silica gel column chromatography using 5% MeOH/CH₂Cl₂ to afford 1.2 g of **D9** as a mixture containing 40% unreacted starting material. The obtained mixture was directly taken for next reaction without further purification.

**¹H NMR (500 MHz, CDCl₃**):δ 8.29 (d, *J* = 8.5 Hz, 2H), 7.56 (d, *J* = 8.5 Hz, 2H), 4.12 (t, *J* = 7.0 Hz, 2H), 3.44 (t, *J* = 7.0 Hz, 2H), 3.01 (s, 3H)

### 6.5.2. N-(2-(methylamino)ethyl)-N-(4-nitrophenyl)methanesulfonamide (D10)

To a stirred solution of compound **D9** (1.2 g, impure) in THF (10 ml) were added triethylamine (1.6 ml) and methylamine (2*M* in THF; 9.3 ml, 18.63 mmol) in a sealed tube at approximately 21 °C under inert atmosphere. The reaction mixture was heated to 80 °C and maintained for 16 h. After complete consumption of the starting material (monitored by TLC), the reaction mixture was cooled to approximately 21 °C and concentrated under reduced pressure to obtain crude **D10.** The crude **D10** was purified by silica gel column chromatography using 15% MeOH/CH₂Cl₂ to afford compound **D10** as a yellow solid (500 mg, 39% overall yield in two steps).

**¹H** NMR **(500 MHz, DMSO-*d₆*)**:δ 8.94 (brs, 1H), 8.31 (d, *J* = 9.0 Hz, 2H), 7.80 (d, *J* = 8.5 Hz, 2H), 4.06 (t, *J* = 6.0 Hz, 2H), 3.15 (s, 3H), 3.00 (t, *J* = 6.0 Hz, 2H), 2.55 (s, 3H)

### 6.5.3. tert-butyl methyl(2-(N-(4-nitrophenyl)methylsulfonamido)ethyl)carbamate (D11)

To a stirred solution of **D10** (500 mg, 1.83 mmol) in CH₂Cl₂ (10 ml) were added triethylamine (0.4 ml, 2.61 mmol) and Boc-anhydride (659 mg, 3.02 mmol) at approximately 21 °C under inert atmosphere and maintained for 5 h. After complete consumption of the starting material (monitored by TLC), the volatiles were removed *in vacuo* to obtain the crude product, which was purified by silica gel column chromatography using 5% MeOH/CH₂Cl₂ to afford **D11** as a colorless thick syrup (320 mg, 47%).

**¹H NMR (400 MHz, DMSO-*d₆***):δ 8.27 (d, *J* = 8.4 Hz, 2H), 7.68 (d, *J* = 8.4 Hz, 2H), 3.91 (t, *J* = 6.4 Hz, 2H), 3.28-3.25 (m, 2H), 3.07 (s, 3H), 2.72-2.70 (m, 3H), 1.33-1.27 (m, 9H)

**LC-MS (ESI**):*m*/*z* 274.2 (M⁺- B°C)

### 6.5.4. tert-butyl (2-(N-(4-aminophenyl)methylsulfonamido)ethyl)(methyl)carbamate (Boc-variant of Fragment B)

To a solution of compound **D11** (250 mg, 0.67 mmol) in EtOH (10 ml) was added Raney-Ni (40 mg) and stirred at approximately 21 °C under hydrogen atmosphere (balloon pressure) for 1 h. After complete consumption of the starting material (monitored by TLC), the reaction mixture was filtered through a pad of Celite^{®} and washed with EtOH (10 ml). The combined filtrate was concentrated *in vacuo* to obtain the crude product, which was purified by silica gel column chromatography using 10% MeOH/CH₂Cl₂ to afford **Boc-variant of Fragment B** as a pale yellow solid (180 mg, 77%).

**H NMR (400 MHz, DMSO-*d₆***):δ 7.01 (d, *J* = 8.4 Hz, 2H), 6.53 (d, *J =* 8.4 HZ, 2H), 5.24 (s, 2H), 3.60 (t, *J* = 6.4 Hz, 2H), 3.18 (t, *J* = 6.4 HZ, 2H), 2.88 (s, 3H), 2.75-2.71 (m, 3H), 1.36-1.33 (m, 9H)

**LC-MS (ESI):***m*/*z* 244.2 (M⁺- B°C)

### 6.5.5. tert-butyl (Z)-(2-(N-(4-(((6-(ethylcarbamoyl)-2-oxoindolin-3-ylidene)(phenyl)methyl)amino)phenyl) methylsulfonamido)ethyl)(methyl)carbamate (D10)

A solution of **Fragment A** (70 mg, 0.22 mmol), **Boc-variant of Fragment B** (155 mg, 0.45 mmol) and TMS-imidazole (159 mg, 1.13 mmol) in THF (3 ml) was heated to 170 °C under microwave for 160 min. After consumption of the starting material (monitored by TLC and LC-MS), the volatiles were removed *in vacuo* to obtain the residue, which was purified by preparative HPLC to afford compound **D10** (50 mg, 36%) as a pale yellow solid.

**¹H NMR (400 MHz, CDCl₃**):δ 12.13 (brs, 1H), 8.01 (brs, 1H), 7.61-7.51 (m, 3H), 7.44-7.41 (m, 3H), 7.13-7.11 (m, 2H), 6.98 (d, *J* = 8.4 HZ, 1H), 6.75 (d, *J* = 8.4 HZ, 2H), 5.96-5.91 (m, 2H), 3.74-3.71 (m, 2H), 3.49-3.41 (m, 2H), 3.30-3.27 (m, 2H), 2.80 (s, 6H), 1.40-1.36 (m, 9H), 1.19 (t, *J* = 7.2 HZ, 3H)

**LC-MS (ESI):m/z** 634.6 [M+H]⁺

### 6.5.6. (Z)-N-ethyl-3-(((4-(N-(2-(methylamino)ethyl)methylsulfonamido)phenyl)amino)(phenyl)meth ylene)-2-oxoindoline-6-carboxamide hydrochloride (Compound D as HCl salt)

To a stirred solution of compound **D10** (20 mg, 0.03 mmol) in diethyl ether (3 ml) was added 4*N* HCl in 1,4-dioxane (0.3 ml) at 0 °C under inert atmosphere. The reaction mixture was stirred at approximately 21 °C for 1 h. After complete consumption of the starting material (monitored by TLC), the volatiles were removed *in vacuo* to obtain the crude product, which was triturated with n-pentane (2 x 4 ml) to afford **Compound D** as an HCl salt (12 mg, 71%) as a pale yellow solid.

**¹H NMR (400 MHz, CD₃OD)**:δ 7.65-7.59 (m, 3H), 7.52.7.50 (m, 2H), 7.40 (s, 1H), 7.31 (d, *J =* 8.8 Hz, 2H), 7.07 (d, *J =* 8.4 Hz, 1H), 6.90 (d, *J* = 8.8 Hz, 2H), 5.95 (d, *J =* 8.4 Hz, 1H), 3.95 (t, *J =* 5.6 Hz, 2H), 3.39-3.32 (m, 2H), 3.05 (t, *J =* 5.6 Hz, 2H), 2.93 (s, 3H), 2.71 (s, 3H), 1.19 (t, *J =* 7.2 Hz, 3H).

**LC-MS** (**ESI)**:m/z 534.7 [M+H]⁺

UPLC **purity:** 96.26%

### 6.6. Example 6: In vitro assays to test activity of Compounds A-D

Compounds A-D were tested to determine whether they could inhibit TGF-β-induced luciferase activity in HEK293T cells *in vitro.*

30,000 HEK293T cells were seeded in a 96 well white flat bottom plate overnight. The next day 100 ng of a SMAD luciferase reporter plasmid per well was transfected into the cells using lipofectamine for 24 hours. The next day cells were treated with Compounds A-D and 100 pM TGF-β for 24 hours. Luciferase activity was measured using the Dual-Glo^{®} luciferase assay kit (Promega). The assay was run twice for Compounds A, B, and D, and three times for Compound C. The results are shown in Table 3.

| **Table 3** | | | |
|---|---|---|---|
| **Compound** | **Experiment 1 IC₅₀ (nM)** | **Experiment 2 IC₅₀ (nM)** | **Experiment 3 IC₅₀ (nM)** |
| Compound A | 18.7 | 29.8 | --- |
| Compound B | 51.8 | 11.3 | --- |
| Compound C | 10.1 | 21.2 | 13.2 |
| Compound D | 1070 | 1520 | --- |

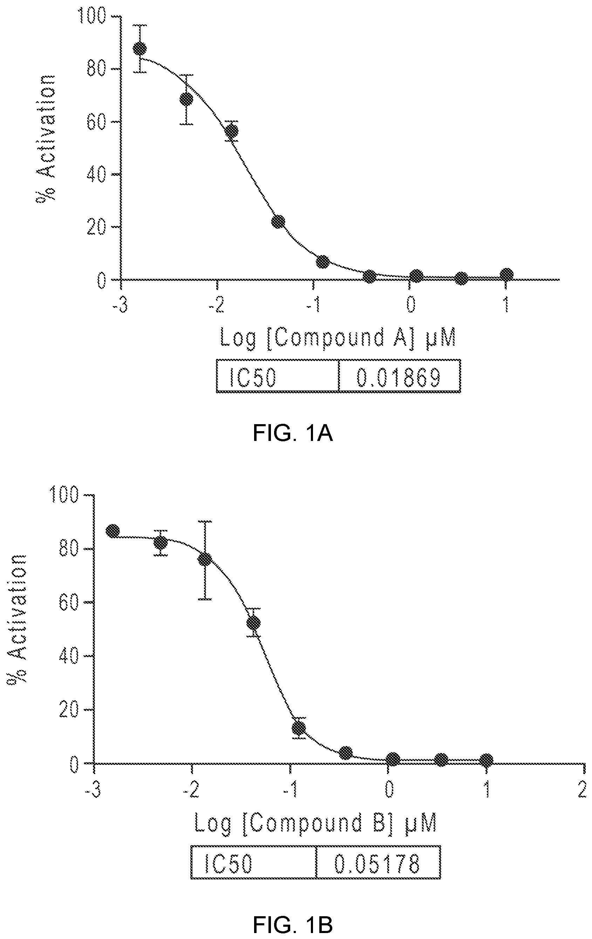

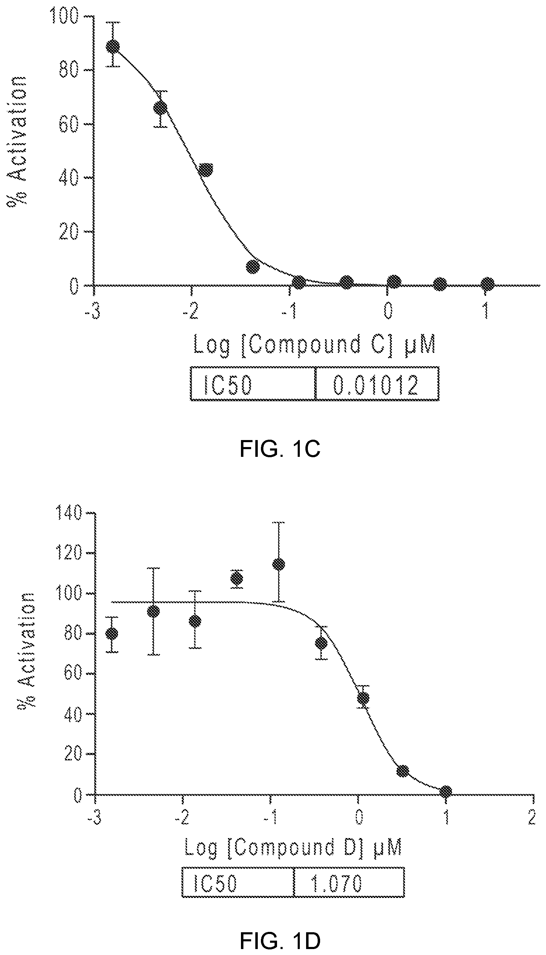

The activity data for Experiment 1 are shown in FIG. 1.

Compounds A-C demonstrated the greatest inhibitory activity.

### 6.7. Example 7: Synthesis of 4-((S)-2-((S)-2-(6-(2,5-dioxo-2H-pyrrol-1(5H)-yl)hexanamido)-3-methylbutanamido)-5-ureidopentanamido)benzyl methyl(2-(4-(4-(3-(6-methylpyridin-2-yl)-1H-pyrazol-4-yl)pyridin-2-yl)phenoxy)ethyl)carbamate

Compound C was linked to a valine-citrulline linker according to the general methodology in Scheme 6 below:

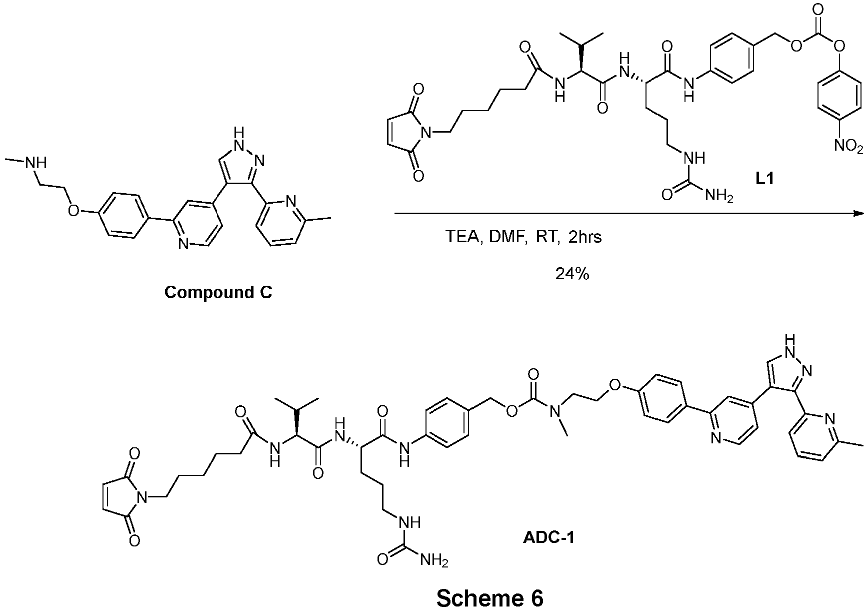

**L1** (122 mg, 0.165 mmol, 1.1 equiv.) and TEA (52 µl, 0.375 mmol, 2.5 equiv.) was added to a solution of **Compound C** (58 mg, 0.150 mmol, 1.0 equiv.) in DMF (2 ml) at 0 °C and the reaction mixture was stirred at approximately 21 °C for 2 hours to afford crude **ADC-1.** The crude ADC-1 was purified by preparative HPLC to afford purified **ADC-1** as a white solid (34 mg, 24 % yield).

### 6.8. Example 8: Generation of Antibody Drug Conjugate 1 (ADC1)

An anti-human FAP antibody is dialyzed overnight into conjugation buffer (25mM Sodium Borate/25mM NaCl, and 0.3mM EDTA, final pH 7.4). Antibodies are reduced using *tris*(2-carboxyethyl)phosphine (TCEP) for 2 hr at reduction ratios of 10-30. **ADC-1** is dissolved in DMSO to a final concentration of 10 mM and then conjugated to antibody in the presence of 15% DMSO at conjugation ratios of 5-30. All reactions are carried out at approximately 21°C. For some drug antibody ratios (DAR), 50% propylene glycol is used as the organic solvent during the conjugation step. The final ADC is dialyzed in PBS overnight, filtered using a 0.22 µm filter and analyzed via HPLC-HIC to determine DAR and HPLC-SEC to determine levels of aggregation. For HPLC-HIC, samples are run over a TSKgel^{®} butyl-NPR column with a flow rate of 0.5 ml/min. Phase A is 25 mM sodium phosphate and 1.5 M ammonium sulfate at pH 6.95 while Phase B is 75% 25 mM sodium phosphate at pH 6.95 and 25% isopropyl alcohol. For HPLC-SEC analysis, a TSKgel^{®} G3000SW column (Tosoh Bioscience) is used with a flow rate of 0.25 ml/min for 25 min, at 280nM.

### 6.9. Example 9: Synthesis of Compound C linked to a disulfide linker (ADC-2)

Compound C was linked to a disulfide linker according to the general methodology in Scheme 7A-B below:

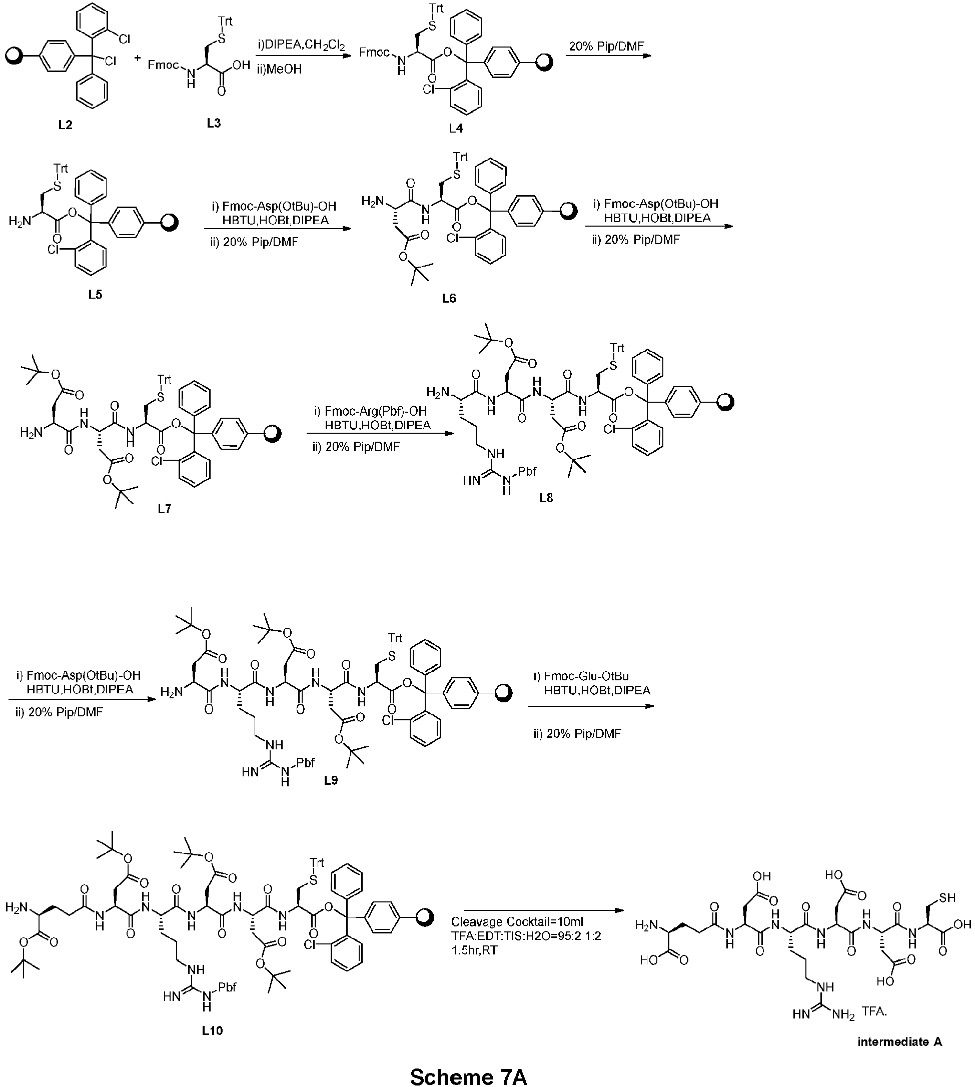

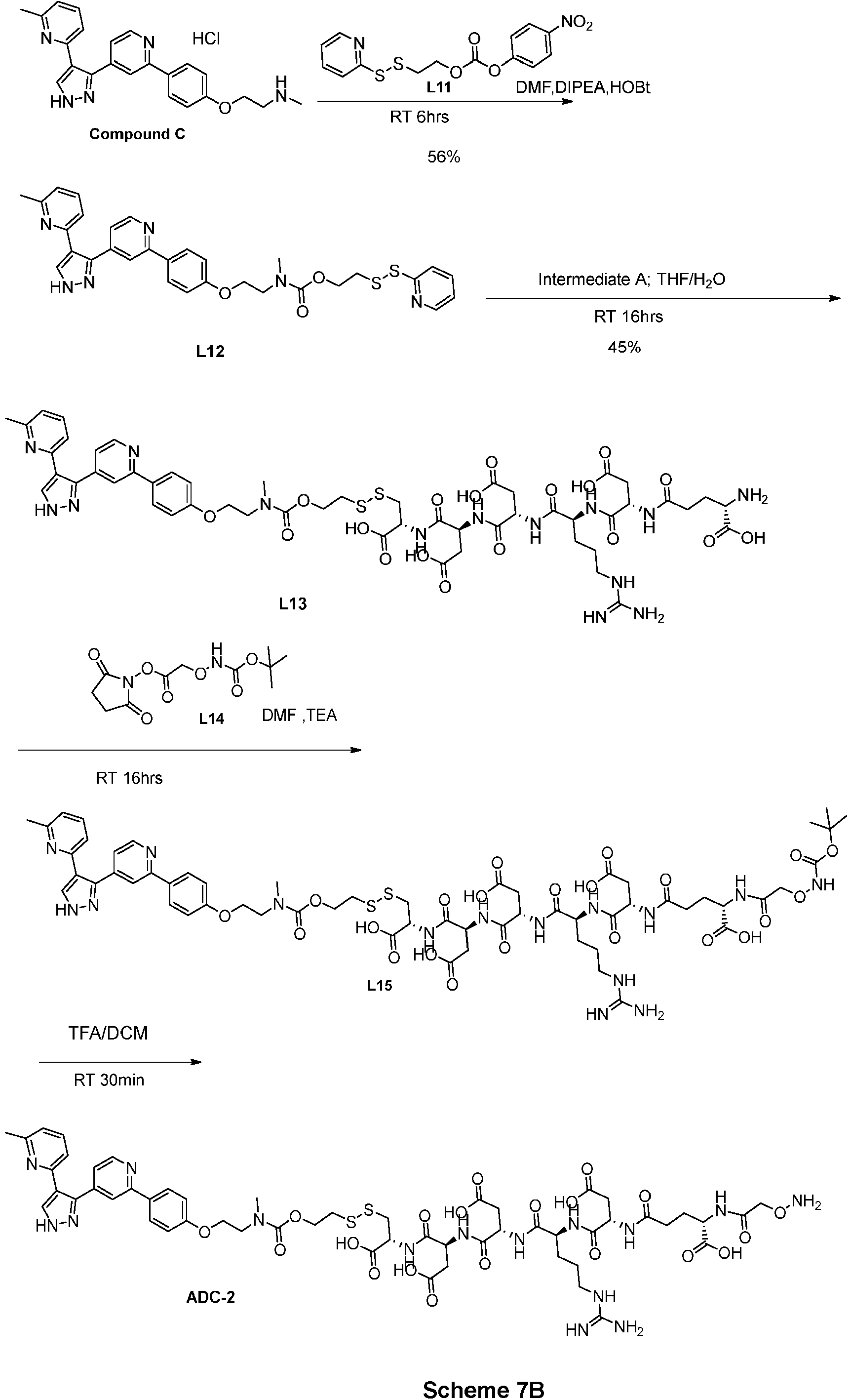

### 6.9.1. Synthesis of Intermediate A

2-chlorotrityl chloride resin **(L2)** (4g, 4 mmol) is washed with DCM (2x40ml), swelled in 50 ml DCM for 10 min, and then drained. Fmoc-Cys(Trt)-OH **(L3)** (7.03g, 12mmol) is dissolved in 40 ml DCM and added to the vessel containing the 2-chlorotrityl chloride resin. 8.7 ml DIPEA (6.8ml, 40 mmol) is added to the vessel, and the mixture is swirled for 2 hr at approximately 21 °C. 10 ml of methanol is then added to the mixture and swirled for 30 minutes. The resulting resin **(L4)** is then drained and washed five times with DMF. Resin **L4** is then deprotected to provide resin **L5** by adding approximately 40 ml of 20% piperidine in DMF to resin **L4,** shaking the mixture, and then draining the liquid from the resin. Another 40 ml of 20% piperidine in DMF is added to the resin and shaken for 15 minutes. The resin **L5** is then drained of liquid and washed with DMF (6 x 40 ml).

Solutions of Fmoc-amino acid are prepared by separately combining Fmoc-Asp(OtBu)-OH(4.93g,12mmol), Fmoc-Asp(OtBu)- OH(4.93g,12mmol), Fmoc-Arg(Pbf)-OH (7.79g,12mmol), Fmoc-Asp(OtBu)-OH(4.93g,12mmol), and Fmoc-Glu-OtBu (5.1g,12mmol) with HBTU/HOBT (4.55g,12mmol/1.62g,12mmol) and DIPEA (2ml,12 mmol).

The Fmoc-Asp(OtBu)-OH solution is added to resin **L5** and shaken for 60 minutes to provide resin **L6.** The resin **L6** is washed with DMF (6 x 40 ml), and then deprotected with 20% piperidine in DMF as above. Resins **L7, L8, L9,** and **L10** are then made by performing sequential couplings using the Fmoc-amino acid solutions and the same procedure used to make resin **L6** from resin **L5.**

In an exemplary synthesis, dry resin **L10** (8g) was added to a flask and 80ml cleavage solution was added (TFA:TES:EDT:H₂O=90:5:3:2, v/v/v/v). The reaction was allowed to proceed for 1.5 hours. The resin was then separated from the reaction mixture by filtration under pressure. The resin was then washed twice with TFA. The filtrates were combined, and a 10-fold volume of cold MTBE was added dropwise. The precipitated peptide **(Intermediate A)** was then centrifuged and washed with cold MTBE four times. **Intermediate A** was then dried at reduced pressure, and purified by preparative HPLC to provide 1.1g of **Intermediate A** as a white solid (yield: 37%). LC-MS (ESI) m/z: 752 [M+H]+.

### 6.9.2. 2-(pyridin-2-yldisulfanyl)ethylmethyl(2-(4-(4-(4- (6-methylpyridin-2-yl)-1H-pyrazol-3-yl)pyridin-2-yl)phenoxy)ethyl)carbamate (L12)

To a solution of **Compound C** (40mg, 0.1038mmol) and 4-nitrophenyl 2-(pyridin-2-yldisulfanyl)ethyl carbonate **(L11)** (80mg, 0.2272mmol) in DMF (5 ml) was added DIPEA (0.5 ml) and HOBt (14mg, 0.1038mmol). The mixture was stirred at approximately 21 °C under N₂ for 16 hrs to provide **L12.** The crude **L12** was purified by preparative-HPLC to give 35 mg of purified **L12** as a white solid (yield 56%).

### 6.9.3. (2R,5S,8S,11S,14S,19S)-19-amino-5,8,14-tris (carboxy methyl)-11-(3-guanidinopropyl)-2-(((2-(methyl(2-(4-(4-(4-(6-methylpyridin-2-yl)-1H-pyrazol-3-yl)pyridin-2-yl)phenoxy)ethyl)carbamoyloxy)ethyl) disulfanyl) methyl)-4,7,10,13,16-pentaoxo-3,6,9,12,15-pentaazaicosane-1,20-dioic acid (L13)

To a solution of **L12** (35 mg, 0.058mmol) in THF/H₂O (5ml/5ml) was added **Intermediate A** (80 mg, 0.106 mmol) under N₂. The mixture was stirred at approximately 21 °C for 16 hr to provide **L13.** The crude **L13** was purified by preparative HPLC to provide 23 mg of purified **L13** as a white solid (yield 31%).

### 6.9.4. (2R,5S,8S,11S,14S,19S)-19-(2-(tert-butoxy carbonyl aminooxy)acetamido) -5,8 , 14 -tris(carboxymethyl) -11-(3-guanidinopropyl) -2-(((2-(methyl(2-(4-(4-(4-(6-methylpyridin-2-yl)-1H-pyrazol-3-yl) pyridin-2 - I)phenoxy)ethyl)carbamoyloxy)ethyl)disulfanyl)methyl)-4,7,10, 13 ,16- pentaoxo-3,6,9,12,15-pentaazaicosane-1,20-dioic acid (L15)

To a solution of **L13** (32 mg, 0.025mmol) in DMF (3ml) was added 2,5-dioxopyrrolidin-1-yl2-(tert-butoxycarbonylaminooxy)acetate **(L14)** (28mg, 0.097 mmol) followed by TEA (0.5ml). The reaction mixture was stirred at approximately 21 °C under N₂ atmosphere for 16 hr to provide **L15.** The crude **L15** was purified by preparative HPLC to provide 12 mg of purified **L15** as white solid (yield 33%)

### 6.9.5. (2R,5S,8S,11S,14S,19S)-19- (2-(aminooxy) acetamido)-5,8,14-tris(carboxymethyl)-11-(3-guanidinopropyl)-2-(((2-(methyl(2-(4-(4(4-(6-methylpyridin-2-yl)-1H-pyrazol-3-yl)pyridin-2-yl)phenoxy) ethyl)carbamoyloxy)ethyl)disulfanyl)methyl)-4,7,10,13,16-pentaoxo-3,6,9,12,15-penta azaicosane-1,20-dioic acid (ADC-2)

To a mixture of **L15** (12mg, 0.0085mmol) in DCM (5ml) was added TFA (1ml). The mixture was stirred at approximately 21 °C for 30 minutes to provide **ADC-2.** The crude **ADC-2** was concentrated and purified with preparative HPLC to provide 3.5 mg of purified **ADC-2** as a white solid (yield 31%).

### 6.10. Example 10: Generation of Antibody Drug Conjugate 2 (ADC2)

**ADC-2** is attached to an anti-human FAP antibody via antibody lysine residues according to the general methodology in Scheme 8 below:

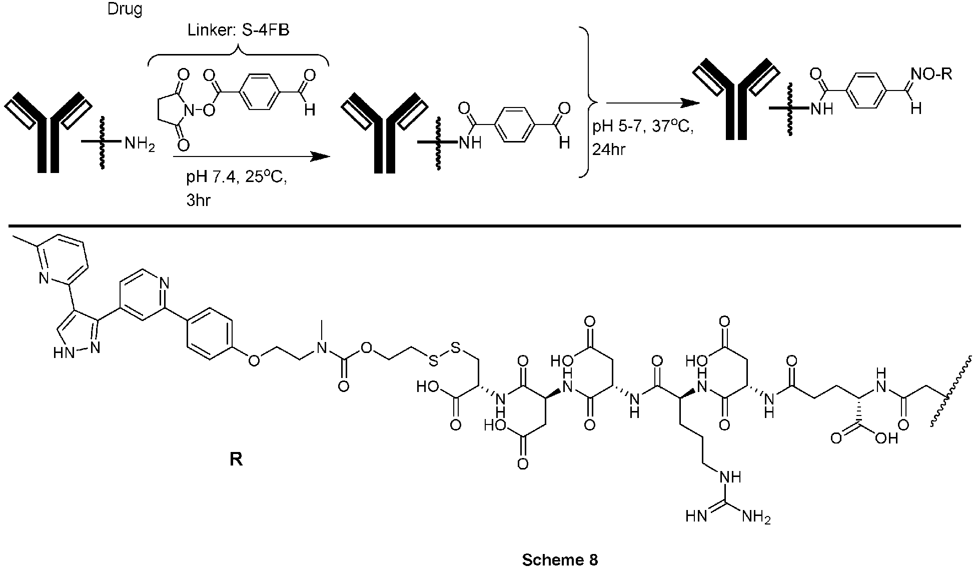

The antibody is dialyzed into PBS, pH 7.4. S-4FB is added to the antibody in PBS, pH 7.4 at different molar ratios and incubated at approximately 21°C for 3 hours The S-4FB-modified antibody solution is combined with a 2-hydrazinopyridine solution (0.5mM, in 100 mM MES buffer, pH 5.0) and incubated at 37° C for 30 minutes at various conjugation ratios, ranging from 5-50. The S4FB/Ab molar substitution ratio is determined by UV-Vis at A354. The modified antibody is purified using a Zeba^{™} spin desalting column, buffer exchanged into 50 mM phosphate buffer (pH 6.5, 150 mM NaCl) and then mixed with linker-S-S-drug **ADC-2** (10mM, in DMSO) at different molar ratios for 24 hours at 37 °C to provide **ADC2.** The next day, ADC2 samples are dialyzed against PBS overnight. The samples are filtered and then tested via HPLC-SEC, SDS-PAGE and LC-MS.

If ADC2 aggregation over 5% is detected by HPLC-SEC, the aggregated components are separated by AKTA with SEC columns (GE Healthcare Life Sciences, Superdex 200 increase 10/300 GL) and analyzed again by HPLC-SEC.

### 6.11. Example 11: Synthesis and characterization of Compound N (Comparative Example)

Compound N was synthesized according to the general methodology in Scheme 9 below:

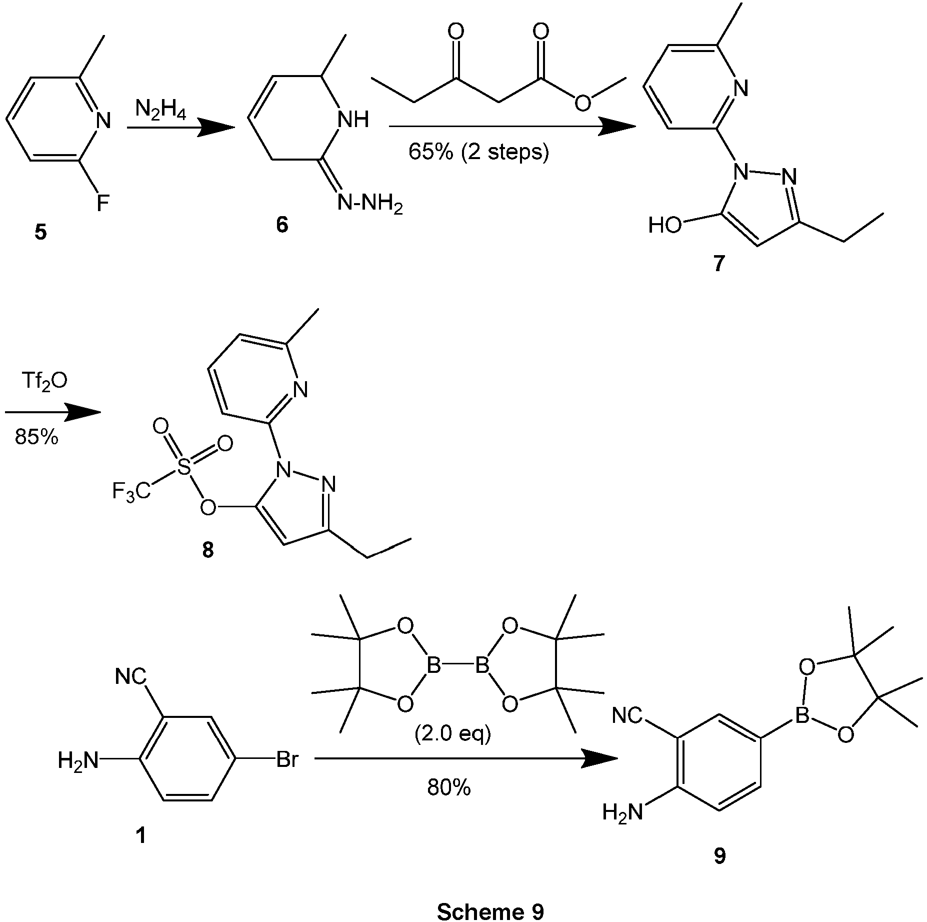

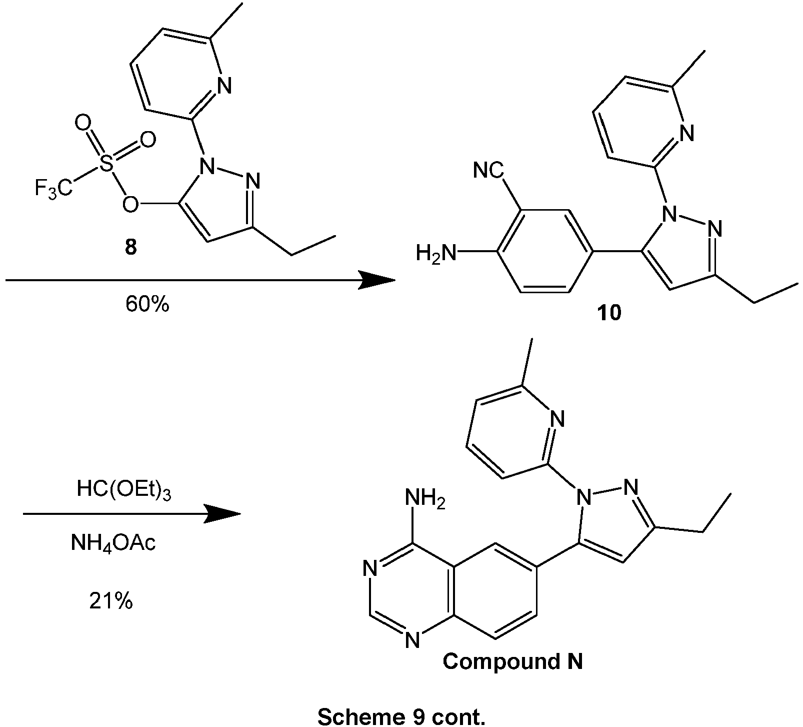

Compound N was compared to Compound C in a number of *in vitro* assays. A summary of their IC50 activity in recombinant kinase assays and their Kᵢ values are shown in Table 4. Table 4 also shows Compound C's activity in inhibiting TGF-β signaling in human HEK cells. Compound C was found to be 10 fold more potent than Compound N in the kinase assays.

| **Table 4** | | | | | |
|---|---|---|---|---|---|
| **ALK5 Small molecule inhibitor** | **IC50 (nM) (Kinase= 25 nM)** | **IC50 (nM) (Kinase= 1.5 nM)** | **Ki (nM) (Steady state equation)** | **Ki (nM) (Morrison equat/ 5 uM ATP)** | **HEK Luciferase Assay (nM)** |
| Compound C | 10 | 1.8 | 2.25 | 0.11 | 11.7 |
| Compound N | 18 | 12 | 18.4 | 2.1 | - |

### 6.12. Example 12: anti-FAP antibody binding to HEK cells

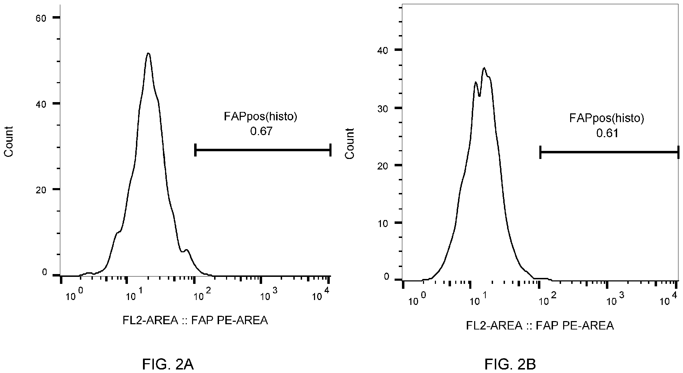

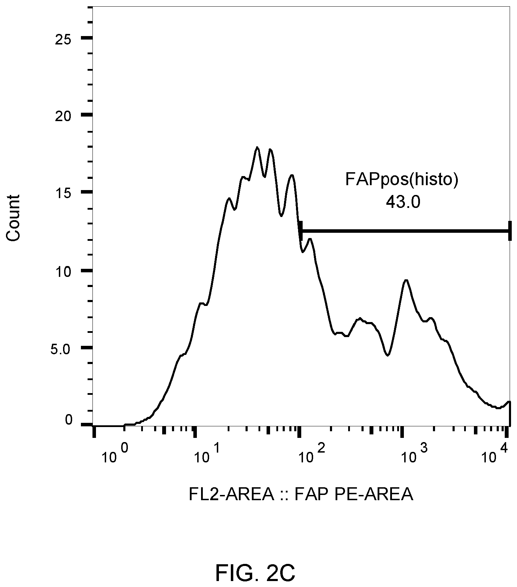

The ability of an anti-FAP antibody (commercially available mouse IgG₁, clone 427819) to bind HEK293 cells expressing human FAP was evaluated by FACS. The anti-FAP antibody bound to HEK293 cells in which human FAP cDNA was transfected and expressed on the cell surface (FIG. 2C), but did not bind to parental HEK cells in which human cDNA was not transfected (FIG. 2B).

### 6.13. Example 13: Production of targeted drug conjugates SYN-301 and SYN-302

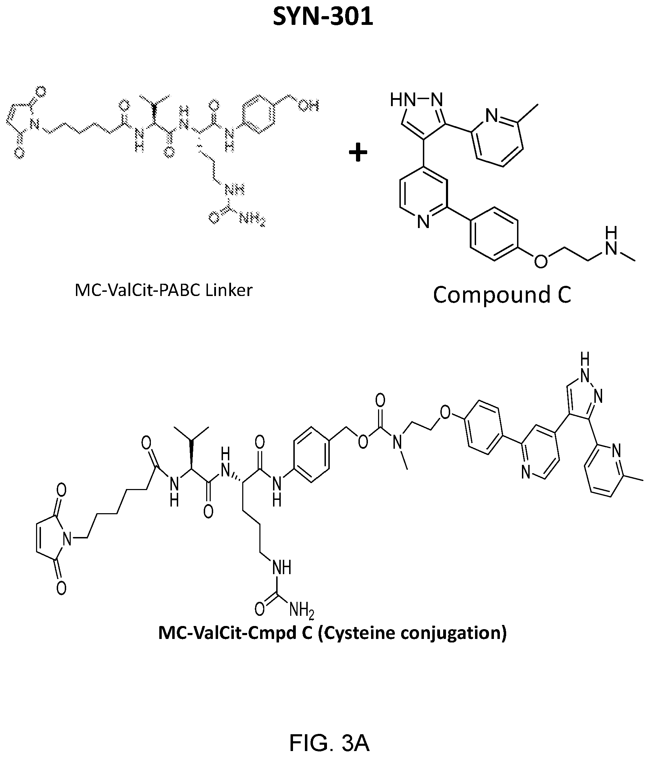

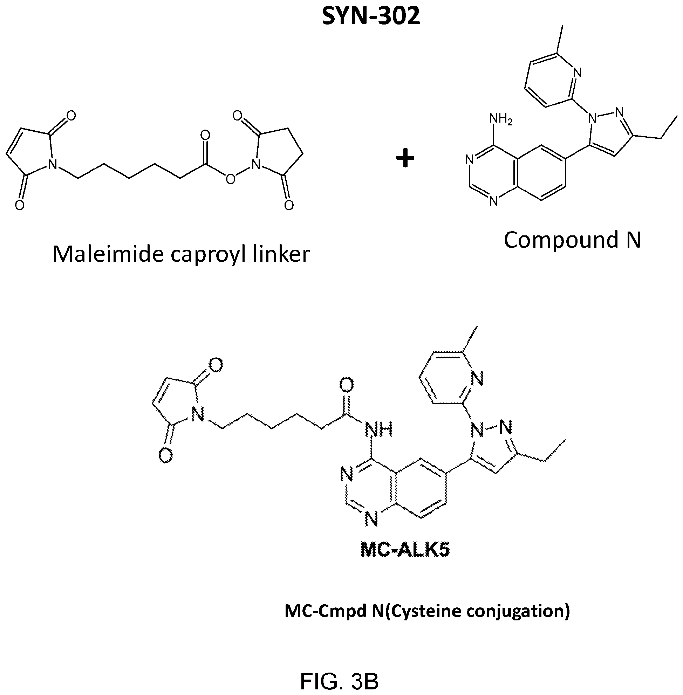

Two targeted drug conjugates were synthesized. In the first, Compound C was conjugated to an anti-FAP antibody (commercially availably mouse IgG₁, clone 427819) using a MC-Val-Cit-PABC cleavable linker (FIG. 3A). This targeted drug conjugate is referred to herein as SYN-301. In the second, Compound was conjugated to an anti-FAP antibody using a non-cleavable (MC) linker (FIG. 3B). This targeted drug conjugate is referred to herein as SYN-302. Drug antibody ratios were determined by reverse phase liquid chromatography using a PLRP-S column (1000 Å pore size, 5 µm particle size, 1 x 50 mm) and percent aggregation was determined by size exclusion chromatography using a TSKgel^{®} G3000SWXL column. A preparation of SYN-301 had a measured drug antibody ratio (DAR) of 5.5 and 4% aggregation, while a preparation of SYN-302 had a measured DAR of 5 and 3.9% aggregation.

### 6.14. Example 14: Evaluation of TGF-β signaling repression by SYN-301 and SYN-302 in HEK cells expressing human FAP protein

To evaluate the ability of SYN-301 and SYN-302 to inhibit TGF-β signaling, an assay was performed using HEK293FT cells expressing human FAP.

HEK293FT cells were transiently transfected with a construct encoding human FAP as well as a TGF-β responsive luciferase expression construct comprising three copies of a SMAD binding element driving expression of the luciferase reporter gene *Iuc2P* (pGL4.48[luc2P/SBE/Hygro]; Promega) and an expression control construct (pGL4.74 encoding Renilla luciferase; Promega) (1 µg : 1µg : 0.125 µg per ml cell culture) with Mirus TranslT^{®}-LT1 transfection reagent. Cells were plated at 350,000 cells/ml in 96 well plates (100 µl per well). After 24 hours, cells were pre-treated with SYN-301, SYN-302, unconjugated anti-FAP antibody or unconjugated Compound C for 4 hours. Then, 1 nM TGF-β was added and cells incubated for 3 hours. Luciferase expression was then measured (Dual-Glo luciferase detection system, Promega).

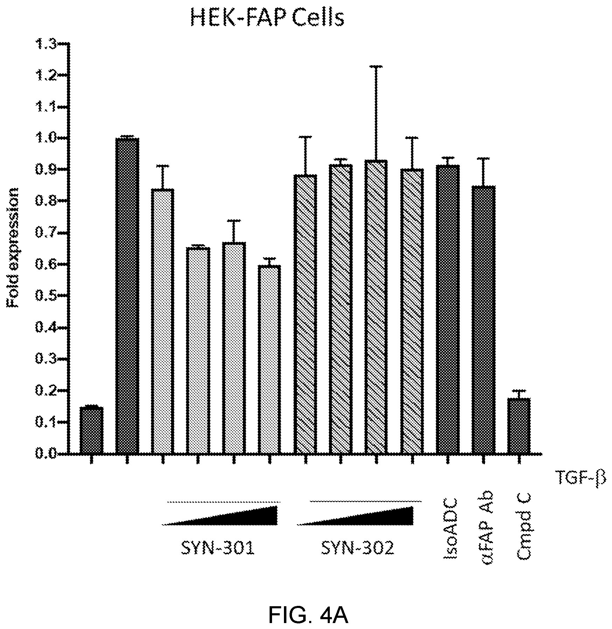

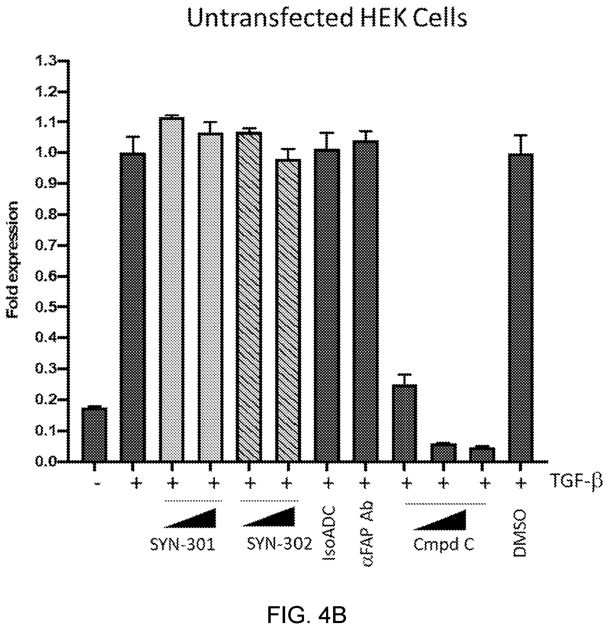

Results are shown in FIGS. 4A-4B. SYN-301, with cleavable linker, was able to inhibit TGF-β signaling in engineered HEK cells expressing FAP (FIG. 4A), while SYN-301 was not observed to have a significant effect on TGF-β signaling in parental HEK cells that do not express FAP (FIG. 4B). SYN-302, having a non-cleavable linker, was observed in this assay to be less efficacious than SYN-301 (FIG. 4A).

### 6.15. Example 15: FAP internalization induced by SYN-301 and SYN-302

To evaluate the ability of SYN-301 and SYN-302 to internalize FAP on target cells endogenously expressing FAP, an internalization assay was performed using WI-38 human lung fibroblast cells.

WI-38 cells were incubated with anti-FAP antibody, SYN-301, SYN-302, or isotype control ADC (a non-specific antibody control conjugated to cleavable ValCit-ALK5 inhibitor Compound C) for 30 minutes at 4 °C to detect cell surface FAP expression. Cells were then washed twice with cold PBS to remove residual antibody/antibody conjugates in the supernatant and then incubated at 37 °C for 3 hours to induce receptor internalization. After the 3 hour incubation, cells were washed and incubated with a PE conjugated rat anti-mouse secondary antibody to detect the remaining cell surface FAP expression. Relative FAP expression was compared to WI-38 cells incubated with the anti-FAP antibody or conjugates at 4 °C as a measurement of total FAP expression.

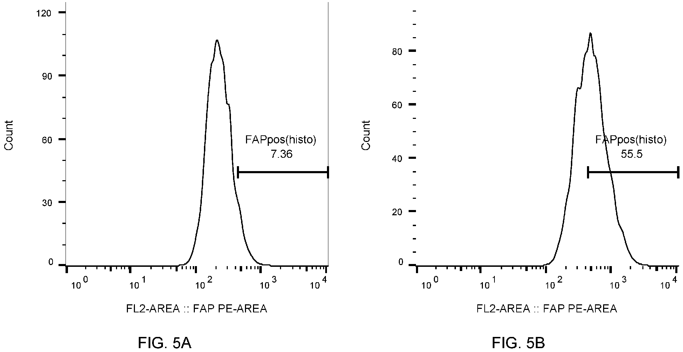

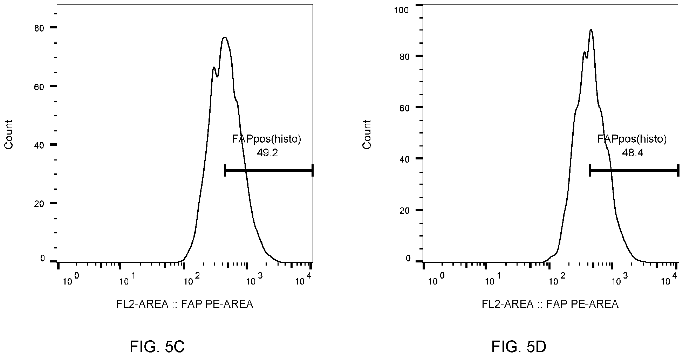

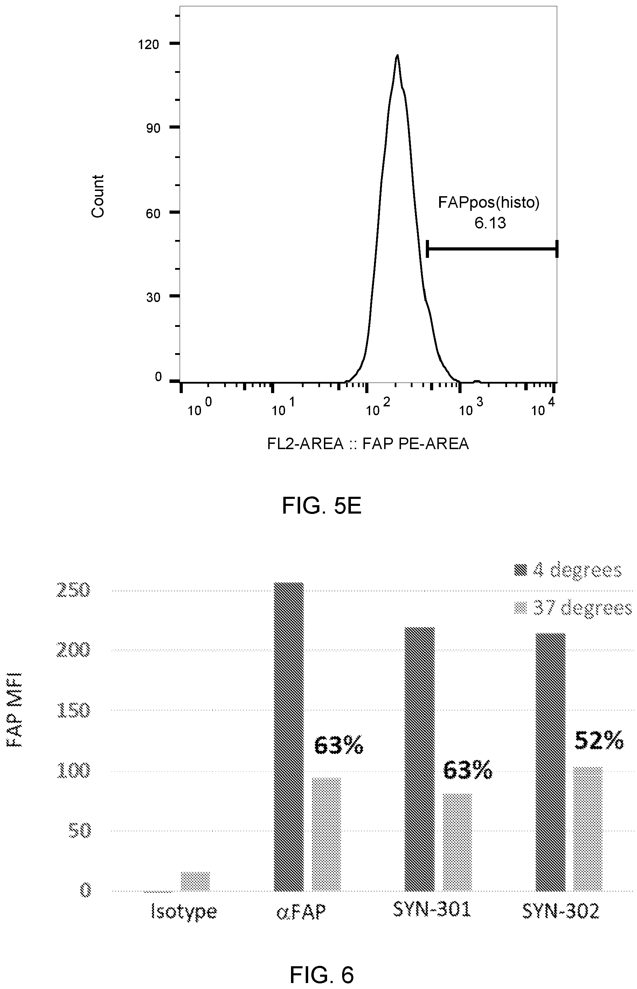

Results are shown in FIGS. 5A-5E and FIG. 6. 50-60% of WI-38 cells expressed FAP (FIGS. 5A-5E), and anti-FAP antibody, SYN-301, and SYN-302 were able to bind to and comparably internalize FAP in WI-38 cells (63%, 63% and 52%, respectively) (FIG. 6).

### 6.16. Example 16: Functional characterization of SYN-301 and SYN-302 in WI-38 cells

Increased expression of type IV collagen (COL4A1), fibronectin (FN1), and Leucine Rich Repeat Containing 15 (LRRC15) are markers of increased fibrosis. WI-38 human lung fibroblast cells were used to evaluate the ability of SYN-301 and SYN-302 to reduce expression of COL4A1, FN1, and LRRC15.

WI-38 human lung fibroblasts were plated at 50,000 cells/ml in 24 wells (500 µl per well) and incubated overnight. Cells were serum starved for 18 hours to reduce serum effects on TGFb-β regulated genes, and then pre-treated with SYN-301, SYN-302, isotype control ADC, anti-FAP antibody or Compound C at 1 µg/ml. TGF-β was added and cells were incubated for 19 hours. Cells were then scraped into RLT buffer (Qiagen) and RNA extracted using the Qiagen RNaeasy Kit. RNA was reverse transcribed into cDNAs, and qPCR was then performed with TaqMan primers for COL4A1, FN1, and LRRC15. GAPDH was used as normalizer.

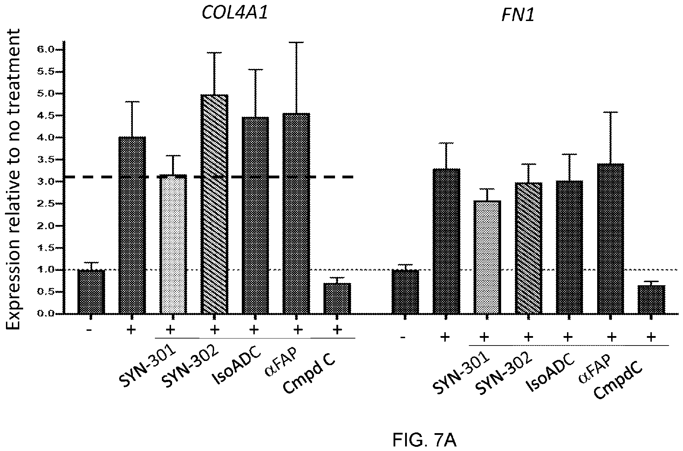

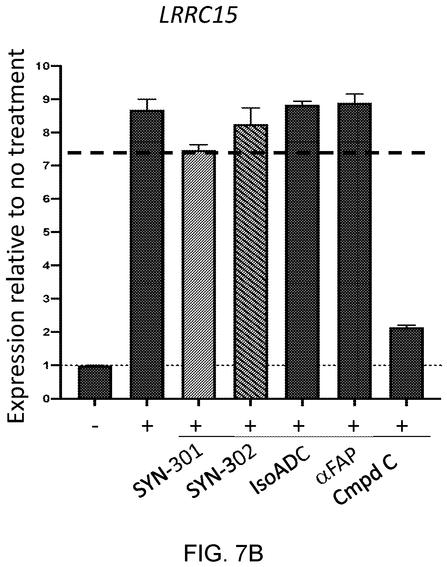

Results are shown in FIGS. 7A-7B. SYN-301 partially blocked TGF-β induced gene responses, reducing COL4A1 expression by approximately 25-30% (FIG. 7A), FN1 expression by approximately 20-25% (FIG. 7A) and LRRC15 expression by approximately 15-20% (FIG. 7B). SYN-302 had a more modest response in blocking TGF-β signaling, while unconjugated anti-FAP antibody and isotype control ADC did not inhibit TGF-β signaling.

## Claims

1. A targeted drug conjugate comprising an ALK5 inhibitor covalently attached to a targeting moiety which comprises an antibody or an antibody fragment that binds to a cell surface molecule expressed on the surface of myofibroblasts, activated fibroblasts, fibroblasts transitioning to myofibroblasts, or a combination thereof, wherein the ALK5 inhibitor is N-methyl-2-(4-(4-(3-(6-methylpyridin-2-yl)-1H-pyrazol-4-yl)pyridin-2-yl)phenoxy)ethan-1-amine.

2. The targeted drug conjugate of claim 1, wherein the ALK5 inhibitor is covalently attached to a targeting moiety which comprises an antibody or an antibody fragment that binds to a cell surface molecule expressed on the surface of cancer-associated fibroblasts.

3. The targeted drug conjugate of claim 1 or claim 2, wherein the ALK5 inhibitor is covalently attached to the targeting moiety via a linker comprising a chain of atoms that covalently attaches the ALK5 inhibitor to the targeting moiety, optionally wherein the linker is a PEG containing linker.

4. The targeted drug conjugate of any one of claims 1 to 3, wherein the ALK5 inhibitor is covalently attached to the targeting moiety via (a) an N-maleimidomethylcyclohexane1-carboxylate, maleimidocaproyl or mercaptoacetamidocaproyl linker or (b) a dipeptide linker, a disulfide linker, or a hydrazone linker, for example a protease-sensitive valine-citrulline dipeptide linker, a glutathione-sensitive disulfide linker, or an acid-sensitive disulfide linker.

5. The targeted drug conjugate of any one of claims 1 to 4, wherein the ALK5 inhibitor is conjugated via one or more cysteine residues on the targeting moiety or one or more lysine residues on the targeting moiety, optionally wherein the ALK5 inhibitor is conjugated via a linker comprising a chain of atoms that covalently attaches the ALK5 inhibitor to the targeting moiety.

6. The targeted drug conjugate of any one of claims 1 to 5, wherein the average number of ALK5 inhibitor molecules per targeting moiety molecule ranges between 2 and 8.

7. The targeted drug conjugate of any one of claims 1 to 6, wherein the targeting moiety comprises a monoclonal antibody, optionally wherein the antibody is human or humanized.

8. The targeted drug conjugate of any one of claims 1 to 6, wherein the targeting moiety comprises an antibody fragment, optionally wherein the antibody fragment is (a) a Fab, a Fab', a F(ab')₂, a Fv, scFv, a dsFv, or single domain antibody or (b) a fragment of a human or humanized antibody.

9. The targeted drug conjugate of any one of claims 1 to 8, wherein the cell surface molecule is FAP, PDGFR-β, FGFR1, PPAR-γ, FSP1, GFAP, fascin, CD147, CXCR4, αvβ6, AXL, or MERTK.

10. The targeted drug conjugate of any one of claims 1 to 8, wherein the cell surface molecule is LRRC15.

11. A pharmaceutical composition comprising the targeted drug conjugate of any one of claims 1 to 10 and a pharmaceutically acceptable carrier.

12. The targeted drug conjugate according to any one of claims 1 to 10 or the pharmaceutical composition according to claim 11 for use in a method of treating fibrosis, optionally wherein the fibrosis is (a) pulmonary fibrosis, liver fibrosis, kidney fibrosis, cardiac fibrosis, skin fibrosis, or esophagus fibrosis or (b) idiopathic pulmonary fibrosis (IPF).

13. The targeted drug conjugate or pharmaceutical composition for use according to claim 12, wherein the targeted drug conjugate is administered as monotherapy.

14. The targeted drug conjugate or pharmaceutical composition for use according to claim 12, wherein the targeted drug conjugate is administered as part of a combination therapy regimen, optionally wherein the combination therapy regimen comprises pirfenidone or nintedanib.

15. The targeted drug conjugate according to any one of claims 1 to 10 or the pharmaceutical composition according to claim 11 for use in a method of treating systemic sclerosis.

16. The targeted drug conjugate according to any one of claims 1 to 10 or the pharmaceutical composition according to claim 11 for use in a method of treating cancer.

## Patentansprüche

1. Zielgerichtetes Wirkstoffkonjugat, das einen ALK5-Inhibitor aufweist, der kovalent an eine Zieleinheit gebunden ist, die einen Antikörper oder ein Antikörperfragment aufweist, der bzw. das sich an ein Zelloberflächenmolekül bindet, das auf der Oberfläche von Myofibroblasten, aktivierten Fibroblasten, Fibroblasten im Übergang zu Myofibroblasten, oder einer Kombination davon exprimiert ist, wobei der ALK5-Inhibitor N-Methyl-2-(4-(4-(3-(6-methylpyridin-2-yl)-1H-pyrazol-4-yl)pyridin-2-yl)phenoxy)ethan-1-amin ist.

2. Zielgerichtetes Wirkstoffkonjugat nach Anspruch 1, wobei der ALK5-Inhibitor kovalent an eine Zieleinheit gebunden ist, die einen Antikörper oder ein Antikörperfragment aufweist, der bzw. das sich an ein auf der Oberfläche von krebsassoziierten Fibroblasten exprimiertes Zelloberflächenmolekül bindet.

3. Zielgerichtetes Wirkstoffkonjugat nach Anspruch 1 oder Anspruch 2, wobei der ALK5-Inhibitor über einen Linker, der eine Atomkette aufweist, die den ALK5-Inhibitor kovalent an die Zieleinheit bindet, kovalent an die Zieleinheit gebunden ist, wobei der Linker optional ein PEG-haltiger Linker ist.

4. Zielgerichtetes Wirkstoffkonjugat nach einem der Ansprüche 1 bis 3, wobei der ALK5-Inhibitor über (a) einen N-Maleimidomethylcyclohexan-1-carboxylat-, Maleimidocaproyl- oder Mercaptoacetamidocaproyl-Linker oder (b) einen Dipeptid-Linker, einen Disulfid-Linker oder einen Hydrazon-Linker, beispielsweise einen proteaseempfindlichen Valin-Citrullin-Dipeptid-Linker, einen Glutathion-empfindlichen Disulfid-Linker oder einen säureempfindlichen Disulfid-Linker, kovalent an die Zieleinheit gebunden ist.

5. Zielgerichtetes Wirkstoffkonjugat nach einem der Ansprüche 1 bis 4, wobei der ALK5-Inhibitor über einen oder mehrere Cysteinreste an der Zieleinheit oder einen oder mehrere Lysinreste an der Zieleinheit konjugiert ist, wobei der ALK5-Inhibitor optional über einen Linker konjugiert ist, der eine Atomkette aufweist, die den ALK5-Inhibitor kovalent an die Zieleinheit bindet.

6. Zielgerichtetes Wirkstoffkonjugat nach einem der Ansprüche 1 bis 5, wobei die durchschnittliche Anzahl an ALK5-Inhibitor-Molekülen pro Zieleinheit-Molekül zwischen 2 und 8 liegt.

7. Zielgerichtetes Wirkstoffkonjugat nach einem der Ansprüche 1 bis 6, wobei die Zieleinheit einen monoklonalen Antikörper aufweist, wobei der Antikörper optional human oder humanisiert ist.

8. Zielgerichtetes Wirkstoffkonjugat nach einem der Ansprüche 1 bis 6, wobei die Zieleinheit ein Antikörperfragment aufweist, wobei das Antikörperfragment optional (a) ein Fab, ein Fab', ein F(ab')₂, ein Fv, ein scFv, ein dsFv oder ein Einzeldomänen-Antikörper oder (b) ein Fragment eines humanen oder humanisierten Antikörpers ist.

9. Zielgerichtetes Wirkstoffkonjugat nach einem der Ansprüche 1 bis 8, wobei das Zelloberflächenmolekül FAP, PDGFR-β, FGFR1, PPAR-γ, FSP1, GFAP, Fascin, CD147, CXCR4, αvβ6, AXL oder MERTK ist.

10. Zielgerichtetes Wirkstoffkonjugat nach einem der Ansprüche 1 bis 8, wobei das Zelloberflächenmolekül LRRC15 ist.

11. Pharmazeutische Zusammensetzung, die das zielgerichtete Wirkstoffkonjugat nach einem der Ansprüche 1 bis 10 und einen pharmazeutisch akzeptablen Träger aufweist.

12. Zielgerichtetes Wirkstoffkonjugat nach einem der Ansprüche 1 bis 10 oder pharmazeutische Zusammensetzung nach Anspruch 11 zur Verwendung in einem Verfahren zur Behandlung von Fibrose, wobei die Fibrose optional (a) Lungenfibrose, Leberfibrose, Nierenfibrose, Herzfibrose, Hautfibrose oder Ösophagusfibrose oder (b) idiopathische Lungenfibrose (IPF) ist.

13. Zielgerichtetes Wirkstoffkonjugat oder pharmazeutische Zusammensetzung zur Verwendung nach Anspruch 12, wobei das zielgerichtete Wirkstoffkonjugat als Monotherapie verabreicht wird.

14. Zielgerichtetes Wirkstoffkonjugat oder pharmazeutische Zusammensetzung zur Verwendung nach Anspruch 12, wobei das zielgerichtete Wirkstoffkonjugat als Teil eines Kombinationstherapieschemas verabreicht wird, wobei das Kombinationstherapieschema optional Pirfenidon oder Nintedanib aufweist.

15. Zielgerichtetes Wirkstoffkonjugat nach einem der Ansprüche 1 bis 10 oder pharmazeutische Zusammensetzung nach Anspruch 11 zur Verwendung in einem Verfahren zur Behandlung von systemischer Sklerose.

16. Zielgerichtetes Wirkstoffkonjugat nach einem der Ansprüche 1 bis 10 oder pharmazeutische Zusammensetzung nach Anspruch 11 zur Verwendung in einem Verfahren zur Behandlung von Krebs.

## Revendications

1. Conjugué de médicament ciblé comprenant un inhibiteur d'ALK5 fixé de manière covalente à une fraction de ciblage qui comprend un anticorps ou un fragment d'anticorps qui se lie à une molécule de surface cellulaire exprimée à la surface de myofibroblastes, de fibroblastes activés, de fibroblastes en transition vers des myofibroblastes, ou d'une combinaison de ceux-ci, dans lequel l'inhibiteur d'ALK5 est la N-méthyl-2-(4-(4-(3-(6-méthylpyridin-2-yl)-1H-pyrazol-4-yl)pyridin-2-yl)phénoxy)éthan-1-amine.

2. Conjugué de médicament ciblé de la revendication 1, dans lequel l'inhibiteur d'ALK5 est fixé de manière covalente à une fraction de ciblage qui comprend un anticorps ou un fragment d'anticorps qui se lie à une molécule de surface cellulaire exprimée à la surface de fibroblastes associés au cancer.

3. Conjugué de médicament ciblé de la revendication 1 ou de la revendication 2, dans lequel l'inhibiteur d'ALK5 est fixé de manière covalente à la fraction de ciblage via un lieur comprenant une chaîne d'atomes qui fixe de manière covalente l'inhibiteur d'ALK5 à la fraction de ciblage, facultativement dans lequel le lieur est un lieur contenant du PEG.

4. Conjugué de médicament ciblé de l'une quelconque des revendications 1 à 3, dans lequel l'inhibiteur d'ALK5 est fixé de manière covalente à la fraction de ciblage via (a) un lieur N-maléimidométhylcyclohexane1-carboxylate, maléimidocaproyle ou mercaptoacétamidocaproyle ou (b) un lieur dipeptidique, un lieur disulfure, ou un lieur hydrazone, par exemple un lieur dipeptidique valine-citrulline sensible à une protéase, un lieur disulfure sensible au glutathion, ou un lieur disulfure sensible à un acide.

5. Conjugué de médicament ciblé de l'une quelconque des revendications 1 à 4, dans lequel l'inhibiteur d'ALK5 est conjugué via un ou plusieurs résidus cystéine sur la fraction de ciblage ou un ou plusieurs résidus lysine sur la fraction de ciblage, facultativement dans lequel l'inhibiteur d'ALK5 est conjugué via un lieur comprenant une chaîne d'atomes qui fixe de manière covalente l'inhibiteur d'ALK5 à la fraction de ciblage.

6. Conjugué de médicament ciblé de l'une quelconque des revendications 1 à 5, dans lequel le nombre moyen de molécules d'inhibiteur d'ALK5 par molécule de fraction de ciblage est compris entre 2 et 8.

7. Conjugué de médicament ciblé de l'une quelconque des revendications 1 à 6, dans lequel la fraction de ciblage comprend un anticorps monoclonal, facultativement dans lequel l'anticorps est humain ou humanisé.

8. Conjugué de médicament ciblé de l'une quelconque des revendications 1 à 6, dans lequel la fraction de ciblage comprend un fragment d'anticorps, facultativement dans lequel le fragment d'anticorps est (a) un Fab, un Fab', un F(ab')₂, un Fv, scFv, un dsFv, ou un anticorps à domaine unique ou (b) un fragment d'un anticorps humain ou humanisé.

9. Conjugué de médicament ciblé de l'une quelconque des revendications 1 à 8, dans lequel la molécule de surface cellulaire est FAP, PDGFR-β, FGFR1, PPAR-γ, FSP1, GFAP, fascine, CD147, CXCR4, αvβ6, AXL, ou MERTK.

10. Conjugué de médicament ciblé de l'une quelconque des revendications 1 à 8, dans lequel la molécule de surface cellulaire est LRRC15.

11. Composition pharmaceutique comprenant le conjugué de médicament ciblé de l'une quelconque des revendications 1 à 10 et un vecteur pharmaceutiquement acceptable.

12. Conjugué de médicament ciblé selon l'une quelconque des revendications 1 à 10 ou composition pharmaceutique selon la revendication 11 pour son utilisation dans un procédé de traitement d'une fibrose, facultativement dans lequel la fibrose est (a) une fibrose pulmonaire, une fibrose hépatique, une fibrose rénale, une fibrose cardiaque, une fibrose cutanée, ou une fibrose de l'œsophage ou (b) une fibrose pulmonaire idiopathique (IPF).

13. Conjugué de médicament ciblé ou composition pharmaceutique pour l'utilisation selon la revendication 12, dans lequel le conjugué de médicament ciblé est administré en monothérapie.

14. Conjugué de médicament ciblé ou composition pharmaceutique pour l'utilisation selon la revendication 12, dans lequel le conjugué de médicament ciblé est administré dans le cadre d'un schéma thérapeutique de combinaison, facultativement dans lequel le schéma thérapeutique de combinaison comprend de la pirfénidone ou du nintédanib.

15. Conjugué de médicament ciblé selon l'une quelconque des revendications 1 à 10 ou composition pharmaceutique selon la revendication 11 pour son utilisation dans un procédé de traitement d'une sclérose systémique.

16. Conjugué de médicament ciblé selon l'une quelconque des revendications 1 à 10 ou composition pharmaceutique selon la revendication 11 pour son utilisation dans un procédé de traitement d'un cancer.
